# EUROPÄISCHE PATENTSCHRIFT

(11) **EP 1 150 980 B1**
(45) Veröffentlichungstag und Bekanntmachung des Hinweises auf die Patenterteilung: **22.08.2007**
(21) Anmeldenummer: 00909205.7
(22) Anmeldetag: 18.02.2000
(51) Int. Cl.: C07D 417/06, C07D 493/04, C07D 405/06, A61K 31/427, A61K 31/4427, A61P 35/00

(54) **16-HALOGEN-EPOTHILON-DERIVATE, VERFAHREN ZU DEREN HERSTELLUNG UND IHRE PHARMAZEUTISCHE VERWENDUNG**
16-HALOGEN-EPOTHILONE DERIVATIVES, METHOD FOR PRODUCING THEM AND THEIR PHARMACEUTICAL USE
DERIVES DE 16-HALOGENO-EPOTHILON, LEUR PROCEDE DE PRODUCTION ET LEUR UTILISATION PHARMACEUTIQUE

(30) Priorität: 18.02.1999 DE 19908765; 04.11.1999 DE 19954230
(43) Veröffentlichungstag der Anmeldung: 07.11.2001
(73) Patentinhaber: Bayer Schering Pharma Aktiengesellschaft, 13353 Berlin (DE)
(72) Erfinder: KLAR, Ulrich, D-13503 Berlin (DE); SKUBALLA, Werner, D-13465 Berlin (DE); BUCHMANN, Bernd, D-16540 Hohen Neuendorf (DE); SCHWEDE, Wolfgang, D-13467 Berlin (DE); SCHIRNER, Michael, D-13156 Berlin (DE)
(86) Internationale Anmeldenummer: PCT/EP2000/001333
(87) Internationale Veröffentlichungsnummer: WO 2000/049021

(56) Entgegenhaltungen:
- EP-A- 0 353 732
- EP-A- 0 435 843
- WO-A-00/00485
- WO-A-98/25929
- WO-A-99/07692
- CRICH, DAVID ET AL: "Asymmetric synthesis of a taxol C-ring by aldol condensation and radical cyclization" TETRAHEDRON (1997), 53(21), 7127-7138 , XP004105693
- NAGASAWA, KAZUO ET AL: "An efficient asymmetric synthesis of 1.alpha.,25-(OH)2 vitamin D3 A-ring synthon" J. ORG. CHEM. (1993), 58(9), 2523-9 , XP002150715
- ALLMENDINGER, THOMAS ET AL: "Fluoroolefin dipeptide isosteres. II. Enantioselective synthesis of both antipodes of the Phe-Gly dipeptide mimic" TETRAHEDRON LETT. (1990), 31(50), 7301-4 , XP002150716

## Beschreibung

Von Höfle et al. wird die cytotoxische Wirkung der Naturstoffe Epothilon A (R = Wasserstoff) und Epothilon B (R = Methyl) 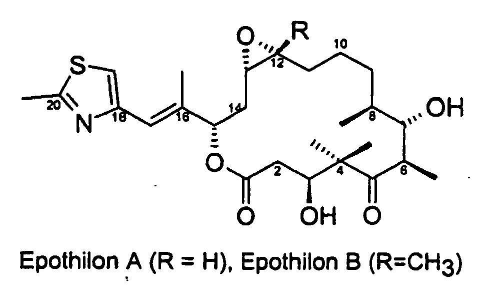 z.B. in Angew. Chem. 1996, 108, 1671-1673, beschrieben. Wegen der in-vitro-Selektivität gegenüber Brust- und Darmzelllinien und ihrer im Vergleich zu Taxol deutlich höheren Aktivität gegen P-Glycoprotein-bildende, multiresistente Tumorlinien sowie ihre gegenüber Taxol verbesserten physikalischen Eigenschaften, z.B eine um den Faktor 30 höhere Wasserlöslichkeit, ist diese neuartige Strukturklasse für die Entwicklung eines Arzneimittels zur Therapie maligner Tumoren besonders interessant. Die Naturstoffe sind sowohl chemisch als auch metabolisch für eine Arzneimittelentwicklung nicht ausreichend stabil. Zur Beseitigung dieser Nachteile sind Modifikationen an dem Naturstoff nötig. Derartige Modifikationen sind nur auf totalsynthetischem Wege möglich und setzen Synthesestrategien voraus, die eine breite Modifikation des Naturstoffes ermöglichen. Ziel der Strukturveränderungen ist es auch, die therapeutische Breite zu erhöhen. Dies kann durch eine Verbesserung der Selektivität der Wirkung und/oder eine Erhöhung der Wirkstärke und/oder eine Reduktion unerwünschter toxischer Nebenwirkungen, wie sie in Proc. Natl. Acad. Sci. USA 1998, 95, 9642-9647 beschrieben sind, erfolgen.
Die Totalsynthese von Epothilon A ist von Schinzer et al. in Chem. Eur. J. 1996, 2, No. 11, 1477-1482 und in Angew. Chem. 1997, 109, Nr. 5, S. 543-544) beschrieben.
Epothilon-Derivate wurden bereits von Höfle et al. in der WO 97/19086 beschrieben. Diese Derivate wurden ausgehend vom natürlichen Epothilon A oder B hergestellt. Auch Epothilon C und D (Doppelbindung zwischen den Kohlenstoffatomen 12 und 13:
Epothilon C = Desoxyepothilon A; Epothilon D = Desoxyepothilon B) sind als mögliche Ausgangsprodukte hierfür beschrieben.
Eine weitere Synthese von Epothilon und Epothilonderivaten wurde von Nicolaou et al. in Angew. Chem. 1997, 109, Nr. 1/2, S. 170 - 172 beschrieben. Die Synthese von Epothilon A und B und einiger Epothilon-Analoga wurde in Nature, Vol. 387, 1997, S. 268-272, die Synthese von Epothilon A und seinen Derivaten in J. Am. Chem. Soc., Vol. 119, No. 34, 1997, S. 7960 - 7973 sowie die Synthese von Epothilon A und B und einiger Epothilon-Analoga in J. Am. Chem. Soc., Vol. 119, No. 34, 1997, S. 7974 - 7991 ebenfalls von Nicolaou et al. beschrieben.
Ebenfalls Nicolaou et al. beschreiben in Angew. Chem. 1997, 109, Nr. 19, S. 2181-2187 die Herstellung von Epothilon A-Analoga mittels kombinatorischer Festphasensynthese. Auch einige Epothilon B-Analoga sind dort beschrieben.

Epothilon-Derivate, z.T. auch Epothilon C und D, sind des weiteren in den Patentanmeldungen WO 99/07692, WO 99/02514, WO 99/01124, WO 99/67252, WO 98/25929, WO 97/19086, WO 98/38192, WO 99/22461 und WO 99158534 beschrieben.

In den bisher bekannt gewordenen Epothilon-Derivaten kann am Kohlenstoffatom 16des Epothilongerüstes kein Halogenatom stehen.

Die Aufgabe der vorliegenden Erfindung besteht darin, neue Epothilon-Derivate zur Verfügung zu stellen, die sowohl chemisch als auch metabolisch für eine Arzneimittelentwicklung ausreichend stabil sind und die hinsichtlich ihrer therapeutischen Breite, ihrer Selektivität der Wirkung und/oder unerwünschter toxischer Nebenwirkungen und/oder ihrer Wirkstärke den natürlichen Derivaten überlegen sind.

Die vorliegende Erfindung beschreibt die neuen Epothilon-Derivate der allgemeinen Formel I, 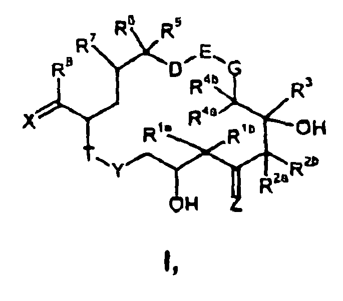 worin
- R^{1a}, R^{1b}: gleich oder verschieden sind und Wasserstoff. C₁-C₁₀-Alkyl, Aryl. C₇-C₂₀-Aralkyl, oder gemeinsam eine -(CH₂)ₘ-Gruppe mit m = 2, 3, 4 oder 5.
- R^{2a}, R^{2b}: gleich oder verschieden sind und Wasserstoff, C₁-C₁₀-Alkyl, Aryl, C₇-C₂₀-Aralkyl oder gemeinsam eine -(CH₂)ₙ-Gruppe mit n = 2. 3, 4 oder 5
- R³: Wasserstoff, C₁-C₁₀-Alkyl, Aryl, C₇-C₂₀-Aralkyl,
- G: ein Sauerstoffatom oder eine Gruppe CH₂.
- R^{4a}, R^{4b}: gleich oder verschieden sind und Wasserstoff, C₁-C₁₀-Alkyl, Aryl, C₇-C₂₀-Aralkyl oder gemeinsam eine -(CH₂)ₚ-Gruppe mit p = 2, 3, 4 oder 5,
- D-E: eine Gruppe H₂C-CH₂, HC=CH, C≡C, 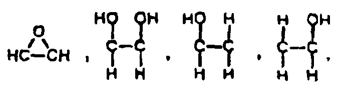 H₂C-O-, -O-CH₂
- R⁵: Wasserstoff. C₁-C₁₀-Alkyl, Aryl, C₇-C₂₀-Aralkyl, CO₂H, CO₂-Alkyl, CH₂OH, CH₂O-Alkyl, CH₂O-Acyl, CN, CH₂NH₂, CH₂N(Alkyl, Acyl)_{1,2}, CH₂Hal
- R⁶, R⁷: je ein Wasserstoffatom, gemeinsam eine zusätzliche Bindung oder ein Sauerstoffatom,
- R⁸: ein Halogenatom oder eine Cyanogruppe,
- X: eine Gruppierung CR¹⁰R¹¹,
wobei einer der Reste
- und der auderen Rest R¹¹: für einen gegebenenfalls substituierten Heteroarylrest
stehen,
- T-Y: eine Gruppe O-C(=O), O-CH₂, CH₂C(=O), NR²⁴-C(=O), NR²⁴-SO₂,
- R²⁴: Wasserstoff, C₁-C₁₀-Alkyl,
- Z: ein Sauerstoffatom oder H/OR¹²,
wobei
R¹² Wasserstoff oder eine Schutzgruppe PG^{Z} ist,
bedeuten.

Das Halogenatom R⁸ kann ein Fluor-, Chlor-, Brom- oder lodatom sein.

Fluor, Chlor und Brom sind bevorzugt, und von diesen insbesondere Fluor und Chlor.

R2^{a} soll vorzugsweise eine Methyl-, Ethyl- Propyl- oder Butylgruppe bedeuten.

Für die Substituenten R^{1a} und R^{1b} steht vorzugsweise gemeinsam eine Trimethylengruppe oder R^{1a} und R^{1b} bedeuten je eine Methylgruppe.
R¹⁰/R¹¹ in der Gruppe X stehen vorzugsweise für 2-Pyridylrest/Wasserstoff oder 2-Methyl-4-thiazolylrest/Wasserstoff oder 2-Hydroxymethyl-4-thiazolylrest/Wasserstoff oder 2-Methyl-4-oxazolylrest/Wasserstoff oder 2-Hydroxymethyl-4-oxazolylrest/Wasserstoff.

T-Y ist vorzugsweise eine Gruppe O-C(=O) oder eine Gruppe NR²⁴-C(=O),

Z bedeutet in erster Linie ein Sauerstoffatom.

Zwischen den Kohlenstoffatomen 10 und 11 befindet sich in den bevorzugten Verbindungen der allgemeinen Formel I eine Einfachbindung, d.h. -D-E- steht für eine Ethylengruppe.

Außerdem steht in den erfindungsgemäßen Verbindungen R³ gewöhnlich für ein Wasserstoffatom.

Für die beiden Substituenten R^{4a}/R^{4b} steht vorzugsweise die Kombination H/CH₃.

Eine Ausführungsform der Erfindung sieht solche Verbindungen der allgemeinen Formel I vor, worin R⁸ für ein Fluor- oder Chloratom steht und R^{1a} + R^{1b} gemeinsam eine Trimethylengruppe bedeuten.

Gemäß einer weiteren Ausführungsform betrifft die Erfindung solche Verbindungen der allgemeinen Formel I, worin R⁸ für ein Fluor- oder Chloratom und R¹⁰/R¹¹ für 2-Pyridylrest/Wasserstoff stehen.

Noch eine andere Variante sind solche Verbindungen der allgemeinen Formel I worin R⁸ für ein Fluor- oder Chloratom und R^{2a}/R^{2b} für Ethyl/Wasserstoff stehen.

Noch eine weitere Ausführungsform der Erfindung sind solche Verbindungen der allgemeinen Formel I, worin R⁸ für ein Fluor- oder Chloratom steht, R^{1a} + R^{1b} gemeinsam eine Trimethylengruppe bedeuten sowie R^{2a}/R^{2b} für EthyltWasserstoff stehen.

Außerdem ist noch diese Variante für die erfindungsgemäßen Verbindungen zu nennen, worin worin R⁸ für ein Fluor- oder Chloratom steht, R^{2a}/R^{2b} für Ethyl/Wasserstoff und R¹⁰/R¹¹ für 2-Pyridylrest/Wasserstoff stehen.

Weitere Ausgestaltungsformen der vorliegenden Erfindung ergeben sich aus den Merkmalen der Unteransprüche.

Die Darstellung der neuen Epothilon-Derivate basiert auf der Verknüpfung dreier Teilfragmente A, B und C. Dieses Verfahren ist zur Herstellung von Epothilon-Derivaten, welche als R⁸ anstelle des erfindungsgemäßen Halogenatoms beispielsweise eine Methyl-oder längere Alkylgruppe enthalten, in der DE 197 51 200.3, Anmeldetag 13.11.1997 sowie in der dazu korrespondierenden WO 99/07692 beschrieben. Die Schnittstellen liegen wie in der allgemeinen Formel I' angedeutet.

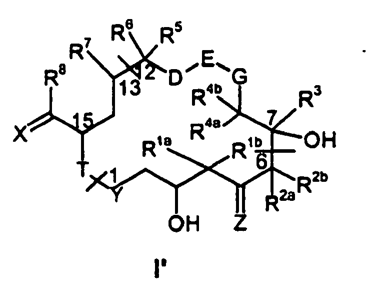

A bedeutet ein C1-C6-Fragment (Epothilon-Zählweise) der allgemeinen Formel 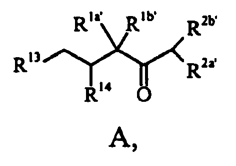 worin
- R^{1a'}, R^{1b'}, R^{2a'} und R^{2b'}: die bereits für R^{1a}, R^{1b}, R^{2a} und R^{2b} genannten Bedeutungen haben und
- R¹³: CH₂OR^{13a}, CH₂-Hal, CHO, CO₂R^{13b}, COHal,
- R14: Wasserstoff, OR^{14a}, Hal, OSO₂R^{14b},
- R^{13a}, R^{14a}: Wasserstoff, SO₂-Alkyl, SO₂-Aryl, SO₂-Aralkyl oder gemeinsam eine -(CH₂)ₒ-Gruppe oder gemeinsam eine CR^{15a}R^{15b}-Gruppe,
- R^{13b}, R^{14b}: Wasserstoff, C₁-C₂₀-Alkyl, Aryl, C₇-C₂₀-Aralkyl,
- R^{15a}, R^{15b}: gleich oder verschieden sind und Wasserstoff, C₁-C₁₀-Alkyl, Aryl, C₇-C₂₀-Aralkyl, oder gemeinsam eine -(CH₂)_{q}-Gruppe,
- Hal: Halogen (F, Cl, Br, I),
- o: 2 bis 4,
- q: 3 bis 6,
einschließlich aller Stereoisomeren sowie deren Gemische
bedeuten sowie
freie Hydroxylgruppen in-R¹³ und R¹⁴ verethert oder verestert, freie Carbonylgruppen in A und R¹³ ketalisiert, in einen Enolether überführt oder reduziert sowie freie Säuregruppen in A in deren Salze mit Basen überführt sein können.
B steht für ein C7-C12-Fragment (Epothilon-Zählweise) der allgemeinen Formel 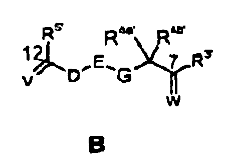 worin
- R^{3'}, R^{4a}', R^{4b'} und R^{5'}: die bereits für R³, R^{4a}, R^{4b} und R⁵ genannten Bedeutungen haben,
- D, E und G: die in der allgemeinen Formel I angegebenen Bedeutungen haben und
- V: ein Sauerstoffatom, zwei Alkoxygruppen OR¹⁷, eine C₂-C₁₀-Alkylen-α,ω-dioxygruppe, die geradkettig oder verzweigt sein kann oder H/OR¹⁶.
- W: ein Sauerstoffatom, zwei Alkoxygruppen OR¹⁹, eine C₂-C₁₀-Alkylen-α,ω-dioxygruppe, die geradkettig oder verzweigt sein kann oder H/OR¹⁸,
- R¹⁶, R¹⁸: unabhängig voneinander Wasserstoff oder eine Schutzgruppe PG¹
- R¹⁷,R¹⁹: unabhängig voneinander C₁-C₂₀-Alkyl,
bedeuten,
C steht für ein C13-C16-Fragment (Epothilon-Zahlweise) der allgemeinen Formel 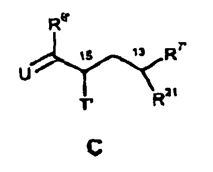 worin
- R^{8'}: ein Fluor- oder Chloratom oder eine Cyanogruppe und
- R^{7'}: ein Wasserstoffatom,
- T': eine Gruppe OR²⁰, wobei R²⁰ ein Wasserstoffatom oder eine Schutzgruppe PG² ist, ein Halogenatom, vorzugsweise ein Brom- oder Iod-Atom, eine Azido- oder eine geschützte Aminogruppe,
- R²¹: eine Hydroxygruppe, Halogen, eine geschützte Hydroxygruppe OPG³, ein Phosphoniumhalogenidrest PPh₃⁺Hal⁻ (Ph = Phenyl; Hal = F, Cl, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
- U: eine Gruppierung CR¹⁰R¹¹,
wobei
R¹⁰, R¹¹ gleich oder verschieden sind und für Wasserstoff, einen C₁-C₂₀-Alkyl-, Aryl-, C₇-C₂₀-Aralkylrest oder
R¹⁰ und R¹¹ zusammen mit dem Methylenkohlenstoffatom gemeinsam für einen 5- bis 7-gliedrigen carbocyclischen Ring stehen,
bedeuten, ausgenommen die Verbindungen (E)-3-Fluor-1-phenyl-hex-2-en-4,6-diol und 2-Brom-3-(tert.-butyldimethylsilyloxy)-1-penten-5-ol.

Als Alkylgruppen R^{1a}, R^{1b}, R^{2a}, R^{2b}, R³, R⁴, R⁵, R⁹, R¹⁰, R¹¹, R¹², R^{13b,} R^{14b}, R^{15a}, R^{15b}, R¹⁷ und R²³ sind gerad- oder verzweigtkettige Alkylgruppen mit 1-20 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R^{1a}, R^{1b}, R^{2a}, R^{2b}, R³, R⁴, R⁵, R⁹, R¹⁰, R¹¹, R¹², R^{13b}, R^{14b}, R^{15a}, R^{15b}, R¹⁷ und R²³ können perfluoriert oder substituiert sein durch 1-5 Halogenatome, Hydroxygruppen, C₁-C₄-Alkoxygruppen, C₆-C₁₂-Arylgruppen (die durch 1-3 Halogenatome substituiert sein können).
Als Arylrest R^{1a}, R^{1b}, R^{2a}, R^{2b}, R³, R⁴, R⁵, R⁹, R¹⁰, R¹¹, R¹², R^{13b}, R^{14b}, R^{15a} und R^{15b} kommen substituierte und unsubstituierte carbocyclische oder heterocyclische Reste mit einem oder mehreren Heteroatomen wie z.B. Phenyl, Naphthyl, Furyl, Thienyl, Pyridyl, Pyrazolyl, Pyrimidinyl, Oxazolyl, Pyridazinyl, Pyrazinyl, Chinolyl, Thiazolyl, die einfach oder mehrfach substituiert sein können durch Halogen, OH, O-Alkyl, CO₂H, CO₂-Alkyl, -NH₂, -NO₂, -N₃, -CN, C₁-C₂₀-Alkyl, C₁-C₂₀-Acyl, C₁-C₂₀-Acyloxy-Gruppen, in Frage. Heteroatome in den Heteroarylresten können oxidiert sein; so kann beispielsweise der Thiazolring in Form des N-Oxids vorliegen.
Wenn nicht anders erwähnt, schließt die Definition "Aryl" immer auch "Heteroaryl" mit ein.
Die Aralkylgruppen in R^{1a}, R^{1b}, R^{2a}, R^{2b}, R³, R⁴, R⁵, R⁹, R¹⁰, R¹¹, R¹² R^{13b}, R^{14b}, R^{15a} und R^{15b} können im Ring bis 14 C-Atome, bevorzugt 6 bis 10 und in der Alkylkette 1 bis 8, bevorzugt 1 bis 4 Atome enthalten. Als Aralkylreste kommen beispielweise in Betracht Benzyl, Phenylethyl, Naphthylmethyl, Naphthylethyl, Furylmethyl, Thienylethyl, Pyridylpropyl. Die Ringe können einfach oder mehrfach substituiert sein durch Halogen, OH, O-Alkyl, CO₂H, CO₂-Alkyl, -NO₂, -N₃, -CN, C₁-C₂₀-Alkyl, C₁-C₂₀-Acyl, C₁-C₂₀-Acyloxy-Gruppen.
Die in X in der allgemeinen Formel I enthaltenen Alkoxygruppen sollen jeweils 1 bis 20 Kohlenstoffatome enthalten, wobei Methoxy-, Ethoxy- Propoxy- Isopropoxy- und t-Butyloxygruppen bevorzugt sind.
Als Vertreter für die Schutzgruppen PG sind Alkyl- und/oder Aryl-substituiertes Silyl, C₁-C₂₀-Alkyl, C₄-C₇-Cycloalkyl, das im Ring zusätzlich ein Sauerstoffatom enthalten kann, Aryl, C₇-C₂₀-Aralkyl, C₁-C₂₀-Acyl sowie Aroyl zu nennen.
Als Alkyl-, Silyl- und Acylreste für die Schutzgruppen PG kommen die dem Fachmann bekannten Reste in Betracht. Bevorzugt sind aus den entsprechenden Alkyl- und Silylethern leicht abspaltbare Alkyl- bzw. Silylreste, wie beispielsweise der Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl, para-Nitrobenzyl-, para-Methoxybenzyl-Rest sowie Alkylsulfonyl- und Arylsulfonylreste. Als Acylreste kommen z.B. Formyl, Acetyl, Propionyl, Isopropionyl, Pivalyl-, Butyryl oder Benzoyl, die mit Amino- und/oder Hydroxygruppen substituiert sein können, in Frage.
Die Acylgruppen PG^{x} bzw. PG^{z} in R⁹ und R¹² können 1 bis 20 Kohlenstoffatome enthalten, wobei Formyl-, Acetyl-, Propionyl-, Isopropionyl und Pivalylgruppen bevorzugt sind.
Als Aminoschutzgruppen kommen die dem Fachmann bekannten Reste in Betracht.
Beispielsweise genannt seien die Boc-, Z-, Benzyl, f-Moc-, Troc-, Stabase- oder Benzostabase-Gruppe.

Der Index m in der aus R^{1a} und R^{1b} gebildeten Alkylengruppe steht vorzugsweise für 2, 3 oder 4.

Die für X mögliche C₂-C₁₀-Alkylen-α,ω-dioxygruppe ist vorzugsweise eine Ethylenketal- oder Neopentylketalgruppe.

Die vorliegende Erfindung betrifft insbesondere die folgenden Verbindungen
(4S,7R,8S,9S,13(Z oder E),16S(Z))-4,8-Dihydroxy-16-(1-fluor-2-(2-methyl-4-thiazolyl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-fluor-2-(2-methyl-4-thiazolyl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7-ethyl-16-(1-fluor-2-(2-methyl-4-thiazolyl)ethenyl)-1-oxa-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-methyl-4-thiazolyl)ethenyl)-8,8,12,16-tetramethyl-4,17-dioxabicyclo[14.1.0]heptadecan-5,9-dion
(4S,7R,8S,9S,13(Z oder E),16S(Z))-4,8-Dihydroxy-16-(1-fluor-2-(2-methyloxazol-4-yl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-fluor-2-(2-methyloxazol-4-yl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7-ethyl-16-(1-fluor-2-(2-methyloxazol-4-yl)ethenyl)-1-oxa-5,5,9,13-tetramethyl-cyclohexadec-13-en-2,6-dion
(1 RS,3S(Z),7S, 10R,11S,12S,16RS)-7,11-Dihydroxy-10-ethyl-3-(1-fluor-2-(2-methyloxazol-4-yl)ethenyl)-8,8,12,16-tetramethyl-4,17-dioxabicycto[14.1.0]heptadecan-5,9-dion
(4S,7R,8S,9S,13(Z oder E),16S(Z))-4,8-Dihydroxy-16-(1-fluor-2-(2-pyridyl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-fluor-2-(2-pyridyl)ethenyl)-8,8,10,12,16-pentamethyl-4,17-dioxabicyclo[14.1.0]heptadecan-5,9-dion
(4S,7R,8S,9S,13(Z oder E), 16S(Z))-4,8-Dihydroxy-7-ethyl-16-(-fluor-2-(2-pyridyl)ethenyl)-1-oxa-5,5,9,13-tetramethyl-cyclohexadec-13-en-2,6-dion
(1RS,3S(Z),7S,10R,11 S,12S,16RS)-7,11-Dihydroxy-10-ethyl-3-(1-fluor-2-(2-pyridyl)ethenyl)-8,8,12,16-tetramethyl-4,17-dioxabicyclo[14.1.O]heptadecan-5,9-dion
(4S,7R,8S,9S,13(Z oder E),16S(Z))-4,8-Dihydroxy-16-(1-fluor-2-(2-methyl-4-thiazolyl)ethenyl)-1-aza-5,5,7,9,13-pentamethyl-cyclohexadec-13-en-2,6-dion
(1 RS,3S(Z),7S,1 0R,11 S,12S,16RS)-7,11-Dihydroxy-3-(1-fluor-2-(2-methyl-4-thiazolyl)ethenyl)-8,8,10.12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-fluor-2-(2-methyl-4-thiazolyl)ethenyl)-1-aza-5,5,9,13-tetramethyl-cyclohexadec-13-en-2,6-dion
(1RS,3S(Z),7S, 10R,11S,12S,16RS)-7,11-Dihydroxy-10-ethyl-3-(1-fluor-2-(2-methyl-4-thiazolyl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-fluor-2-(2-methyloxazol-4-yl)ethenyl)-1 -aza-5,5,7.9,13-pentamethyl-cyclohexadec-1 3-en-2,6-dion
(1RS,3S(Z),7S,10R,11 S,12S,16RS)-7,11-Dihydroxy-3-(1-fluor-2-(2-methyloxazol-4-yl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-fluor-2-(2-methyloxazol-4-yl)ethenyl)-1-aza-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-methyloxazol-4-yl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-fluor-2-(2-pyridyl)ethenyl)-1-aza-5,5,7,9,13-pentamethyl-cyclohexadec-13-en-2,6-dion
(1RS,3S(Z),7S,10R,11S,12S,16RS)-7,11-Dihydroxy-3-(1-fluor 2-(2-pyridyl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-fluor-2-(2-pyridyl)ethenyl)-1-aza-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-pyridyl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-chlor-2-(2-methyl-4-thiazolyl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-chlor-2-(2-methyl-4-thiazolyl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7 -ethyl-16-(1-chlor-2-(2-methyl-4-thiazolyl)ethenyl)-1-oxa-5,5,9,13-tetramethyl-cyclohexadec-13-en-2,6-dion
(1 RS,3S(Z),7S,1 OR,11 S,12S,16RS)-7,11-Dihydroxy-10-ethyl-3-(1-chlor-2-(2-methyl-4-thiazolyl)ethenyl)-8,8,12,16-tetramethyl-4,17-dioxabicyclo[14.1.0]heptadecan-5,9-dion
(4S,7R,8S,9S,13(Z oder E),16S(Z))-4,8-Dihydroxy-16-(1-chlor-2-(2-methyloxazol-4-yl)ethenyl)-1-oxa-5,5,7,9,13-pentamethyl-cyclohexadec-13-en-2,6-dion
(1RS,3S(Z),7S,10R,11 S,12S,16RS)-7,11-Dihydroxy-3-(1-chlor-2-(2-methyloxazol-4-yl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7 -ethyl-16-(1-chlor-2-(2-methyloxazo)-4-yl)ethenyl)-1-oxa-5,5,9,13-tetramethyl-cyclohexadec-13-en-2,6-dion
(1RS,3S(Z),7S,10R,11S,12S,16RS)-7,11-Dihydroxy-10-ethyl-3-(1-chlor-2-(2-methyloxazol-4-yl)ethenyl)-8,8,12,16-tetramethyl-4,17-dioxabicyclo[14.1.0]heptadecan-5,9-dion
(4S,7R,8S,9S,13(Z oder E),16S(Z))-4;8-Dihydroxy-16-(1-chlor-2-(2-pyridyl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-chlor-2-(2-pyridyl)ethenyl)-8,8.10,12,16-pentamethyl-4,17-dioxabicyclo[14.1.0]heptadecan-5,9-dion
(4S,7R,8S,9S,13(Z oder E),16S(Z))-4,8-Dihydroxy-7-ethyl-16-(1-chlor-2-(2-pyridyl)ethenyl)-1-oxa-5,5,9, 13-tetramethyl-cyclohexadec-13-en-2,6-dion
(1RS,3S(Z),7S,10R,11S,12S,16RS)-7,11-Dihydroxy-10-ethyl-3-(1-chlor-2-(2-pyridyl)ethenyl)-8,8,12,16-tetramethyl-4,17-dioxabicyclo[14.1.0]heptadecan-5,9-dion
(4S,7R,8S,9S, 13(Z oder E), 16S(Z))-4,8-Dihydroxy-16-(1-chlor-2-(2-methyl-4-thiazolyl)ethenyl)-1-aza-5,5,7,9,13-pentamethyl-cyclohexadec-13-en-2,6-dion
(1RS,3S(Z),7S,10R,11S,12S,16RS)-7,11-Dihydroxy-3-(1-chlor-2-(2-methyl-4-thiazolyl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-chlor-2-(2-methyl-4-thiazolyl)ethenyl)-1-aza-5,5,9,13-tetramethyl-cyclohexadec-13-en-2,6-dion
(1 RS,3S(Z),7S,10R,11S,12S,16RS)-7,11-Dihydroxy-10-ethyl-3-(1-chlor-2-(2-methyl-4-thiazolyl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-chlor-2-(2-methyloxazol-4-yl)ethenyl)-1-aza-5,5,7,9,13-pentamethyl-cyclohexadec-13-en-2,6-dion
(1RS,3S(Z),7S,10R,11S,12S,16RS)-7,11-Dihydroxy-3-(1-chlor-2-(2-methyloxazol-4-yl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-chlor-2-(2-methyloxazol-4-yl)ethenyl)-1-aza-5,5,9,13-tetramethyl-cyclohexadec-13-en-2,6-dion
(1RS,3S(Z),7S,10R,11S,12S,16RS)-7,11-Dihydroxy-10-ethyl-3-(1-chlor-2-(2-methyloxazol-4-yl)ethenyl)-8,8,12,16-tetramethyl-4-aza-17-oxabicycto[14.1.0]heptadecan-5,9-dion
(4S,7R,8S,9S,13(Z oder E),16S(Z))-4,8-Dihydroxy-16-(1-chlor-2-(2-pyridyl)ethenyl)-1-aza-5,5,7,9,13-pentamethyl-cyclohexadec-13-en-2,6-dion
(1RS,3S(Z),7S,10R,11S,12S,16RS)-7,11-Dihydroxy-3-(1-chlor-2-(2-pyridyl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-chlor-2-(2-pyridyl)ethenyl)-1-aza-5,5, 9,13-tetramethyl-cyclohexadec-13-en-2,6-dion
(1RS,3S(Z),7S,10R,11S,12S,16RS)-7,11-Dihydroxy-10-ethyl-3-(1-chlor-2-(2-pyridyl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-fluor-2-(2-methyl-4-thiazolyl)ethenyl)-1-oxa-7,9,13-trimethyl-5,5-(1,3-trimethylen)cyclohexadec-13-en-2,6-dion
(1 RS,3S(Z),7S,10R,11S,12S,16RS)-7,11-Dihydroxy-3-(1-fluor-2-(2-methyl-4-thiazolyl)ethenyl)-10,12,16-trimethyl-8,8-(1,3-trimethylen)-4,17-dioxabicyclo[14.1.0]hepta-deca-5,9-dion
(4S,7R,8S,9S,13(Z oder E),16S(Z))-4,8-Dihydroxy-9,13-dimethyl-7-ethyl-16-(1-fluor-2-(2-methyl-4-thiazolyl)ethenyl)-1-oxa-5,5-(1,3-trimethylen)cyclohexadec-13-en-2,6-dion
-(1RS,3S(Z),7S,10R,11S,12S,16RS)-7,11-Dihydroxy-12,16-dimethyl-10-ethyl-3-(1-fluor-2-(2-methyl-4-thiazolyl)ethenyl)-8,8-(1,3-trimethylen)-4,17-dioxabicyclo[14.1.0]heptadecan-5,9-dion
4S,7R,8S,9S,13(Z oder E),16S(Z))-4,8-Dihydroxy-16-(1-fluor-2-(2-methyl-4-thiazolyl)ethenyl)-1-aza-7,9,13-trimethyl-5,5-(1,3-trimethylen)cyclohexadec-13-en-2,6-dion
(1RS.3S(Z),7S,10R,11S,12S,16RS)-7,11-Dihydroxy-3-(1-fluor-2-(2-methyl-4-thiazolyl)ethenyl)-10,12,16-trimethyl-8,8-(1,3-trimethylen)-4-aza-17-oxabicyclo[14,1.0]hepta-deca-5.9-dion
(4S,7R,8S,9S,13(Z oder E),16S(Z))-4,8-Dihydroxy-9,13-dimethyl-7-ethyl-16-(1-fluor 2-(2-methyl-4-thiazolyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-fluor-2-(2-methyl-4-thiazolyl)ethenyl)-8,8-(1,3-trimethylen)-4-aza-17-oxabicyclo[14.1.0]heptadecan-5,9-dion
(4S,7R,8S,9S,13(Z oder E),16S(Z))-4,8-Dihydroxy-16-(1-chlor-2-(2-methyl-4-thiazolyl)ethenyl)-1 -oxa-7,9,13-trimethyl-5,5-(1,3-trimethylen)cyclohexadec-I 3-en-2,6-dion
(1RS,3S(Z),7S,10R,11S,12S,16RS)-7,11-Dihydroxy-3-(1-chlor-2-(2-methyl-4-thiazolyl)ethenyl)-10,12,16-trimethyl-8,8-(1,3-trimethylen)-4,17-dioxabicyclo[14.1.0]hepta-deca-5,9-dion
(4S.7R,8S,9S,13(Z oder E),16S(Z))-4,8-Dihydroxy-9,13-dimethyl-7-ethyl-16-(1-chlor-2-(2-methyl-4-thiazolyl)ethenyl)-1-oxa-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(11-chlor-2-(2-methyl-4-thiazolyl)ethenyl)-8,8-(1,3-trimethylen)-4,17-dioxabicyclo[14.1.0]hepta-decan-5,9-dion
4S,7R,8S,9S,13(Z oder E),16S(Z))-4,8-Dihydroxy-16-(1-chlor-2-(2-methyl-4-thiazolyl)ethenyl)-1-aza-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(-chlor-2-(2-methyl-4-thiazolyl)ethenyl)-10, 12,16-trimethyl-8,8-(1,3-trimethylen)-4-aza-17-oxabicyclo[14.1.0]hepta-deca-5,9-dion
(4S,7R,8S,9S,13(Z oder E),16S(Z))-4,8-Dihydroxy-9,13-dimethyl-7-ethyl-16-(1-chlor-2-(2-methyl-4-thiazolyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-chlor-2-(2-methyl-4-thiazolyl)ethenyl)-8,8-(1,3-trimethylen)-4-aza-17-oxabicyclo[14.1.0]hepta-decan-5,9-dion
(4S,7R,8S,9S,13(Z oder E),16S(Z))-4,8-Dihydroxy-16-(1-fluor-2-(2-pyridyl)ethenyl)-1-oxa-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-fluor-2-(2-pyridyl)ethenyl)-10,12,16-trimethyl-8,8-(1,3-trimethylen)-4,17-dioxabicyclo[14.1.0]hepta-deca-5,9-dion
(4S,7R,8S,9S,13(Z oder E),16S(Z))-4,8-Dihydroxy-9,13-dimethyl-7-ethyl-16-(1-fluor-2-(2-pyridyl)ethenyl)-1-oxa-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-fluor-2-(2-pyridyl)ethenyl)-8,8-(1,3-trimethylen)-4,17-dioxabicyclo[14.1.0]hepta-decan-5,9-dion
4S,7R,8S,9S,13(Z oder E),16S(Z))-4,8-Dihydroxy-16-(1-fluor-2-(2-pyridyl)ethenyl)-1-aza-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-fluor-2-(2-pyridyl)ethenyl)-10,12,16-trimethyl-8,8-(1,3-trimethylen)-4-aza-17-oxabicyclo[14.1.0]hepta-deca-5,9-dion
(4S,7R,8S,9S,13(Z oder E), 16S(Z))-4,8-Dihydroxy-9, 13-dimethyl-7 -ethyl-16-(1-fluor-2-(2-pyridyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-fluor-2-(2-pyridyl)ethenyl)-8,8-(1,3-trimethylen)-4-aza-17-oxabicyclo[14.1.O]hepta-decan-5,9-dion
(4S,7R,8S,9S,13(Z oder E),16S(Z))-4,8-Dihydroxy-16-(1-chlor-2-(2-pyridyl)ethenyl)-1-oxa-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-chlor-2-(2-pyridyl)ethenyl)-10,12,16-trimethyl-8,8-(1,3-trimethylen)-4,17-dioxabicyclo[14.1.0]hepta-deca-5,9-dion
(4S,7R,8S,9S,13(Z oder E),16S(Z))-4,8-Dihydroxy-9,13-dimethyl-7-ethyl-16-(1-chlor-2-(2-pyridyl)ethenyl)-1-oxa-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-chlor-2-(2-pyridyl)ethenyl)-8,8-(1,3-trimethylen)-4,17-dioxabicyclo[14.1.0]hepta-decan-5,9-dion
4S,7R,8S,9S,13(Z oder E),16S(Z))-4,8-Dihydroxy-16-(1-chfor-2-(2-pyridyl)ethenyl)-1-aza-7,9,13-trimethyl-5,5-(1,3-trimethylen)cyclohexadec-13-en-2,6-dion
(1RS, 3S(Z),7S,10R,11S,12S,16RS)-7,11-Dihydroxy-3-(1-chlor-2-(2-pyridyl)ethenyl)-10,12,16-trimethyl-8,8-(1,3-trimethylen)-4-aza-17-oxabicyclo[14.1.0]hepta-deca-5,9-dion
(4S,7R,8S,9S,13(Z oder E). 16S(Z))-4.8-Dihydroxy-9.13-dimethy)-7-ethy)-16-(1 -chtor-2-(2-pyridyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-chlor-2-(2-pyridyl)ethenyl)-8,8-(1,3-trimethylen)-4-aza-17-oxabicyclo[14.1.0]hepta-decan-5,9-dion

### Darstellung der Teilfragmente A und B:

Die Teilfragmente (Synthesebausteine) der allgemeinen Formeln A und B lassen sich wie in der DE 19751200.3 bzw. der korrespondierenden WO 99/07692 beschrieben herstellen.

### Darstellung der Teilfragmente C:

Die Darstellung der erfindungsgemäßen Teilfragmente der Formel C, in denen R⁸' ein Fluoratom bedeutet, kann, wie in den nachfolgenden Formelschemata innerhalb der Herstellung der erfindungsgemäßen Verbindungen der Beispiele 1 bis 4 angegeben ist, durchgeführt werden.
Durch Variation des (Hetero)arylrestes im Ausgangsprodukt im Reaktionsschritt a) (im vorliegenden Fall ist dies der 2-Methyl-4-thiazolylrest) kommt man zu entsprechend substituierten Bausteinen der Formel C und letztendlich Verbindungen der Formel I.
Die Darstellung von Fragmenten der Formel C, in denen R⁸' ein Chloratom bedeutet, ist innerhalb des Beispiels 5 beschrieben.
Stellt R^{8'} ein Bromatom dar, wird dieses analog wie ein Chloratom in den Fragmenten C eingeführt.

### Formelschemata zu den Beispielen 1 bis 4

### Beispiel 1

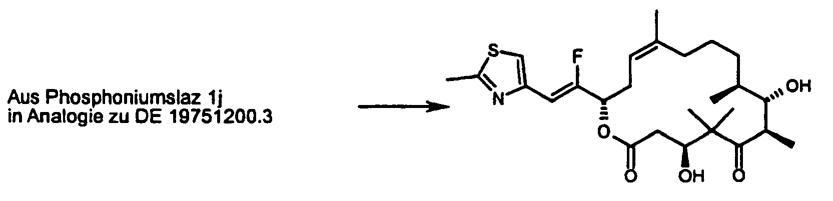

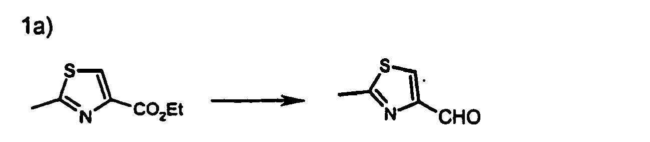

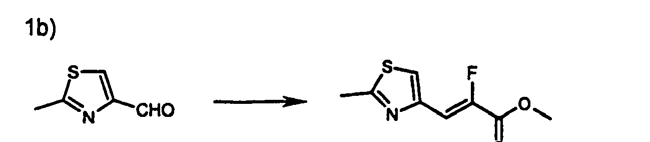

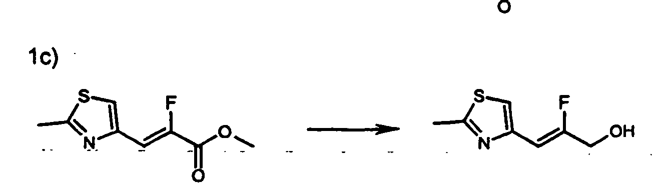

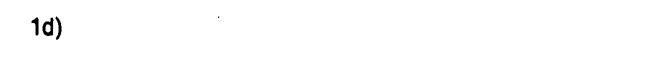

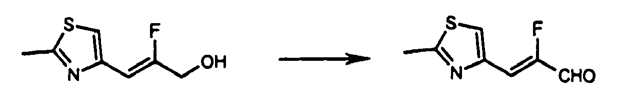

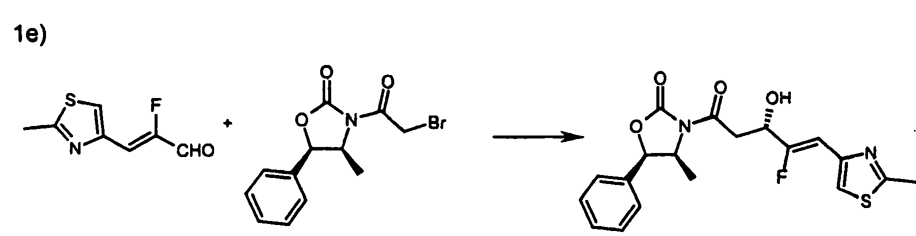

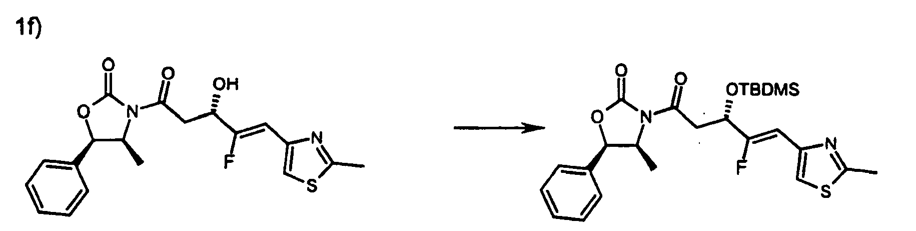

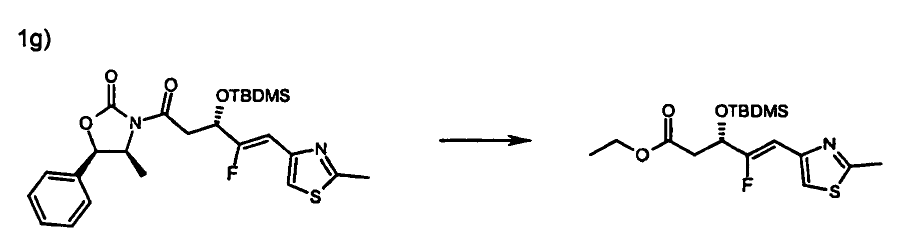

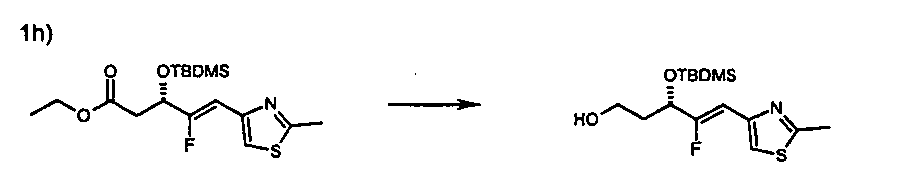

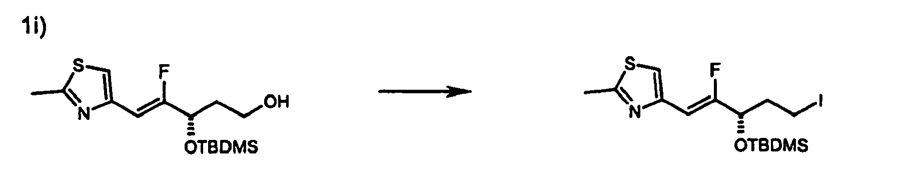

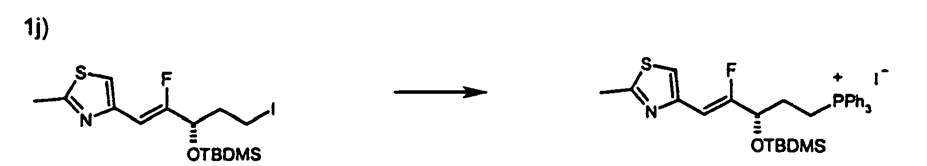

### Beispiel 2

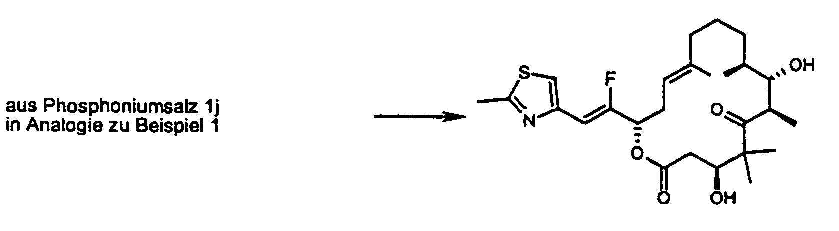

### Beispiel 3

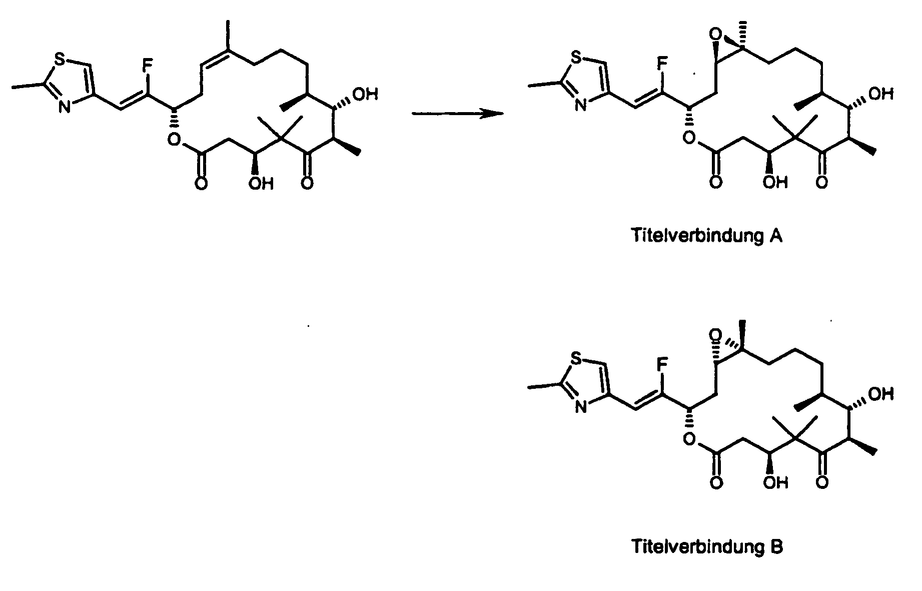

### Beispiel 4

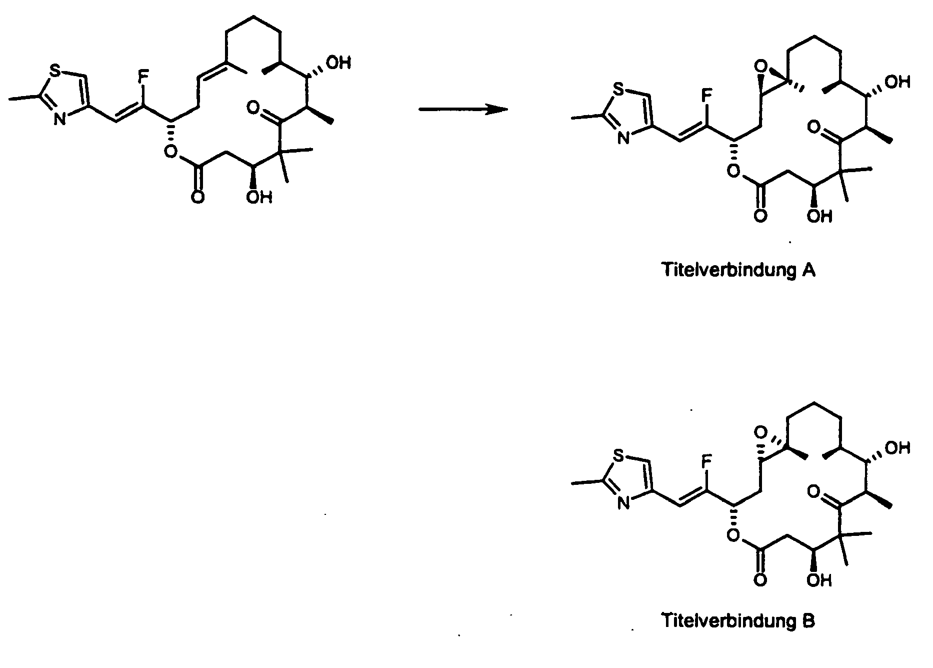

Die vorliegende Erfindung betrifft neben den Verbindungen der allgemeinen Formel I außerdem die neuen C13-C16-Epothilon-Bausteine der allgemeinen Formel C als Zwischenprodukte 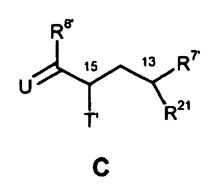 worin
- R⁸': ein Fluor- oder Chloratom oder eine Cyanogruppe und
- R⁷': ein Wasserstoffatom,
- T': eine Gruppe OR²⁰, wobei R²⁰ ein Wasserstoffatom oder eine Schutzgruppe PG² ist, Halogen oder eine Azido- oder eine geschützte Aminogruppe,
- R²¹: eine Hydroxygruppe, Halogen, eine geschützte Hydroxygruppe OPG³, ein Phosphoniumhalogenidrest PPh₃⁺Hal⁻ (Ph = Phenyl; Hal = F, Cl, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
- U: eine Gruppierung CR¹⁰R¹¹,
wobei
R¹⁰, R¹¹ gleich oder verschieden sind und für Wasserstoff, einen C₁-C₂₀-Alkyl-, Aryl-, C₇-C₂₀-Aralkylrest oder
R¹⁰ und R¹¹ zusammen mit dem Methylenkohlenstoffatom gemeinsam für einen 5- bis 7-gliedrigen carbocyclischen Ring stehen,
bedeuten, ausgenommen die Verbindungen (E)-3-Fluor-1-phenyl-hex-2-en-4,6-diol und 2-Brom-3-(tert.-butyldimethylsilyloxy)-1-penten-5-ol.

Erfindungsgemäß sind solche Verbindungen der allgemeinen Formel C bevorzugt,
worin
R^{8'} für ein Fluor-, Chlor- oder Bromatom, und/oder
U für ein Sauerstoffatom steht, und/oder
der für R¹⁰ und/oder R¹¹ stehende Arylrest für einen gegebenenfalls mit 1 bis 3 Resten, ausgewählt aus der Gruppe der Substituenten Halogen, freie Hydroxygruppe oder geschützte Hydroxygruppe OPG⁵, CO₂H, CO₂-Alkyl, C₁-C₄-Alkyl, Azido, Nitro, Nitril, Amino (NH₂), substituierten Phenylrest oder für einen gegebenenfalls mit 1 bis 2 C₁-C₄-Alkylresten substituierten 5- oder 6-gliedrigen Heteroarylrest, insbesondere für einen aus der Gruppe 2-, 3-Furanyl-, 2-, 3-, 4-Pyridinyl-, 2-, 4-, 5-Thiazolyl-, 2-, 4- und 5-lmidazolylrest, der gegebenenfalls durch 1 oder 2 C₁-C₄-Alkylreste substituiert ist, ausgewählten Substituenten steht und/oder
PG² und PG³ aus der Gruppe der Substituenten Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl-, para-Nitrobenzyl-, para-Methoxybenzyl-, Acetyl-, Propionyl-, Butyryl- und Benzoylrest ausgewählt sind,
insbesondere PG² ein tert.-Butyldimethylsilyl-, Acetyl, Benzoyl-, Benzyl-, Tetrahydropyranyl-Rest ist.

Als Schutzgruppen PG⁴ und PG⁵ kommen alle schon vorstehend für PG² und PG³ angegebenen Schutzgruppen in Frage.

Die vorliegende Erfindung betrifft außerdem Teilfragmente der allgemeinen Formel BC 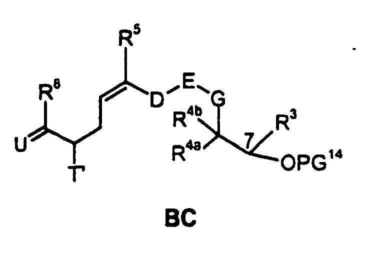 worin R³, R^{4a}, R^{4b}, R⁵, R⁸, D, E, G, T' und U die bereits genannten Bedeutungen haben und PG¹⁴ ein Wasserstoffatom oder eine Schutzgruppe PG darstellt.

Die vorliegende Erfindung betrifft außerdem noch Teilfragmente der allgemeinen Formel ABC 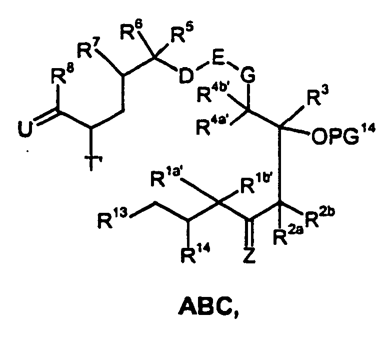 worin R^{1a}', R^{1b}', R^{2a}', R^{2b}', R³, R^{4a}', R^{4b}', R⁵, R⁶, R⁷, R⁸, R¹³, R¹⁴, D, E, G, T', U und Z die bereits genannten Bedeutungen haben.

### Darstellung der Teilfragmente ABC und deren Zyklisierung zu 1:

Die Darstellung und Zyklisierung erfolgt ebenfalls analog wie in der DE 19751200.3 bzw. der korrespondierenden WO 99/07692 beschrieben, wobei nunmehr Fragment C als Substituent R^{8'} insbesondere ein Fluor-, Chlor- oder Bromatom aufweist:
Teilfragmente der allgemeinen Formel AB 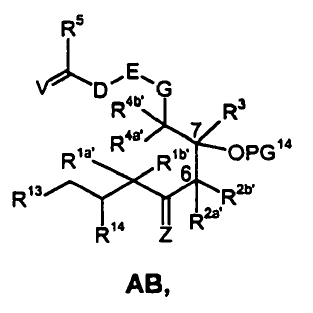 worin R^{1a'}, R^{1b'}, R^{2a'}, R^{2b'}, R³, R^{4a'}, R^{4b'}, R⁵, R¹³, R¹⁴, D, E, G, V und Z die bereits genannten Bedeutungen haben und PG¹⁴ ein Wasserstoffatom oder eine Schutzgruppe PG darstellt, werden aus den zuvor beschriebenen Fragmenten A und B nach dem in Schema 1 gezeigten Verfahren erhalten.

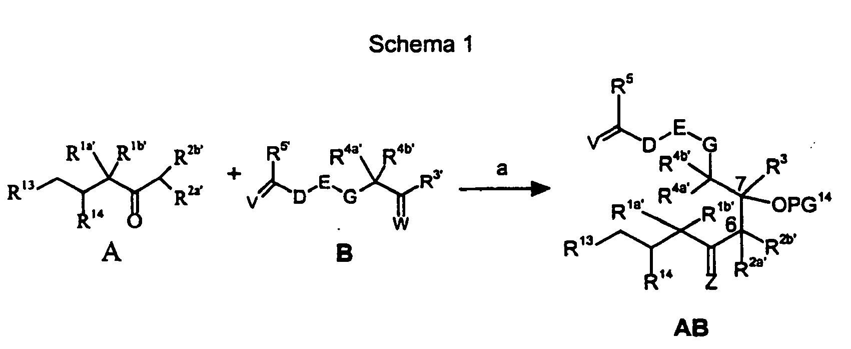

### Schritt a (A + B → AB):

Die Verbindung B, worin W die Bedeutung eines Sauerstoffatomes hat und eventuell vorhandene zusätzliche Carbonylgruppen geschützt sind, wird mit dem Enolat einer Carbonylverbindung der allgemeinen Formel A alkyliert. Das Enolat wird durch Einwirkung starker Basen wie z.B. Lithiumdiisopropylamid, Lithiumhexamethyldisilazan bei niedrigen Temperaturen hergestellt.

Teilfragmente der allgemeinen Formel ABC 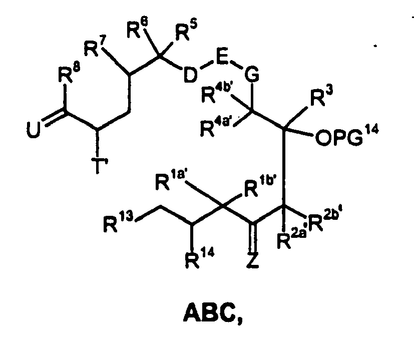 worin R^{1a'}, R^{1b'}, R^{2a'}, R^{2b'}, R³, R^{4a'}, R^{4b'}, R⁵, R⁶, R⁷, R⁸, R¹³, R¹⁴, D, E, G, T', U und Z die bereits genannten Bedeutungen haben, werden aus den zuvor beschriebenen Fragmenten AB und C nach dem in Schema 2 gezeigten Verfahren erhalten.

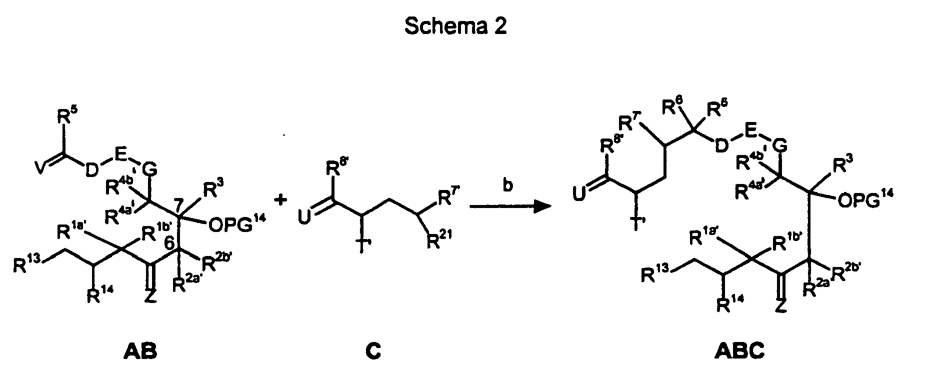

### Schritt b (AB + C → ABC):

Die Verbindung C, in der R²¹ die Bedeutung eines Wittigsalzes hat und eventuell vorhandene zusätzliche Carbonylgruppen geschützt sind, wird durch eine geeignete Base wie z.B. n-Butyllithium, Lithiumdiisopropylamid, Kalium-tert.butanolat, Natrium- oder Lithium-hexamethyldisilazid deprotoniert und mit einer Verbindung AB, worin V die Bedeutung eines Sauerstoffatomes hat, umgesetzt.

### Schritt c (ABC → 1):

Die Verbindungen ABC, in denen R¹³ eine Carbonsäure CO₂H darstellt, T' für OR²⁰ steht und R²⁰ ein Wasserstoffatom darstellt, setzt man nach den, dem Fachmann bekannten Methoden für die Bildung großer Macrolide zu Verbindungen der Formel 1, in denen T-Y die Bedeutung O-C(=O) besitzt, um. Bevorzugt wird die in "Reagents for Organic Synthesis, Vol. 16, p 353" beschriebene Methode unter Verwendung von 2,4,6-Trichlorbenzoesäurechlorid und geeigneten Basen wie z.B. Triethylamin, 4-Dimethylaminopyridin, Natriumhydrid.

### Schritt d (ABC → I):

Die Verbindungen ABC, in denen R¹³ eine Gruppe CH₂OH und R²⁰ ein Wasserstoffatom darstellt, lassen sich vorzugsweise unter Verwendung von Triphenylphosphin und Azodiestern wie beispielsweise Azodicarbonsäurediethylester zu Verbindungen der Formel I, in denen T-Y die Bedeutung von O-CH₂ hat, umsetzen. Die Verbindungen ABC, in denen R¹³ eine Gruppe CH₂OSO₂Alkyl oder CH₂OSO₂Aryl oder CH₂OSO₂Aralkyl und R²⁰ ein Wasserstoffatom darstellt, lassen sich nach Deprotonierung mit geeigneten Basen wie beispielsweise Natriumhydrid, n-Buthyllithium, 4-Dimethylaminopyridin, Hünig-Base, Alkalihexamethyldisilazanen zu Verbindungen der Formel I, in denen T-Y die Bedeutung von O-CH₂ hat, zyklisieren.

Alternativ zu vorstehender Route lassen sich Teilfragmente der allgemeinen Formel BC 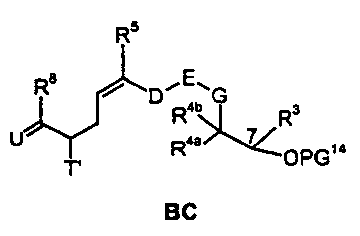 worin R³, R^{4a}, R^{4b}, R⁵, R⁸, D, E, T' und U die bereits genannten Bedeutungen haben und PG¹⁴ ein Wasserstoffatom oder eine Schutzgruppe PG darstellt, aus den zuvor beschriebenen Fragmenten B und C nach dem in Schema 3 gezeigten Verfahren erhalten.

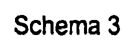

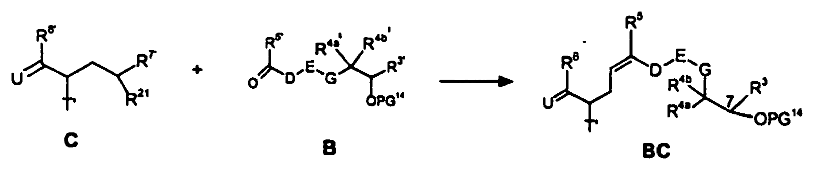

Zur Einführung einer Stickstoffunktion an C-15 kann wahlweise auf der Stufe C' (Fragment C mit T' = OR²⁰) oder BC' (Fragment BC mit T' = OR²⁰) an der Position 15 die Sauerstoffunktion direkt (C"' bzw. BC"' mit T' = Nf = Azid oder geschütztes Amin) oder über die Zwischenstufe eines Halogenatomes in eine Stickstoffunktion umgewandelt werden:

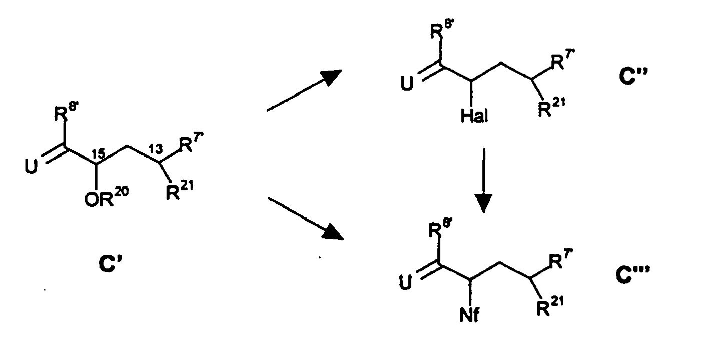

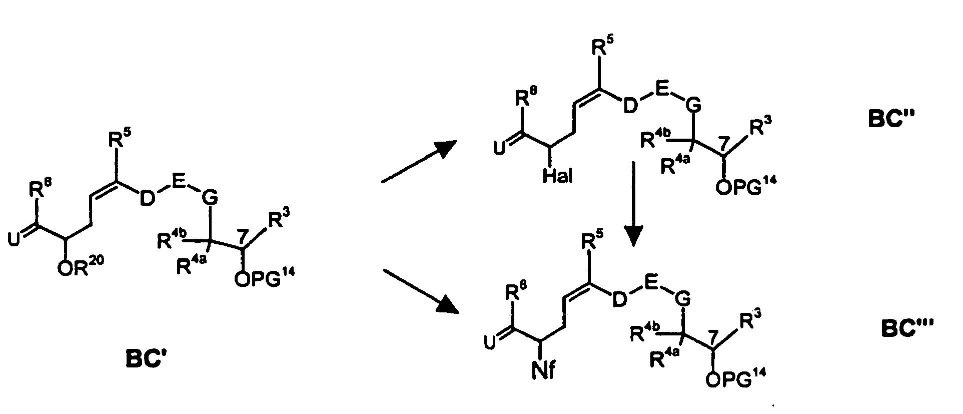

Stellt R²⁰ ein Wasserstoff dar, so kann die Hydroxylgruppe nach den dem Fachmann bekannten Verfahren in ein Halogenatom, vorzugsweise ein Chlor-, Brom,- oder lodatom überführt werden, das anschließend in eine Stickstoffunktion Nf, wobei Nf vorzugsweise ein Azid oder ein geschütztes Amin darstellt, überführt wird. Alternativ kann die Hydroxylgruppe an C-15 (R²⁰ in der Bedeutung von Wasserstoff) in eine Abgangsgruppe vorzugsweise in ein Alkyl- oder Aralkyl-sulfonat überführt und dieses dann durch ein Stickstoffnukleophil Nf substituiert werden.

Teilfragmente der allgemeinen Formel ABC 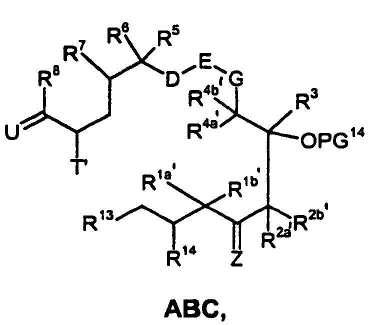 worin R^{1a'}, R^{1b'}, R^{2a'}, R^{2b'}, R³, R^{4a'}, R^{4b'}, R⁵, R⁶, R⁷, R⁸, R¹³, R¹⁴, D, E, G, T', U und Z die bereits genannten Bedeutungen haben, werden aus den zuvor beschriebenen Fragmenten BC und A nach dem in Schema 4 gezeigten Verfahren erhalten.

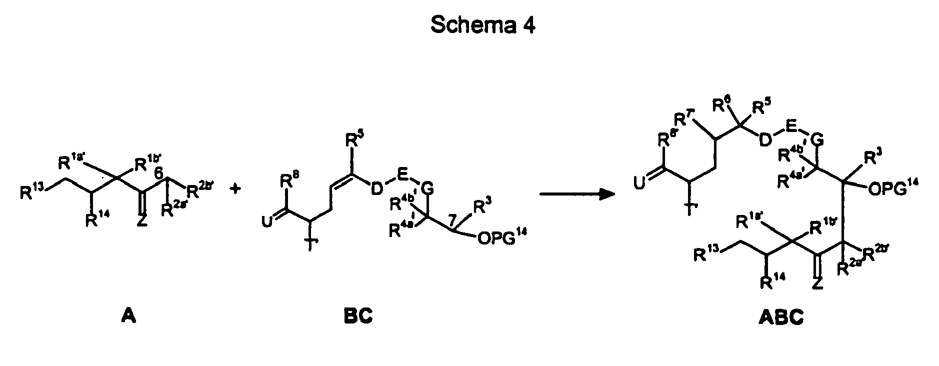

Die Einführung der Stickstoffunktion an C-15 kann wie bereits für C'" bzw. BC"' beschrieben, auch auf der Stufe ABC erfolgen. Die flexible. Funktionalisierung der beschriebenen Bausteine A, B und C gewährleistet auch eine von dem oben beschriebenen Verfahren abweichende Verknüpfungsreihenfolge, die zu den Bausteinen ABC führt. Diese Verfahren sind in der folgenden Tabelle zusammengestellt:

| Verknüpfungsmöglichkeiten | Verknüpfungsmethoden a bis e | Voraussetzungen |
|---|---|---|
| A + B → A-B | a: Aldol (siehe Schema 1) | Z = W = Sauerstoff |
| B + C → B-C | b: Wittig (analog Schema 2) e: McMurry | U = Sauerstoff und R²¹ = Wittigsalz oder Phosphinoxid oder Phosphonat |
| | | U = V = Sauerstoff |
| A + C → A-C | c: Veresterung (z. B. 2,4,6-Trichlorbenzoylchlorid / 4-Dimethylaminopyridin) | R¹³ = CO₂R^{13b} oder COHal und R²⁰ = Wasserstoff |
| | d: Veretherung (z.B. Mitsunobu) | R¹³ = CH₂OH und R²⁰ = Wasserstoff oder SO₂-Alkyl oder SO₂-Aryl oder SO₂-Aralkyl |
| | f: Amidbildung (z.B. mit (PhO)₂P(O)N₃) in . | R¹³ = CO₂R^{13b} oder COHal und R²⁰ = Wasserstoff |
| | Gegenwart einer Base (z.B. NaHCO₃) in einem inerten Lösungsmittel (z.B. DMF). | T = NH₂, NHR²⁴ |

Nach diesen Verfahren lassen sich die Bausteine A, B und C, wie in Schema 5 angegeben, verknüpfen:

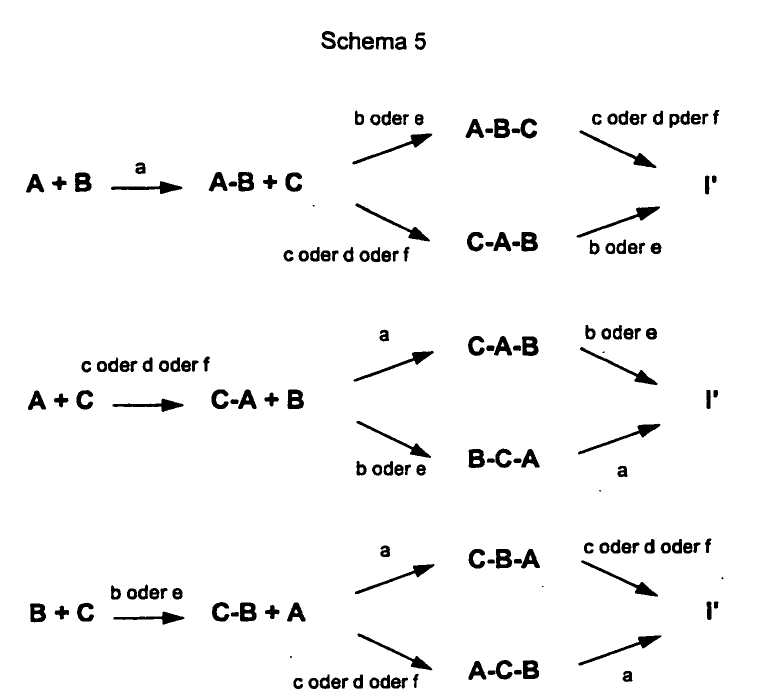

Freie Hydroxylgruppen in **I, I', A, B, C, AB, ABC** können durch Veretherung oder Veresterung, freie Carbonylgruppen durch Ketalisierung, Enoletherbildung oder Reduktion weiter funktionell abgewandelt sein.

Die Erfindung vorliegende Erfindung betrifft alle Stereoisomeren der beschriebenen und beanspruchten Verbindungen und auch deren Gemische.

### Biologische Wirkungen und Anwendungsbereiche der neuen Derivate:

Die neuen Verbindungen der Formel I sind wertvolle Pharmaka. Sie interagieren mit Tubulin, indem sie gebildete Mikrotubuli stabilisieren und sind somit in der Lage, die Zellteilung phasenspezifisch zu beeinflussen. Dies betrifft vor allem schnell wachsende, neoplastische Zellen, deren Wachstum durch interzelluläre Regelmechnismen weitgehend unbeeinflußt ist. Wirkstoffe dieser Art sind prinzipiell geeignet zur Behandlung maligner Tumoren. Als Anwendungsbereich seien beispielweise genannt die Therapie von Ovarial-, Magen-, Colon-, Adeno-, Brust-, Lungen-, Kopf- und Nacken-Karzinomen, dem malignen Melanom, der akuten lymphozytären und myelocytären Leukämie. Die erfindungsgemäßen Verbindungen eignen sich aufgrund ihrer Eigenschaften prinzipiell zur Anti-Angiogenese-Therapie sowie zur Behandlung chronischer entzündlicher Erkrankungen wie beispielsweise der Psoriasis oder der Arthritis. Zur Vermeidung unkontrollierter Zellwucherungen an sowie der besseren Verträglichkeit von medizinischen Implantaten lassen sie sich prinzipiell in die hierfür verwendeten polymeren Materialien auf- bzw. einbringen. Die erfindungsgemäßen Verbindungen können alleine oder zur Erzielung additiver oder synergistischer Wirkungen in Kombination mit weiteren in der Tumortherapie anwendbaren Prinzipien und Substanzklassen verwendet werden.
Als Beispiele seien genannt die Kombination mit
O Platinkomplexen wie z.B. Cisplatin, Carboplatin,
O interkalierenden Substanzen z.B. aus der Klasse der Anthracycline wie z.B. Doxorubicin oder aus der Klasse der Antrapyrazole wie z.B. CI-941,
O mit Tubulin interagierenden Substanzen z.B. aus der Klasse der Vinka-Alkaloide wie z.B. Vincristin, Vinblastin oder aus der Klasse der Taxane wie z.B. Taxol, Taxotere oder aus der Klasse der Makrolide wie z.B. Rhizoxin oder andere Verbindungen wie z.B. Colchicin, Combretastatin A-4,
O DNA Topoisomeraseinhibitoren wie z.B. Camptothecin, Etoposid, Topotecan, Teniposid,
O Folat- oder Pyrimidin-Antimetaboliten wie z.B. Lometrexol, Gemcitubin,
O DNA alkylierenden Verbindungen wie z.B. Adozelesin, Dystamycin A,
O Inhibitoren von Wachstumsfaktoren (z.B. von PDGF, EGF, TGFb, EGF) wie z.B. Somatostatin, Suramin, Bombesin-Antagonisten,
O Inhibitoren der Protein Tyrosin Kinase oder der Protein Kinasen A oder C wie z.B. Erbstatin, Genistein, Staurosporin, Ilmofosin, 8-Cl-cAMP,
O Antihormonen aus der Klasse der Antigestagene wie z.B. Mifepriston, Onapriston oder aus der Klasse der Antiöstrogene wie z.B. Tamoxifen oder aus der Klasse der Antiandrogene wie z.B. Cyproteronacetat,
O Metastasen inhibierenden Verbindungen z.B. aus der Klasse der Eicosanoide wie z.B. PGl₂, PGE₁, 6-Oxo-PGE₁ sowie deren stabiler Derivate (z.B. Iloprost, Cicaprost, Misoprostol),
O Inhibitoren onkogener RAS-Proteine, welche die mitotische Signaltransduktion beeinflussen wie beispielsweise Inhibitoren der Famesyl-Protein-Transferase,
O natürlichen oder künstlich erzeugten Antikörpern, die gegen Faktoren bzw. deren Rezeptoren, die das Tumorwachstum fördern, gerichtet sind wie beispielsweise der erbB2-Antikörper.

### In vitro Aktivität von Epothilon-Derivaten an humanen Tumorzellinien

a) IC₅₀-Werte [nM] für die Wachstumshemmung humaner MCF-7-Brust- und multi-drug-resistenter NCI/ADR-Karzinomzellinien von Epothilon-Derivaten mit 13Z-Olefin im Kristall-Violett-Assay im Vergleich zu Epothilon D.

**Tabelle 1:**

| Verbindung | MCF-7 | NCI/ADR | Selektivität* |
|---|---|---|---|
| Epothilon D | 23 | 50 | 2,2 |
| Taxol | 4,0 | >>100 | >>25 |
| Bsp.1 | 4,3 | 12 | 2,8 |
| Bsp. 5 | 5,1 | 37 | 7,3 |
| Bsp.9 | 5,0 | 10 | 2,0 |
| Bsp.13 | 5,8 | 28 | 4,8 |
| Bsp. 17 | 6,1 | 33 | 5,4 |

| | | | |
|---|---|---|---|
| *: Selektivität = IC₅₀-(NCI/ADR) : IC₅₀ (MCF-7) | | | |

Die Verbindungen der Beispiele 1, 9, 13 und 17 besitzen eine signifikat höhere Wirkstärke im Vergleich zur strukturell ähnlichen Referenzverbindung Epothilon D. Alle Verbindungen zeigen im Unterschied zu Taxol eine Wirkung an der multi-drug-resistenten Zellinie NCI/ADR.
b) IC₅₀-Werte [nM] für die Wachstumshemmung humaner MCF-7-Brust- und multi-drug-resistenter NCl/ADR-Karzinomzellinien von Epothilon-Derivaten mit 13,14-α-Epoxid im Kristall-Violett-Assay im Vergleich zu Epothilon B.

**Tabelle 2:**

| Verbindung | MCF-7 | NCl/ADR | Selektivität* |
|---|---|---|---|
| Epothilon B | 0,6 | 3,5 | 5,8 |
| Taxol | 4,0 | >> 100 | >>25 |
| Bsp. 3B | 0,3 | 1,4 | 4,7 |
| Bsp. 7A | 0,8 | 6,0 | 7,5 |
| Bsp. 10A | 2,1 | 3,9 | 1,9 |
| Bsp. 14A | 0,5 | 3,5 | 7,0 |
| Bsp. 20A | 0,6 | 4,6 | 7,6 |

| | | | |
|---|---|---|---|
| *: Selektivität = IC₅₀-(NCI/ADR): IC₅₀ (MCF-7) | | | |

Die Verbindungen der Beispiele 3B, 14A und 20A besitzen eine vergleichbare oder signifikat höhere Wirkstärke im Vergleich zur strukturell ähnlichen Referenzverbindung Epothilon B. Alle Verbindungen zeigen im Unterschied zu Taxol eine Wirkung an der multi-drug-resistenten Zellinie NCI/ADR. Verbindungen der Beispiele 3B und 10A zeigen eine verbesserte Selektivität an der multi-drug-resistenten Zellinie NCI/ADR im Vergleich zur Referenzverbindung Epothilon B.

Die Erfindung betrifft auch Arzneimittel auf Basis der pharmazeutisch verträglichen, d.h. in den verwendeten Dosen nicht toxischen Verbindungen der allgemeinen Formel 1, gegebenenfalls zusammen mit den üblichen Hilfs- und Trägerstoffen.
Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens oder Myrj, Magnesiumstearat, wäßrigen oder nicht wäßrigen Trägem, Paraffinderivaten, Netz-, Dispergier-, Emulgier-, Konservierungsmitteln und Aromastoffen zur Geschmackskorrektur (z.B. etherischen Ölen) gemischt werden.

Die erfindungsgemäßen Verbindungen können in Form der α-, β- oder γ-Cyclodextrinclathrate, vorliegen oder in Liposomen verkapselt sein.

Die Erfindung betrifft somit auch pharmazeutische Zusammensetzungen, die als Wirkstoff zumindest eine erfindungsgemäße Verbindung enthalten. Eine Dosiseinheit enthält etwa 0,1-100 mg Wirkstoff(e). Die Dosierung der erfindungsgemäßen Verbindungen liegt beim Menschen bei etwa 0,1-1000 mg pro Tag.

Die nachfolgenden Beispiele dienen der näheren Erläuterung der Erfindung, ohne sie darauf einschränken zu wollen.

### Beispiele für die Herstellung der erfindungsgemäßen Verbindungen der allgemeinen Formel I

### Beispiel 1

### (4S,7R,8S,9S,13(Z),16S(Z))-4,8-Dihydroxy-16-(1-fluor-2-(2-methyl-4-thiazolyl)ethenyl)-1-oxa-5,5,7,9,13-pentamethyl-cyclohexadec-13-en-2,6-dion

In Analogie zu den in DE 19751200.3 beschriebenen Verfahren erhält man aus dem Phosphoniumsalz aus Beispiel 1j 36.5 mg der Titelverbindung als schwach gelbgefärbtes Öl.
¹H-NMR (DMSO-d6): δ = 0.93 (3H), 0.94 (3H), 1.10 (3H), 0.8-1.4 (6H), 1.21 (3H), 1.62 (1 H), 1.66 (3H) 1.87 (1H), 2.24 (1H), 2.3-2.6 (3H), 2.64 (3H), 2.73 (1 H), 3.13 (1H), 3.53 (1H), 4.22 (1H), 5.16 (1H), 5.36 (1 H), 6.22 (1H), 7.46 (1H) ppm.

### Beispiel 1a

### 2-Methylthiazol-4-carbaldehyd

Zu einer Lösung aus 60 g 2-Methylthiazol-4-carbonsäureethylester in 1070 ml Methylenchlorid tropft man bei -75°C unter Stickstoff langsam 476 ml einer 1.2 molaren Lösung von DIBAH in Toluol. Man rührt 2 Stunden nach. Dann tropft man langsam 150 ml Isopropanol, anschließend 230 ml Wasser dazu, entfernt das Kältebad und rührt bei 25°C 2 Stunden kräftig nach. Der entstandene Niederschlag wird abgesaugt und mit Essigester nachgewaschen. Das Filtrat wird im Vakuum eingeengt und der so erhaltene Rückstand durch Chromatographie an Kieselgel gereinigt. Mit Hexan/Ether 1:1 erhält man 35.6 g der Titelverbindung als farbloses Öl.
¹H-NMR (CDCl₃): δ = 2.8 (3H), 8.05 (1H), 10.0 (1H) ppm.

### Beispiel 1b

### (2Z)-3-(2-Methylthiazol-4-yl)-2-fluor-2-propensäureethylester

Zu einer Suspension von 9.64 g Natriumhydrid (60%ige Suspension in Mineralöl) in 120 ml Dimethoxyethan fügt man bei 0°C eine Lösung von 58.7 g Phosphonofluoressigsäuretriethylester in 120 ml Dimethoxyethan. Man rührt 40 Minuten und tropft dann eine Lösung von 15.4 g des unter Beispiel 1a hergestellten Aldehyds in 120 ml Dimethoxyethan zu und rührt anschließend 2 Stunden bei 24°C unter Argon. Nach dem versetzen mit wäßriger Ammoniumchlorid-Lösung extrahiert man dreimal mit Essigester, wäscht die organische Phase mit verdünnter Natriumchlorid-Lösung, trocknet über Natriumsulfat und dampft im Vakuum ein. Das Gemisch der Z- und E-konfigurierten Olefine trennt man durch Säulenchromatographie an Kieselgel. Mit Hexan/Essigester 4:6 bis 3:7 erhält man neben 3.9 g einer Mischfraktion 7.5 g (2E)-3-(2-Methylthiazol-4-yl)-2-fluor-2-propensäureethylester und 7.3 g der Titelverbindung als farblose Öle.
¹H-NMR (CDCl₃): δ = 1.36 (3H), 2.73 (3H), 4.33 (2H), 7.20 (1 H), 7.67 (1 H) ppm.

### Beispiel 1 c

### (2Z)-3-(2-Methylthiazol-4-yl)-2-fluor-2-propen-1-ol

Zu einer Lösung aus 18,8 g des vorstehend hergestellten Esters in 260 ml Toluol tropft man bei -70°C unter Stickstoff 136 ml einer 1.2 molaren Lösung von DIBAH in Toluol. Nach einer Stunde tropft man langsam 55 ml Isopropanol und anschließend 68 ml Wasser dazu und rührt 2 Stunden kräftig nach. Der entstandene Niederschlag wird abgesaugt und gut mit Essigester gewaschen. Das Filtrat wird im Vakuum eingeengt, der so erhaltene Rückstand durch Chromatographie an Kieselgel gereinigt. Mit Hexan/0-70% Essigester erhält man 13,4 g der Titelverbindung als farbloses Öl.
¹H-NMR (CDCl₃): δ = 2.69 (3H), 3.71 (1H). 4.27 (2H), 6.18 (1 H), 7.35 (1 H) ppm.

### Beispiel 1d

### (2Z)-3-(2-Methylthiazol-4-yl)-2-fluor-2-propenal

Zu einer Lösung aus 13,28 g des vorstehend hergestellten Alkohols in 200 ml Toluol gibt man portionsweise insgesamt 53.3 g Braunstein und rührt kräftig unter Stickstoff 4 Stunden nach. Braunstein wird über Celite abgesaugt, gut mit Essigester gewaschen, und das Filtrat im Vakuum eingeengt. Der so erhaltene Rückstand durch Chromatographie an Kieselgel gereinigt. Mit Hexan/0-30% Essigester erhält man 9,93 g der Titelverbindung als farbloses Öl.
¹H-NMR (CDCl₃): δ = 2.77 (3H), 6.95 (1H), 7.88 (1H), 9.36 (1 H) ppm.

### Beispiel 1e

### (3S,4Z)-5-(2-Methylthiazol-4-yl)-1-[(4S,5R)-4-methyl-5-phenyl-1,3-1,3-oxazolidin-2-on-3-yl]-3-hydroxy-4-fluor-4-penten-1-on

Es werden 17.6 g wasserfreies Chrom(II)chlorid in 210 ml THF unter Argon vorgelegt und mit 766 mg Lithiumiodid versetzt. Anschließend wird eine Lösung aus 9,8 g des vorstehend hergestellten Aldehyds und 18.8 g (4S,5R)-3-(Bromacetyl)-4-methyl-5-phenyloxazolidin-2-on in 38 ml THF dazugetropft. Es wird 3 Stunden nachgerührt. Man gibt 150 ml gesättigte Natriumchlorid-Lösung dazu, rührt 30 Minuten und trennt die Phasen. Die wäßrige Phase wird zweimal mit Essigester extrahiert, die vereinigten organischen Phasen einmal mit Wasser, einmal mit gesättigter Natriumchlorid-Lösung extrahiert. Die organische Phase wird über Natriumsulfat getrocknet, abfiltriert und das Filtrat im Vakuum eingeengt. Der so erhaltene Rückstand wird durch Chromatographie an Kieselgel gereinigt. Mit Hexan/0-60% Essigester erhält man 11,22 g der Titelverbindung neben 9,53 g einer Mischfraktion und 1,8 g der entsprechenden diastereomeren Titelverbindung als helle Öle.
¹ H-NMR (CDCl₃): δ = 0.93 (3H), 2.71 (3H), 3.36 (1H), 3.52 (1H), 4.82 (1H), 5.72 (1H), 6.29 (1 H), 7.2-7.5 (6H) ppm.

### Beispiel 1f

### (3S,4Z)-5-(2-Methylthiazol-4-yl)-1 -[(4S,5R)-4-methyl-5-phenyl-1,3-1,3-oxazolidin-2-on-3-yl]-3-(tert.-butyl-dimethylsilyloxy)-4-fluor-4-penten-1-on

Zu einer Lösung aus 11,2 g der vorstehend hergestellten Titelverbindung in 86 ml Methylenchlorid tropft man bei -70°C unter Stickstoff 4,68 ml Lutidin und rührt 5 Minuten nach. Dann wird langsam 8,56 ml tert.-Butyldimethylsilyltrifluormethansulfonat zugetropft. Nach einer Stunde versetzt man mit gesättigter Ammoniumchloridlösung und läßt das Reaktionsgemisch auf 25°C erwärmen. Man verdünnt mit Ether, wäscht einmal mit Wasser und einmal mit gesättigter Natriumchlorid-Lösung. Die organische Phase wird über Natriumsulfat getrocknet und im Vakuum eingeengt. Der so erhaltene Rückstand wird durch Chromatographie an Kieselgel gereinigt. Mit Hexan/Ether 1:1 erhält man 9,3 g der Titelverbindung als farbloses Öl.
¹H-NMR (CDCl₃): δ = 0.15 (6H), 0.90 (9H), 0.93 (3H), 2.70 (3H), 3.27 (1H), 3.57 (1H), 4.77 (1H), 4.90 (1 H), 5.66 (1H), 6.15 (1H), 7.26-7.50 (6H) ppm.

### Beispiel 1g

### (3S,4Z)-5-(2-Methylthiazol-4-yl)-3-(tert-butyl-dimethylsilyloxy)-4-fluor-4-pentensäureethylester

Zu einer Lösung aus 15,5 g der vorstehend hergestellten Titelverbindung in 70 ml Ethanol gibt man 2,8 ml Titan(IV)ethylat und kocht 4 Stunden am Rückfluß unter Stickstoff. Die Reaktionslösung wird im Vakuum eingeengt, der Rückstand in 70 ml Essigester aufgenommen, mit Wasser versetzt und 20 Minuten gerührt. Titanoxid wird abgesaugt, gut mit Essigester gewaschen und das Filtrat im Vakuum eingeengt. Der Rückstand wird mit Hexan versetzt, die Kristalle werden abgesaugt und zweimal mit Hexan gewaschen. Das Filtrat wird im Vakuum eingeengt. Der so erhaltenen Rückstand wird durch Chromatographie an Kieselgel gereinigt. Mit Hexan/0-50% Essigester erhält man 11,9 g der Titelverbindung als farbloses Öl.
¹H-NMR (CDCl₃): δ = 0.11 (6H), 0.91 (9H), 1.26 (3H), 2.70 (2H), 2.71 (3H), 4.15 (2H), 4.74 (1H), 6.12 (1H), 7.37 (1H) ppm.

### Beispiel 1h

### (3S,4Z)-5-(2-Methylthiazol-4-yl)-3-(tert.-butyl-dimethylsilyloxy)-4-fluor-4-penten-1-ol

Zu einer Lösung aus 10.5 g der vorstehend hergestellten Titelverbindung in 250 ml Toluol tropft man unter Stickstoff bei-70°C langsam 58.6 ml einer 1.2 molaren Lösung von DIBAH in Toluol und rührt eine Stunde bei-30°C. Man tropft langsam bei-70°C 10 ml Isopropanol dazu, anschließend 22 ml Wasser und rührt bei 25°C 2 Stunden kräftig nach. Der Niederschlag wird abgesaugt, gut mit Essigester gewaschen, und das Filtrat im Vakuum eingeengt. Der so erhaltene Rückstand wird durch Chromatographie an Kieselgel gereinigt. Mit Hexan/0-70% Essigester erhält man 7.73 g der Titelverbindung als gelbes Öl.
¹H-NMR (CDCl₃): δ = 0.12 (3H), 0.16 (3H), 0.93 (9H), 2.00 (2H), 2.72 (3H), 3.77 (1H), 3.86 (1H), 4.53 (1H), 6.13 (1H), 7.36 (1H) ppm.

### Beispiel 1i

### (3S.4Z)-5-(2-Methylthiazol-4-yl)-3-(tert.-butyl-dimethylsilyloxy)-1-iod-4-fluor-4-penten

Zu einer Lösung aus 7,31 g Triphenylphosphin in 106 ml Methylenchlorid gibt man 1,90 g Imidazol. Zu dieser Lösung gibt man 7,07 g lod, läßt 10 Minuten rühren und tropft dann eine Lösung aus 7.7 g der vorstehend hergestellten Titelverbindung in 28 ml Methylenchlorid zu und rührt 30 Minuten. Es wird abfiltriert, gut mit Ether gewaschen, und das Filtrat im Vakuum eingeengt. Der so erhaltene Rückstand wird durch Chromatographie an Kieselgel gereingt. Mit Hexan/0-10% Essigester erhält man 8,2 g der Titelverbindung als farbloses Öl.
¹ H-NMR (CDCl₃): δ = 0.11 (3H), 0.16 (3H), 0.93 (9H), 2.23 (2H), 2.71 (3H), 3.24 (2H), 4.36 (1H), 6.12 (1H), 7.36 (1H) ppm.

### Beispiel 1j

### (3S,4Z)-5-(2-Methylthiazol-4-yl)-3-(tert.-butyl-dimethylsilyloxy)-4-fluor-4-penten-triphenylphosphoniumiodid

Man mischt 8,16 g der vorstehend hergestellten Titelverbindung mit 5,33 g Triphenylphosphin und rührt unter Stickstoff bei 100°C 2 Stunden. Nach dem Abkühlen wird der feste Rückstand zweimal mit Ether und wenig Essigester verrieben, wobei die überstehende Lösung abpipettiert wird. Dann wird der Rückstand in Methanol gelöst und im Vakuum eingeengt. Der feste Schaum wird wieder in wenig Methanol gelöst, mit Toluol versetzt und wieder im Vakuum eingeengt. Dieser Vorgang wird zweimal wiederholt, anschließend wird der Rückstand im Hochvakuum getrocknet. Man erhält 12,4 g der Titelverbindung als feste Substanz.
Fp.: 70-72°C

### Beispiel 2

### (4S,7R,8S,9S,13(E),16S(Z))-4,8-Dihydroxy-16-(1-fluor-2-(2-methyl-4-thiazolyl)ethenyl)-1-oxa-5,5,7, 9,13-pentamethyl-cyclohexadec-13-en-2,6-dion

In Analogie zu Beispiel 1 erhält man aus dem Phosphoniumsalz aus Beispiel 1j 41,5 mg der Titelverbindung als schwach gelbgefärbtes Öl.
¹H-NMR (CDCl₃): δ = 0.99 (3H), 1.05 (3H), 0.8-1.4 (6H), 1.16 (3H), 1.30 (3H), 1.5-1.7 (1H). 1.76 (1H). 2.00 (1H). 2.18 (1H). 2.43 (1H). 2.56 (1H). 2.63 (2H), 2.70 (3H), 3.25 (1H), 3.40 (2H), 3.66 (1H), 4.30 (1H), 5.13 (1H), 5.61 (1H), 6.18 (1H), 7.48 (1H) ppm.

### Beispiel 3

### (1R,3S(Z),7S,10R,11S,12S,16S)-7,11-Dihydroxy-3-(1-fluor-2-(2-methyl-4-thiazolyl)ethenyl)-8,8,10,12,16-pentamethyl-4,17-dioxabicyclo[14.1.0]heptadecan-5,9-dion (A) und

(1S,3S(Z),7S,10R,11S,12S,16R)-7,11-Dihydroxy-3-(1-fluor-2-(2-methyl-4-thiazolyl)ethenyl)-8,8,10.12,16-pentamethyl-4,17-dioxabicyclo[14.1.0]heptadecan-5,9-dion (B)

Zu 15 mg der in Beispiel 1 hergestellten Titelverbindung in 0,3 ml Acetonitril gibt man bei 0°C unter Argon 0,172 ml EDTA und 0,288 ml 1,1,1-Trifluoraceton, anschließend eine Mischung aus 35,0 mg Oxon und 20,2 mg Natriumhydrogencarbonat. Man rührt 3,5 Stunden bei 0°C. Man versetzt mit 2 ml Natriumthiosulfatlösung, rührt 5 Minuten und verdünnt mit 80 ml Essigester. Die organische Phase wird einmal mit halbgesättigter Natriumchlorid-Lösung gewaschen, über Natriumsulfat getrocknet und im Vakuum eingeengt. Der so erhaltene Rückstand wird durch zweifache präparative Dickschichtchromatographie gereinigt. Mit Methylenchlorid/Essigester 2:8 (1.PDC) bzw. Methylenchlorid/methanol 98:2 (2.PDC), erhält man 2,5 mg der Titelverbindung A als unpolare Komponente und 6 mg der Titelverbindung B als polare Komponente als farblose Öle.
¹H-NMR (MeOH-d4) von A: δ = 0.99 (3H), 1.04 (3H), 0.8-1.9 (11H), 1.30 (3H), 1.41 (3H), 2.17 (2H), 2.47 (1H), 2.58 (1H), 2.71 (3H), 3.01 (1H), 3.2-3.4 (1H), 3.78 (1H), 4.33 (1 H), 4.8-5.0 (1 H), 5.71 (1H). 6.26 (1H). 7.53(1 H) ppm.
¹H-NMR (MeOH-d4) von B: δ = 0.99 (3H), 1.01 (3H), 0.9-1.9 (6H), 1.12 (3H), 1.30 (3H), 1.33 (3H), 1.95-2.10 (4H), 2.18 (2H), 2.41 (1H). 2.48 (1 H), 2.70 (3H), 3.2-3.4 (1H), 3.63 (1H), 3.85 (1H), 4.34 (1H), 5.34 (1H), 5.63 (1H), 6.19 (1H), 7.51 (1H) ppm.

### Beispiel 4

### (1R,3S(Z),7S,10R,11S,12S,16R)-7,11-Dihydroxy-3-(1-fluor-2-(2-methyl-4-thiazolyl)ethenyl)-8,8,10,12,16-pentamethyl-4,17-dioxabicycio[14.1.0]heptadecan-5,9-dion (A) und

### (1S,3S(Z),7S,10R,11S,12S,16S)-7,11-Dihydroxy-3-(1-fluor-2-(2-methyl-4-thiazolyl)ethenyl)-8,8,10,12,16-pentamethyl-4,17-dioxabicyclo[14.1.0]heptadecan-5,9-dion (B)

In Analogie zu Beispiel 3 erhält man aus 38 mg der in Beispiel 2 hergestellten Titelverbindung 8.8 mg der Titelverbindung A als unpolare Komponente und 9.0 mg der Titelverbindung B als polare Komponente als farblose Öle.
¹H-NMR (MeOH-d4) von A: δ = 0.95 (3H), 1.00 (3H), 0.8-1.65 (8H), 1.14 (3H), 1.28 (3H), 1.33 (3H), 1.91 (1H). 2.18 (2H), 2.54 (2H), 2.68 (3H), 3.05 (1H), 3.43 (1H), 3.63 (1H), 4.26 (1H), 5.66 (1H), 6.24 (1H), 7.52 (1H) ppm.
¹H-NMR (MeOH-d4) von B: δ = 0.95 (3H), 1.02 (3H), 0.8-1.7 (8H), 1.14 (3H), 1.29 (3H), 1.32 (3H), 1.77 (1H), 2.09 (1H), 2.23 (1H), 2.5-2.65 (2H), 2.69 (3H), 3.14 (1 H), 3.33 (1H), 3.70 (1H), 4.38 (1H), 5.66 (1H), 6.21 (1H), 7.51 (1H) ppm.

### Beispiel 5

### (4S,7R,8S,9S,13(Z),16S(Z))-4,8-Dihydroxy-16-(1-chlor-2-(2-methyl-4-thiazolyl) ethenyl)-1-oxa-5,5, 7 ,9, 13-pentamethyl-cyclohexadec-13-en-2,6-dion

### Beispiel 5a

### 2-Methylthiazol-4-carbaldehyd

50 g Ethyl-2-methylthiazole-4-carboxylat werden in 700 ml Methylenchlorid gelöst, auf -70°C gekühlt und vorsichtig mit 390 ml Düsobutylaluminiumhydrid (1,2 molar in Toluol) versetzt. Nach 1h war die Umsetzung noch nicht vollständig, es wurden nochmals 40 ml Diisobutylaluminiumhydrid zugetropft. Nach weiteren 40 Min. wurde das Reaktionsgemisch vorsichtig mit 100 ml Isopropanol versetzt und 15 Minuten gerührt. Anschließend werden 215 ml Wasser zugetropft und das Kühlbad entfernt. Nach 2h wurde der kristalline Niederschlag über ein Fritte abgesaugt, mit Essigester gewaschen und das Filtrat im Vakuum eingedampft. Man erhält 36,1 g der Titelverbindung.
¹H-NMR (CDCl₃): δ = 2,8 (3H), 8,05 (1 H), 10,00 (1 H) ppm.

### Beispiel 5b

### (2Z)-3-(2-Methylthiazol-4-yl)-2-chlor-2-propensäureethylester

Zu einer Suspension von 9 g Natriumhydrid (60%ige Suspension in Mineralöl) in 165 ml Dimethoxyethan fügt man, innerhalb von 15 Minuten, bei 0°C unter Stickstoff, eine Lösung von 97 g Triethyl-2-chloro-2-phosphonoacetat in 165 ml Dimethoxyethan. Man rührt 45 Minuten bei 24°C und tropft dann eine Lösung von 31,8 g der unter Beispiel 5a hergestellten Titelverbindung in 165 ml Dimethoxyethan zu und rührt anschließend 1 Stunde nach. Nach dem Versetzen mit wäßriger Ammoniumchlorid-Lösung extrahiert man 3x mit Essigester, wäscht die organische Phase mit verdünnter Natriumchlorid-Lösung, trocknet über Natriumsulfat und dampft im Vakuum ein. Das Gemisch der Z- und E-konfigurierten Olefine trennt man durch Säulenchromatographie an Kieselgel. Nach Säulenchromatographie mit Hexan/Essigester 10-30% und anschließender Kristallisation aus Hexan erhält man 32 g der Titelverbindung. (FP. 61°C-62°C)
¹H-NMR (CDCl₃): δ = 1,37 (3H), 2,76 (3H), 4,33 (2H), 8,13 (1H), 8,18 (1H) ppm.

### Beispiel 5c

### (2Z)-3-(2-Methylthiazol-4-yl)-2-chlor 2-propen-1-ol

In Analogie zu Beispiel 1 c erhält man aus 32 g des in Beispiel 5b hergestellten Esters, in Toluol als Lösungsmittel, 22,8 g der Titelverbindung.

### Beispiel 5d

### (2Z)-3-(2-Methylthiazol-4-yl)-2-chlor-2-propenat

9,8 g des in Beispiel 5c hergestellten Alkohols werden in 500 ml Methylenchlorid gelöst und mit 26,14 ml Triethylamin versetzt. Anschließend werden 16,14 g SO₃-Pyridin-Komplex addiert und 1h bei 24°C gerührt. Nun wird mit Ammoniumchlorid-Lösung versetzt, mit Essigester extrahiert, die organische Phase mit gesättigter Natriumchlorid-Lösung gewaschen und über Natriumsulfat getrocknet. Nach eindampfen im Vakuum erhält man 10,03 g der Titelverbindung.

### Beispiel 5e

### (3S,4Z)-5-(2-Methylthiazol-4-yl)-1-[(4S,5R)-4-methyl-5-phenyl-1,3-oxazolidin-2-on-3-yl]-3-hydroxy-4-chlor-4-penten-1-on

In Analogie zu Beispiel 1e erhält man aus 3,3 g des in Beispiel 5d hergestellten Aldehyds 1,4 g der Titelverbindung.
¹H-NMR (CDCl₃): δ = 0,95 (3H), 2,7 (3H), 3,38 (1H), 3,45-3,55 (1H), 3,56 (1H), 4,8 (1 H), 4,89 (1H), 5,7 (1H), 7,18 (1H), 7,28-7,48 (5H), 7,83 (1H) ppm.

### Beispiel 5f

### (3S,4Z)-5-(2-Methylthiazol-4-yl)-1-[(4S,5R)-4-methyl-5-phenyl-1,3-oxazolidin-2-on-3-yl]-3-(tert.-butyl-dimethylsilyloxy)-4-chlor-4-penten-1-on

In Analogie zu Beispiel 1f erhält man aus 1,4 g des in Beispiel 5e hergestellten Alkohols 580 mg der Titelverbindung.
¹H-NMR (CDCl₃): δ = 0,11 (3H), 0,15 (3H), 0,9 (9H), 0,85-0,95 (3H), 2,7 (3H), 3,26 (1H), 3,58 (1H), 4,77 (1H), 4,99 (1H), 5,64 (1H), 7,05 (1H), 7,25-7,46 (5H), 7,83 (1H) ppm.

### Beispiel 5g

### (3S,4Z)-5-(2-Methylthiazol-4-yl)-3-(tert-butyl-dimethylsilyloxy)-4-chlor-4-pentensäureethylester

In Analogie zu Beispiel 1g erhält man aus 12,5 g des in Beispiel 5f hergestellten Silylethers 9,1 g der Titelverbindung.
¹H-NMR (CDCl₃): δ = 0,09 (3H), 0,1 (3H), 0,9 (9H), 1,26 (3H), 2,68-2,78 (2H), 2,72 (3H), 4,15 (2H), 4,82 (1H), 7,04 (1H), 7,8 (1 H) ppm.

### Beispiel 5h

### (3S,4Z)-5-(2-Methylthiazol-4-yl)-3-(tert.-butyl-dimethylsilyloxy)-4-chlor-4-penten-1-ol

In Analogie zu Beispiel 1h erhält man aus 9,1 g des in Beispiel 5g hergestellten Ethylesters 7,5 g der Titelverbindung.
¹H-NMR (CDCl₃): δ = 0,09 (3H), 0,14 (3H), 0,94 (9H), 1,92-2,12 (3H), 2,72 (3H), 3,68-3,88 (2H), 4,58 (1 H), 7,04 (1 H), 7,81 (1 H) ppm.

### Beispiel 5i

### (3S,4Z)-5-(2-Methylthiazol-4-yl)-3-(tert.-butyl-dimethylsilyloxy)-1 -iod-4-chlor-4-penten

In Analogie zu Beispiel 1i erhält man aus 1,7 g des in Beispiel 5h hergestellten Alkohols 2,02 g der Titelverbindung.
¹H-NMR (CDCl₃): δ = 0,08 (3H), 0,14 (3H), 0,92 (9H), 2,1-2,33 (2H), 2,72 (3H), 3,2 (2H), 4,45 (1 H), 7,03 (1 H), 7,82 (1 H) ppm.

### Beispiel 5j

### (3S,4Z)-5-(2-Methylthiazol-4-yl)-3-(tert.-butyl-dimethylsilyloxy)-4-chlor-4-pententriphenylphosphoniumiodid

In Analogie zu Beispiel 1j erhält man aus 9.6 g des in Beispiel 5i hergestellten lodids 14.8 g der Titelverbindung.
¹H-NMR (CDCl₃): δ =0,1 (3H), 0,18 (3H), 0,9 (9H), 2,07 (2H), 2,69 (3H), 3,47-3,63 (1H), 3,68-3,85 (1H), 4,99(1H), 7,21 (1H), 7,67-7,87(16H) ppm.

### Beispiel 5k

### (2S,6FJZ,9S,1 OZ)-10-chlor-9-[[dimethyl(1,1-dimethylethyl)silyl]oxy]-11-(2-methyl-4-thiazolyl)-2,6-dimethyl-undeca-6,10-dienol-tetrahydropyran-2-yl-ether

Zu einer Lösung aus 8 g des in Beispiel 5j hergestellten Phosphoniumsalzes in 22 ml Tetrahydrofuran werden, bei 0°C unter Stickstoff, vorsichtig 6,94 ml Butyllithium (1,6molar in Hexan) getropft und 20 Minuten gerührt (dunkelrote Lösung). Nun wurden 1,69 g (6S)-6-Methyl-7-(tetrahydro-2H-pyran-2-yl(oxy)-heptan-2-on, gelöst in 11 ml Tetrahydrofuran, zum Reaktionsgemisch getropft. Das Reaktionsgemisch rührte 30 Minuten nach und wurde anschließend mit 11 ml gesättigter Ammoniumchlorid-Lösung versetzt. Nach weiteren 5 Minuten wurde das Reaktionsgemisch mit Essigester verdünnt, 1x mit gesättigter Natriumchlorid-Lösung gewaschen, über Natriumsulfat getrocknet und im Vakuum eingedampft. Nach Säulenchromatographie mit Hexan/Ether 0-50% erhält man 4,8 g der Titelverbindung.
¹H-NMR (CDCl₃): δ = 0,05-0,1 (6H), 0,85-0,95 (12H), 1,0-2,52 (14H), 1,6 (3H), 2,7 (3H), 3,07-3,27 (1H), 3,42-3,54 (3H), 3,86 (1H), 4,26 (1H), 4,56 (1H), 5,12 (1H), 6,97 (1 H), 7,81 (1 H) ppm.

### Beispiel 51

### (2S,6E/Z,9S, 10Z)-10-chlor-9-[[dimethyl(1,1-dimethylethyl)silyl]oxy]-11-(2-methyl-4-thiazolyl)-2,6-dimethyl-undeca-6,10-dienol

Zu einer Lösung aus 2,9 g des in Beispiel 5k hergestellten Olefins in 40 ml Ethanol werden 134,38 mg Pyridinium-p-toluolsulfonat addiert und 6 Stunden, bei 55°C unter Stickstoff, gerührt. Anschließend wird im Vakuum eingedampft. Nach Säulenchromatographie mit Hexan/Essigester 0-30% erhält man 1,73 g der Titelverbindung.
¹H-NMR (CDCl₃): δ = 0,05-0,1 (6H), 0,92 (9H), 1,02/1,09 (3H), 1,59/1,61 (3H), 1,15-1,8 (4H), 1,93-2,08 (2H), 2,23-2,52 (3H), 2,72 (3H), 4,27 (1), 5,15 (1H). 6,95/6,98 (1 H), 7,81 (1 H), 9,54/9,6 (1 H) ppm.

### Beispiel 5m

### (2S,6E/Z,9S,10Z)-10-chlor-9-[[dimethyl(1,1-dimethylethyl)silyl]oxy]-11-(2-methyl-4-thiazolyl)-2,6-dimethyl-undeca-6,10-dienal

Zu einer Lösung aus 1,5 g des in Beispiel 5I hergestellten Alkohols in 32,7 ml Methylenchlorid und 11 ml Dimethylsulfoxid gibt man bei Raumtemperatur unter Stickstoff 2,28 ml Triethylamin. Anschließend wird das Reaktionsgemisch mit 1,042 g S0₃-Pyridin-Komplex versetzt und 35 Minuten gerührt. Nach Zugabe von gesättigter Ammoniumchlorid-Lösung wird 5 Minuten nachgerührt, mit Ether verdünnt, mit halbgesättigter Natriumchlorid-Lösung gewaschen, die organische Phase über Natriumsulfat getrocknet und im Vakuum eingedampft. Man erhält 216 mg der Titelverbindung.

### Beispiel 5n

### (3S,6R,7S,8S,12E/Z,15S,16Z)-16-Chlor-17-(2-methyl-4-thiazolyl)-5-oxo-1,3,15-tris[[dimethyl(1,1-dimethylethyl)silyl]oxy]-4,4,6,8,12-pentamethyl-heptadeca-12,16-dien-7-ol

3,3 ml Butyllithium (1,6 molar in Hexan) werden auf 0°C gekühlt und vorsichtig mit einer Lösung aus 535 mg Diisopropylamin in 12 ml Tetrahydrofuran versetzt. Anschließend wir das Reaktionsgemisch auf -70°C gekühlt und mit einer Lösung aus 1,78 g (3S)-1,3-Bis[[dimethyl(1,1-dimethylethyl)silyl]oxy]-4,4-dimethyl-heptan-5-on in 12 ml Tetrahydrofuran zugetropft. Es wird 1 Stunde bei bleibender Temperatur gerührt. Nun wird eine Lösung aus 1,34 g des in Beispiel 5m herstellten Aldehyds in 9,7 ml Tetrahydrofuran zum Reaktionsgemisch getropft und nochmals 1,5 Stunden gerührt. Anschließend wird mit gesättigter Ammoniumchlorid-Lösung versetzt, mit Ether verdünnt, 2 x mit halbgesättigter Natriumchlorid-Lösung gewaschen, die organische Phase mit Natriumsulfat getrocknet und im Vakuum eingeengt. Nach Säulenchromatographie mit Hexan/Essigester 25% erhält man 2,52 g der Titelverbindung.
¹H-NMR (CDCl₃): δ = 0,0-0,1 (18H), 0,77/0,81 (3H), 0,7-1,8 (8H), 0,85-0,9 (27H), 1,0 (3H), 1,07 (3H), 1,21 (3H), 1,58 (3H), 1,9-2,04 (2H), 2,34-2,47 (2H), 2,71 (3H), 3,28 (2H), 3,53-3,7 (2H), 3,88 (1H), 4,18-4,28 (1H), 5,11 (1H), 6,92 (1H), 7,79 (1H) ppm.

### Beispiel 5o

### (3S,6R,7S,8S,12E/Z,15S,16Z)-16-Chlor-17-(2-methyl-4-thiazolyl)-5-oxo-1,3,7,15-tetrakis[[dimethyl(1,1-dimethylethyl)silyl]oxy]-4,4,6,8,12-pentamethyl-heptadeca-12,16-dien

Zu einer Lösung aus 1,52 g des in Beispiel 5n hergestellten Alkohols gelöst in 21,3 ml Methylenchlorid tropft man bei 0°C unter Stickstoff 722 µl Lutidin. Nach 5 Minuten gibt man 813 µl tert.-Butyldimethylsilyltrülat zum Reaktionsgemisch und rührt 1,5 Stunden nach. Anschließend wird mit Ether verdünnt, 1x mit 1 N Salzsäure, 2x mit gesättigter Natriumchlorid-Lösung gewaschen, die organische Phase mit Natriumsulfat getrocknet und im Vakuum eingedampft. Nach Säulenchromatographie mit Hexan/Ether 0-20% erhält man 221 mg der Titelverbindung.

### Beispiel 5p

### (3S,6R,7S,8S,12E/Z,15S,16Z)-16-Chlor-17-(2-methyl-4-thiazolyl)-5-oxo-3,7,15-tris[[dimethyl(1,1-dimethylethyl)silyl]oxy]-4,4,6,8,12-pentamethyl-heptadeca-12,16-dien-1-ol

Zu einer Lösung aus 1,9 g des in Beispiel 50 hergestellten Silylethers in 15 ml Methylenchlorid und 15 ml Methanol gibt man bei 0°C unter Stickstoff 453,45 mg Campher-10-sulfonsäure und rührt 2 Stunden nach. Anschließend wird mit 13 ml Triethylamin versetzt, nach 5 Minuten wird das Reaktionsgemisch auf gesättigte Natriumhydrogencarbonat-Lösung gegeben, mit Methylenchlorid verdünnt, die organische Phase 1x mit gesättigter Natriumchlorid-Lösung gewaschen, über Natriumsulfat getrocknet und im Vakuum eingedampft. Man erhält 1,41 g der Titelverbindung.
¹H-NMR (CDCl₃): δ = 0,02-0,13 (18H), 0,85-0,96 (30H), 1,08 (3H), 1,23 (3H), 1,6 (3H), 1,0-2,1 (10H), 2,32-2,52 (2H), 2,72 (3H), 3,13 (1H), 3,65 (2H), 3,8 (1H), 4,08 (1H), 4,21-4,3 (1H), 5,13 (1H), 6,98 (1H), 7,8 (1H) ppm.

### Beispiel 5q

### (3S,6R,7S,8S,12E/Z,15S,16Z)-16-Chlor-17-(2-methyl-4-thiazolyl)-5-oxo-3,7,15-tris[[dimethyl(1,1-dimethylethyl)silyl]oxy]-4,4,6,8,12-pentamethyl-heptadeca-12,16-dienal

Zu einer Lösung aus 1,4 g des in Beispiel 5p hergestellten Alkohols in 19 ml Methylenchlorid und 4,5 ml Dimethylsulfoxid gibt man bei Raumtemperatur unter Stickstoff 1,14 ml Triethylamin. Anschließend wird das Reaktionsgemisch mit 520 mg SO₃-Pyridin-Komplex versetzt und 2 Stunden gerührt. Nach Zugabe von gesättigter Ammoniumchlorid-Lösung wird 5 Minuten gerührt, mit Ether verdünnt, 2x mit halbgesättigter Natriumchlorid-Lösung gewaschen, die organische Phase über Natriumsulfat getrocknet und im Vakuum eingedampft. Man erhält 1,44 g der Titelverbindung.

### Beispiel 5r

### (3S,6R,7S,8S,12E/Z,15S,16Z)-16-Chlor-17-(2-methyl-4-thiazolyl)-5-oxo-3,7,15-tris[[dimethyl(1,1-dimethylethyl)silyl]oxy]-4,4,6,8,12-pentamethyl-heptadeca-12,16-diensäure

Zu einer Lösung aus 1,44 g des in Beispiel 5q hergestellten Aldehyds in 35 ml Aceton gibt man bei -30°C unter Stickstoff 1,89 ml Jones Reagenz. Nach 45 Minuten wird das Reaktionsgemisch mit 1,3 ml Isopropanol versetzt, 10 Minuten gerührt, mit Ether verdünnt, 3x mit halbgesättigter Natriumchlorid-Lösung gewaschen, die organische Phase über Natriumsulfat getrocknet und im Vakuum eingedampft. Nach Reinigung des Rohproduktes mittels präparativer Dünnschichtchromatographie mit Hexan/Ether 50% (3x gelaufen) erhält man 202 mg der Titelverbindung.
¹H-NMR (CDCl₃): δ = 0,03-0,16 (18H), 0,88-0,94 (30H), 1,09 (3H), 1,15 (3H), 1,18 (3H), 1,7 (3H), 1,0-2,44 (12H), 2,7 (3H), 3,15 (1H), 3,72 (1H), 4,32 (1H), 4,42 (1H), 5,19 (1H), 7,25 (1H), 7,87 (1H) ppm.

### Beispiel 5s

### (3S,6R,7S, 8S,12E/Z,15S,16Z)-16-Chlor-17-(2-methyl-4-thiazolyl)-5-oxo-3,7-bis[[dimethyl(1, 1-dimethylethyl)silyl]oxy]-15-hydroxy-4,4,6,8,12-pentamethyl-heptadeca-12,16-diensäure

Zu einer Lösung aus 22 mg der in Beispiel 5r hergestellten Carbonsäure in 4,3 ml Tetrahydrofuran gibt man bei Raumtemperatur unter Stickstoff 433,7 mg Tetrabutylammoniumfluorid und rührt 1,5h nach. Anschließend wird mit Essigester verdünnt, 1x mit 0,5 N Salzsäure, 2x mit halbgesättigter Natriumchlorid-Lösung gewaschen, über Natriumsulfat getrocknet und im Vakuum eingedampft. Nach Säulenchromatographie mit Hexan/Essigester 50% erhält man 43 mg der Titelverbindung.
¹H-NMR (CDCl₃): δ = 0,03-0,17 (12H), 0,83-0,98 (21H), 1,08 (3H), 1,18 (6H), 1,1-2,6 (12H), 1,73 (3H), 1,95 (2H), 2,22 (2H), 2,71 (3H), 3,16 (1H), 3,77 (1H), 4,33 (1H), 4,42 (1H), 5,2 (1H), 7,29 (1H), 7,85 (1H) ppm.

### Beispiel 5t

### (A) (4S, 7R,8S,9S,13(E),16S(Z))-4,8-Bis[[dimethyl(1,1-dimethylethyl)silyl]oxy]-16-(1-chlor-2-(2-methyl-4-thiazolyl)ethenyl)-1 -oxa-5,5,7,9,13-pentamethyl-cyclohexadec-1 3-en-2,6-dion

### (B) (4S,7R,8S,9S,13(Z),16S(Z))-4,8-Bis[[dimethyl(1,1-dimethylethyl)silyl]oxy]-16-(1-chlor-2-(2-methyl-4-thiazolyl)ethenyl)-1-oxa-5,5,7,9,13-pentamethyl-cyclohexadec-13-en-2,6-dion

Zu einer Lösung aus 180 mg des in Beispiel 5s hergestellten Alkohols in 3,4 ml Tetrahydrofuran gibt man bei 0°C unter Stickstoff 72,7 µl Triethylamin. Anschließend werden 48,2 µl 2,4,6-Trichlorbenzoylchlorid addiert und 1 Stunde gerührt. Nun wird diese Suspension über 3 Stunden mit einer Dosierpumpe zu einer Lösung aus 289,91 mg 4-N,N-Dimethylaminopyridin in 25,4 ml Toluol getropft und 1 Stunde gerührt. Dann wird das Reaktionsgemisch wird im Vakuum eingedampft. Nach Säulenchromatographie mit Hexan/Essigester 20% und anschließender Reinigung mittels präparativer Dünnschichtchromatographie mit Methylenchlorid/Methanol 0,5% erhält man 32 mg (E-Verbindung) Titelverbindung A und 81 mg (Z-Verbindung) der Titelverbindung B.
(B)¹H-NMR (CDCl₃): δ = 0,02-0,15 (12H), 0,85 (9H), 0,97 (9H), 0,9-2,95 (11H), 1,0 (3H), 1,1 (3H), 1,15 (3H), 1,27 (3H), 1,57 (3H), 2,71 (3H), 3,04 (1H), 3,9 (1H), 4,03 (1H), 5,13 (1H), 5,19 (1H), 7,06 (1H), 7,83 (1H) ppm.

### Beispiel 5

### (4S,7R,8S,9S,13(Z),16S(Z))-4,8-Dihydroxy-16-(1-chlor-2-(2-methyl-4-thiazolyl)ethenyl)-1-oxa-5,5,7,9,13-pentamethyl-cyclohexadec-13-en-2,6-dion

Zu einer Lösung aus 80 mg der in Beispiel 5t hergestellten Titelverbindung B in 314 µl Methylenchlorid gibt man bei -20°C unter Stickstoff 702 µl einer 20%ige Lösung von Trifluoressigsäure in Methylenchlorid und rührt 5,5 Stunden bei 0°C nach. Anschließend wird das Reaktionsgemisch im Vakuum eingedampft. Nach Säulenchromatographie mit Hexan/Essigester 50% erhält man 43,8 mg der Titelverbindung.
¹H-NMR (OMSO-d⁶, 100°C): δ = 0,94 (3H), 0,82-3,3 (14H), 1,11 (3H), 1,23 (6H), 1,67 (3H), 2,64 (3H), 3,58 (1H), 4,27 (1H), 5,16 (1H), 5,39 (1H), 7,06 (1H), 7,77 (1H) ppm.

### Beispiel 6

### (4S,7R,8S,9S,13(E),16S(Z))-4,8-Dihydroxy-16-(1-chlor-2-(2-methyl-4-thiazolyl)ethenyl)-1-oxa-5,5,7,9,13-pentamethyl-cyclohexadec-13-en-2,6-dion

Zu einer Lösung aus 45 mg der in Beispiel 5t hergestellten Titelverbindung A in 177 µl Methylenchlorid gibt man bei -20°C unter Stickstoff 395 µl einer 20%ige Lösung von Trifluoressigsäure in Methylenchlorid und rührt 5,5 Stunden bei 0°C nach. Anschließend wird das Reaktionsgemisch im Vakuum eingedampft. Nach Säulenchromatographie mit Hexan/Essigester 50% erhält man 27 mg der Titelverbindung.
¹H-NMR (DMSO-d₆, 100°C): 0,8-2,7 (13H), 0,91 (3H), 1,11 (3H), 1,12 (6H), 1,6 (3H), 2,65 (3H), 3,25 (1H), 3,54 (1H), 4,46 (1H), 5,18 (1H), 5,44 (1H), 7,05 (1H), 7,83 (1H) ppm.

### Beispiel 7

### (A) (1S,3S(Z),7S,10R,11S,12S,16R)-7,11-Dihydroxy-3-(1-chlor-2-(2-methyl-4-thiazolyl)ethenyl)-8,8,10,12,16-pentamethyl-4,17-dioxabicyclo[14.1.0]heptadecan-5,9-dion

### (B) (1R,3S(Z),7S,10R,11S,12S,16S)-7,11-Dihydroxy-3-(1-chlor-2-(2-methyl-4-thiazotyl)ethenyl)-8,8,10,12,16-pentamethyl-4,17-dioxabicyclo[14.1.0]heptadecan-5,9-dion

Zu einer Lösung aus 14 mg des in Beispiel 5 hergestellten Epothilon-D-Derivates in 0,3 ml Acetonitril gibt man bei 0°C unter Stickstoff 154,8 µg Ethylendiamintetraessigsäuredi-Natriumsalz und 324,73 µg 1,1,1-Trifluoraceton. Anschließend werden 34,65 µg Oxone und 17,74 µg Natriumhydrogencarbonat zum Reaktionsgemisch gegeben und 4 Stunden gerührt. Nun wird mit 2 ml Natriumthiosulfat-Lösung versetzt, mit 100 ml Essigester verdünnt, mit halbgesättigter Natriumchlorid-Lösung gewaschen, über Natriumsulfat getrocknet und im Vakuum eingedampft. Nach Reinigung des Rohproduktes mittels präparativer Dünnschichtchromatographie mit Methylenchlorid/Methanol 20% erhält man 3,8 mg (polar) A und 2,5 mg (unpolar) B der Titelverbindung.
(A) ¹H-NMR (MeOH-d⁴): δ = 0,8-2,6 (9H), 1,03 (3H), 1,2 (3H), 1,29 (6H), 1,33 (3H), 2,7 (3H), 2,93 (1H), 3,67 (1H), 4,23 (1H), 5,63 (1H), 7,12 (1H), 7,4.4 (1H) ppm.

### Beispiel 8

### (A) (1S,3S(Z),7S,10R,11S,12S,16S)-7,11-Dihydroxy-3-(1-chlor-2-(2-methyl-4-thiazolyl)ethenyl)-8,8,10,12,16-pentamethyl-4,17-dioxabicyclo[14.1.0]heptadecan-5,9-dion

### (B) (1R,3S(Z),7S,10R,11S,12S,16R)-7,11-Dihydroxy-3-(1-chlor-2-(2-methyl-4-thiazolyl)ethenyl)-8,8,10,12,16-pentamethyl-4,17-dioxabicyclo[14.1.0]heptadecan-5,9-dion.

Zu einer Lösung aus 14 mg der in Beispiel 6 hergestellten Epothilon-D-Derivates in 0,3 ml Acetonitril gibt man bei 0°C unter Stickstoff 154,8 µg Ethylendiamintetraessigsäuredi-Natriumsalz und 324,73 µg 1,1,1-Trifluoraceton. Anschließend werden 34,65 µg Oxone und 17,74 µg Natriumhydrogencarbonat zum Reaktionsgemisch gegeben und 4 Stunden gerührt. Nun wird mit 2 ml Natriumthiosulfat-Lösung versetzt, mit 100 ml Essigester verdünnt, mit halbgesättigter Natriumchlorid-Lösung gewaschen, über Natriumsulfat getrocknet und im Vakuum eingedampft. Nach Reinigung des Rohproduktes mittels präparativer Dünnschichtchromatographie mit Methylenchlorid/Methanol 20% erhält man 6,8 mg (polar) A und 3,4 mg (unpolar) B der Titelverbindung.

### Beispiel 9

### (4S,7R,8S,9S,13(Z),16S(Z))-4,8-Dihydroxy-16-(1-fluor-2-(2-methyt-4-thiazolyl)ethenyl)-1 -oxa-7,9,13-trimethyl-5,5-(1,3-trimethylen)cyclohexadec-1 3-en-2,6-dion

Analog zu Beispiel 5 werden aus 431 mg (0,585 mmol) der unter 9j beschriebenen Verbindung A 235 mg der Titelverbindung erhalten.
¹H-NMR (CDCl₃): δ = 1.00 (3H), 1.27 (3H), 1.66 (3H), 2.70 (3H), 2.75-3.04 (3H), 3.43 (1 H), 3.68 (1 H), 4.42 (1 H), 5.13 (1H), 5.37-5.46 (1H), 6.15-6.29 (1H), 7.36 (1H) ppm.

### Beispiel 9a

### (2S,6E1Z,9S,1 OZ)-9-[[Dimethyl(1,1-dimethylethyl)silyl]oxy]-1 0-fluor-11-(2-methyl-4-thiazolyl)-2,6-dimethylundeca-6,10-dienol-tetrahydropyran-2-yl-ether

Analog zu Beispiel 5k werden aus 2,47 g (10,8 mmol) 6(S)-6-Methyl-7-(tetrahydro-2H-pyran-2-yl(oxy))heptan-2-on (Darstellung siehe: DE 19751200.3) und 11.4 g (16,2-mmol) der unter Beispiel 1j beschriebenen Verbindung 3,52 g der Titelverbindung erhalten.
¹H-NMR (CDCl₃): δ = 0.08 (6H), 0.85-0.95 (12H), 0.60 + 0.69 (3H), 2.37-2.50 (2H), 2.70 (3H), 3.10-3.30 (1H), 3.45-3.65 (2H), 3.82-3.92 (1H), 4.13-4.26 (1H), 4.57 (1H), 5.14 (1H), 5.98-6.12 (1H), 7.33 (1H) ppm.

### Beispiel 9b

### (2S,6E/Z,9S,10Z)-9-[[Dimethyl(1,1-dimethylethyl)silyl]oxy]-10-fluor-11-(2-methyl-4-thiazolyl)-2,6-dimethylundeca-6,10-dienol

Aus 3,52 g (6,70 mmol) der unter 9a beschriebenen Verbindung werden analog zu Beispiel 512,81 g der Titelverbindung erhalten.
¹H-NMR (CDCl₃): δ = 0.09 (6H), 0.87 (3H), 0.91 (9H), 1.58 + 1.69 (3H), 1.95-2.05 (2H), 2.35-2.52 (2H), 2.70 (3H), 3.38-3.55 (2H), 4.32 (1H). 5.14 (1H), 5.95-6.12 (1H), 7.34 (1 H) ppm.

### Beispiel 9c

### (2S,6E/Z,9S,10Z)-9-[[Dimethyl(1,1-dimethylethyl)silyl]oxy]-10-fluor-11-(2-methyl-4-thiazolyl)-2,6-dimethylundeca-6,10-dienal

Aus 2,81 g (6,37 mmol) der unter 9b beschriebenen Verbindung werden analog zu Beispiel 5m 2,80 g der Titelverbindung erhalten.
¹H-NMR (CDCl₃): δ = 0.08 (6H), 0.90 (9H), 1.03-1.10 (3H), 1.58 + 1.67 (3H), 1.86 (1H), 1.95-2.11 (2H), 2.24-2.51 (3H), 2.70 (3H), 3.75 (1H). 4.15-4.27 (1H). 5.18 (1H), 5.97-6.14 (1 H), 7.34 (1H). 9.55 + 9.59 (1 H) ppm.

### Beispiel 9d

### (3S,6R,7S,8S,12E/Z,15S,16Z)-16-Fluor-17-(2-methyl-4-thiazolyl)-5-oxo-6,8,12-trimethyl-4,4-(1,3-trimethylen)-1,3,15-tris[[dimethyl(1,1-dimethylethyl)silyl]oxy]heptadeca-12,16-dien-7-ol

Aus 2,77 g (6,68 mmol) (S)-1-(1,3-Bis[[dimethyl(1,1-dimethylethyl)silyl]oxypropyl] cyclobutyl)-propan-1-on (Darstellung siehe: DE 19751200.3) und 1,65 g (3,75 mmol) der unter 9c beschriebenen Verbindung werden analog zu Beispiel 5n 2,18 g der Titelverbindung erhalten.
¹H-NMR (CDCl₃): δ = 0.04 (6H), 0.08 (6H), 0.15 (3H), 0.17 (3H), 0.79 (3H), 0.86-0.97 (27H), 1.03 (3H), 1.25-1.41 (2H), 1.59 + 1.68 (3H), 1.69-1.87 (4H), 1.90-2.09 (2H), 2.23-2.50 (4H), 2.70 (3H), 3.20-3.36 (2H), 3.58 (2H), 4.08-4.25 (2H), 5.14 (1H), 5.98-6.13 (1H), 7.33 (1H) ppm.

### Beispiel 9e

### (3S,6R,7S,8S,12E/Z,15S,16Z)-16-Fluor-17-(2-methyl-4-thiazolyl)-5-oxo-1,3,7,15-tetrakis[[dimethyl(1,1-dimethylethyl)silyl]oxy]-6,8,12-trimethyl-4,4-(1,3-trimethylen)heptadeca-12,16-dien

Aus 2,18 g (2,55 mmol) der unter 9d beschriebenen Verbindung werden analog zu Beispiel 50 2,47 g der Titelverbindung erhalten:
¹H-NMR (CDCl₃): δ = 0.00-0.20 (24H), 0.85-1.00 (39H), 1.06 (3H), 1.48 + 1.67 (3H), 2.20-2.47 (4H), 2.72 (3H), 3.08 (1H), 3.59 (2H), 3.78 (1H), 4.10 (1H), 4.14-4.25 (1H), 5.15 (1H), 6.00-6.13 (1H), 7.35 (1H) ppm.

### Beispiel 9f

### (3S,6R,7S,8S,12E/Z,15S,16Z)-16-Fluor-17-(2-methyl-4-thiazolyl)-5-oxo-6,8,12-trimethyl-4,4-(1,3-trimethylen)-3,7,15-tris[[dimethyl(1,1-dimethylethyl)silyl]oxy]heptadeca-12,16-dien-1-ol

Aus 2,47 g (2,55 mmol) der unter 9e beschriebenen Verbindung werden analog zu Beispiel 5p 1,626 g der Titelverbindung erhalten.
¹H-NMR (CDCl₃): δ = 0.03-0.13 (12H), 0.04-0.20 (6H), 0.86-1.03 (30H), 1.08 (3H), 1.59+ 1.68 (3H), 1.70-2.50 (10H), 2.72 (3H), 3.12 (1H), 3.64 (2H), 3.81 (1H), 4.08 (1H), 4.13-4.27 (1H), 5.15 (1H), 6.00-6.17 (1H), 7.35 (1H) ppm.

### Beispiel 9g

### (3S,6R,7S,8S,12E/Z,15S,16Z)-16-Fluor-17-(2-methyl-4-thiazolyl)-5-oxo-6,8,12-trimethyl-4,4-(1,3-trimethylen)-3,7,15-tris[[dimethyl(1,1-dimethylethyl)silyl]oxy]heptadeca-12,16-dienal

Aus 1,626 g (1,91 mmol) der unter 9f beschriebenen Verbindung werden analog zu Beispiel 5q 1,628 g der Titelverbindung erhalten.
¹H-NMR (CDCl₃): δ = 0.02-0.12 (15H), 0.18 (3H), 0.85-1.00 (30 H), 1.05-1.10 (3H), 1.59 + 1.68 (3H), 1.70-2.55 (10H), 2.71 (3H), 3.75 (1H), 4.12-4.25 (1H), 4.53 (1H), 5.17 (1H), 6.00-6.15 (1H), 7.33 (1H), 9.75 (1H) ppm.

### Beispiel 9h

### (3S,6R,7S,8S,12E/Z,15S,16Z)-16-Fluor-17-(2-methyl-4-thiazolyl)-5-oxo-6,8,12-trimethyl-4,4-(1,3-trimethylen)-3,7.15-tris[[dimethyl(1,1-dimethylethyl)silyl]oxy]heptadeca-12,16-diensäure

Aus 1,628 g (1,91 mmol) der unter 9g beschriebenen Verbindung werden analog zu Beispiel 5r 1,161 g der Titelverbindung erhalten.
¹H-NMR (CDCl₃): δ = 0.02-0.15 (15H), 0.19 (3H), 0.84-1.00 (30H), 1.10-1.07 (3H), 1.56 + 1.69 (3H), 2.10-2.55 (10H), 2.70 (3H), 2.97-3.14 (1H), 3.78 (1H), 3.84 (1H), 4.09-4.27 (2H), 4.41 + 4.48 (1 H), 5.10-5.23 (1 H), 6.10 + 6.31 (1 H), 7.37 (1 H) ppm.

### Beispiel 9i

### (3S,6R,7S,8S,12E/Z,15S,16Z)-3,7-Bis[[dimethyl(1,1-dimethylethyl)silyl]oxy]-16-fluor-15-hydroxy-17-(2-methyl-4-thiazolyl)-5-oxo-6,8,12-trimethyl-4,4-(1,3-trimethylen)heptadeca-12,16-diensäure

Aus 1,161 g (1,34 mmol) der unter 9h beschriebenen Verbindung werden analog zu Beispiel 5s 1,01 g der Titelverbindung erhalten.
¹H-NMR (CDCl₃): δ = 0.01-0.15 (9H), 0.17 (3H), 0.83-1.01 (21H), 1.07-1.15 (3H), 1.61 + 1.73 (3H), 2.07-2.60 (10H), 2.71 (3H), 2.92-3.11 (2H), 2.85 (1H). 3.80 (1H). 4.18-4.30 (1H), 4.40 + 4.48 (1H), 5.11-5.22 (1H), 6.19 + 6.37 (1H), 7.37 (1H) ppm.

### Beipiel 9j

### 4S,7R,8S,9S,13(Z),16S(Z)-4,8-Bis[[dimethyl(1,1-dimethylethyl)silyl]oxy]-16-(1-fluor-2-(2-methyl-4-thiazolyl)ethenyl-1-oxa-7,9,13-trimethyl-5,5-(1,3-trimethylen)cyclohexa-dec-13-en-2,6-dion (A) und 4S,7R,8S,9S,13(E),16S(Z)-4,8-Bis[[dimethyl(1,1-dimethylethyl)silyl]oxy]-16-(1-fluor-2-(2-methyl-4-thiazolyl)ethenyl-1-oxa-7,9,13-trimethyl-5,5-(1,3-trimethylen)cyclohexa-dec-13-en-2,6-dion (B)

Aus 1,01 g (1,34 mmol) der unter 9i beschriebenen Verbindung werden analog zu Beispiel 5t 434 mg der Titelverbindung A und 395 mg der Titelverbindung B erhalten.
¹H-NMR (CDCl₃) von A: δ = -0.07 (3H), 0.07-0.20 (9H), 0.80 (9H), 0.93 (9H), 0.98 (3H), 1.22 (3H), 1.68 (3H), 1.80-1.90 (1H), 2.00-2.10 (1H), 2.20-2.50 (4H), 2.60-2.68 (4H), 2.72 (3H), 2.76-3.00 (2H), 3.92 (1H), 4.41 (1H), 5.08-5.12 (2H), 6.08-6.22 (1H), 7.38 (1 H) ppm.
¹H-NMR (CDCl₃) von B: δ = 0.02 (3H), 0.07 (3H), 0.11 (3H), 0.14 (3H), 0.90 (9H), 0.93 (9H), 1.02 (3H), 1.25 (3H), 1.51 (3H), 1.70-2.15 (8H), 2.30-2.60 (4H), 2.72 (3H), 2.77-2.93 (2H), 4.19 (1H), 4.59 (1H), 5.10 (1H), 5.42 (1H), 6.09-6.23 (1H), 7.36 (1 H) ppm.

### Beispiel 10

### (1S,3S(Z),7S, 10R,11 S,12S,16R)-7,11-Dihydroxy-3-((1-fluor)-2-(2-methyl-4-thiazolyl)ethenyl)-10, 12, 16-trimethyl-8,8-(1,3-trimethylen)-4,17-dioxabicyclo[14.1.0]hepta-deca-5,9-dion (A) und (1R,3S(Z),7S,10R,11S,12S,16S)-7,11-Dihydroxy-3-((1-fluor)-2-(2-methyl-4-thiazolyl)ethenyl)-10,12,16-trimethyl-8,8-(1,3-trimethylen)-4,17-dioxabicyclo[14.1.0]hepta-deca-5,9-dion (B)

Aus 50 mg (0,098 mmol) der unter Beispiel 9 beschriebenen Verbindung werden analog zu Beispiel 7 31 mg der Titelverbindung A und 7 mg der Titelverbindung B erhalten. ¹H-NMR (CDCl₃) von A: δ = 0.99 (3H), 1.25 (3H), 1.28 (3H), 2.71 (3H), 2.81 (1H), 3.02-3.12 (1H), 3.62-3.77 (2H), 4.40 (1H), 5.56-5.68 (1H), 6.17-6.81 (1H), 7.37 (1H) ppm.
¹H-NMR (CDCl₃) von B: δ = 0.92 (3H), 1.20 (3H), 1.38 (3H), 2.75 (3H), 3.00 (1H), 3.11 (1H), 3.86 (1H), 4.42 (1H), 5.29 (1H), 6.26-6.39 (1H), 7.41 (1H) ppm.

### Beispiel 11

### (4S,7R,8S,9S,13(Z), 16S(Z))-4,8-Dihydroxy-16-((1-fluor-2-(2-methyl-4-thiazolyl)ethenyl)-1-oxa-7,9,13-trimethyl-5,5-(1,3-trimethylen)cyclohexadec-13-en-2,6-dion

Aus 395 mg (0,54 mmol) der unter 9j beschriebenen Verbindung B werden analog zu Beispiel 5t 200 mg der Titelverbindung erhalten.
¹ H-NMR (CDCl₃): δ = 1.00 (3H), 1.25 (3H), 1.54 (3H), 2.69 (1H). 2.97-3.08 (1H), 3.63 (1H), 4.44 (1H), 5.09 (1H), 5.54-5.63 (1H), 6.11-6.25 (1H), 7.38 (1H) ppm.

### Beispiel 12

### (1S,3S(Z),7S,10R,11S,12S,16S)-7,11-Dihydroxy-3-((1-fluor)-2-(2-methy)-4-thiazolyl)ethenyl)-10,12,16-trimethyl-8,8-(1,3-trimethylen)-4,17-dioxabicyclo[14.1.0]heptadeca-5,9-dion (A) und (1R,3S(Z),7S,10R,11S,12S,16R)-7,11-Dihydroxy-3-(1-fluor)-2-(2-methyl-4-thiazolyl)ethenyl)-10,12,16-trimethyl-8,8-(1,3-trimethylen)-4,17-dioxabicyclo[14.1.0]heptadeca-5,9-dion (B)

Aus 100 mg (0,197 mmol) der unter Beispiel 11 beschriebenen Verbindung werden analog zu Beispiel 7 41 mg der Titelverbindung A und 36 mg der Titelverbindung B erhalten.
¹H-NMR (CDCl₃) von A: δ = 0.93 (3H), 1.19 (3H), 1.22 (3H), 2.70 (3H), 2.88 (1H), 3.11 (1H), 3.19 (1H), 3.65 (1H), 3.72 (1H), 4.45 (1H), 5.61-5.72 (1H), 6.12-6.26 (1H), 7.37 (1H) ppm.
¹H-NMR (CDCl₃) von B: δ = 0.98 (3H), 1.22-1.27 (6H), 2.72 (3H), 2.93 (1H), 3.07-3.17 (1H). 3.30 (1H). 3.67 (1H). 3.85 (1H), 4.40 (1H). 5.68-5.77 (1 H), 6.22-6.36 (1H), 7.41 (1H) ppm.

### Beispiel 13

### (4S,7R,8S,9S,13(Z),16S(Z))-4,8-Dihydroxy-9,13-dimethyl-7-ethyl-16-(1-fluor-2-(2-methyl-4-thiazolyl)ethenyl)-1-oxa-5,5-(1,3-trimethylen)cyclohexadec-13-en-2,6-dion

Analog zu Beispiel 5 werden aus 400 mg (0,534 mmol) der unter 13g beschriebenen Verbindung 181 mg der Titelverbindung erhalten.
¹H-NMR (CDCl₃): δ = 0.94 (3H), 1.01 (3H), 1.69 (3H), 2.68-2.82 (1H), 2.71 (3H), 2.96 (1H), 3.38 (1H), 3.68 (1H), 4.42 (1H), 5.10 (1H), 5.42 (1H), 6.13-6.27 (1H), 7.37 (1H) ppm,

### Beispiel 13a

### (3S,6R,7S,8S,12E/Z,15S,16Z)-8,12-Dimethyl-6-ethyl-16-fluor-17-(2-methyl-4-thiazolyl)-5-oxo-4,4-(1,3-trimethylen)-1,3,15-tris[[dimethyl(1,1-dimethylethyl)silyl]oxy]heptadeca-12,16-dien-7-ol

Aus 2,975 g (6,937 mmol) (S)-1-(1,3-Bis[[dimethyl(1,1-dimethylethyl)silyl]oxypropyl]-cyclobutyl)-butan-1-on (Darstellung siehe: DE 19751200.3) und 1,695 g (3,854 mmol) der unter 9c beschriebenen Verbindung werden analog zu Beispiel 5n 2,042 g der Titelverbindung erhalten.
¹H-NMR (CDCl₃): δ = 0.01-0.20 (18H), 0.84-1.00 (33H), 1.60 + 1.69 (3H), 2.69 (3H), 3.11 (1H), 3.22 (1H). 3.40 (1H). 3.62 (2H), 4.06-4.25 (2H), 5.97-6.12 (1H), 7.34 (1H) ppm.

### Beispiel 13b

### (3S,6R,7S,8S,12E/Z,15S,16Z)-8,12-Dimethyl-6-ethyl-16-fluor-17-(2-methyl-4-thiazolyl) 5-oxo-1,3,7,15-tetrakis[[dimethyl(1,1-dimethylethyl)silyl]oxy]-4,4-(1,3-trimethylen)heptadeca-12,16-dien

Aus 2,042 g (2,351 mmol) der unter 13a beschriebenen Verbindung werden analog zu Beispiel 5o 2,311 g der Titelverbindung erhalten.
¹H-NMR (CDCl₃): δ = 0.00-0.20 (24 H), 0.80-0.99 (42 H), 1.60 + 1.68 (3H), 2.70 (3H), 3.02 (1H), 3.60 (2H), 3.86 (1H), 4.04-4.25 (2H), 5.97-6.13 (1H), 7.32 (1H) ppm.

### Beispiel 13c

### (3S,6R,7S,8S,12E/Z,15S,16Z)-8,12-Dimethyl-6-ethyl-16-fluor-17-(2-methyl-4-thiazolyl)-5-oxo-4,4-(1,3-trimethylen)-3,7,15-tris[[dimethyl(1,1-dimethylethyl)silyl]oxy]heptadeca-12,16-dien-1-ol

Aus 2,311 g (2,351 mmol) der unter 13b beschriebenen Verbindung werden analog zu Beispiel 5p 1,593 g der Titelverbindung erhalten.
¹H-NMR (CDCl₃): δ = 0.02-0.19 (18H), 0.80-0.99 (33H), 1.57 (3H) + 1.67 (3H), 2.70 (3H), 3.04 (1H), 3.60-3.71 (2H), 3.87 (1H), 4.04-4.25 (2H), 5.13 (1H), 5.95-6.11 (1H), 7.33 (1H) ppm.

### Beispiel 13d

### (3S,6R,7S,8S,12E/Z,15S,16Z)-8,12-Dimethyl-6-ethyl-16-fluor-17-(2-methyl-4-thiazolyl)-5-oxo-4,4-(1,3-trimethylen)-3,7,15-tris[[dimethyl(1,1-dimethylethyl)silyl]oxy]heptadeca-12,16-dienal

Aus 1,593 g (1,834 mmol) der unter 13c beschriebenen Verbindung werden analog zu Beispiel 5q 1,589 g der Titelverbindung erhalten.
¹H-NMR (CDCl₃): δ = 0.04-0.20 (18H), 0.82-1.00 (33H), 1.58 (3H) + 1.68 (3H), 2.71 (3H), 3.04 (1H), 3.86 (1H), 4.19 (1H), 4.55 (1H), 5.17 (1H), 5.98-6.12 (1H), 7.33 (1H), 9.79 (1H) ppm.

### Beispiel 13e

### (3S,6R,7S,8S,12Z,15S,16Z)-8,12-Dimethyl-6-ethyl-16-fluor-17-(2-methyl-4-thiazolyl)-5-oxo-4,4-(1,3-trimethylen)-3,7,15-tris[[dimethyl(1,1-dimethylethyl)silyl]oxy]hepta-deca-12,16-diensäure (A) und (3S,6R,7S,8S,12E,15S,16Z)-8,12-Dimethyl-6-ethyl-16-fluor-17-(2-methyl-4-thiazolyl)-5-oxo-4,4-(1,3-trimethylen)-3,7,15-tris[[dimethyl(1,1-dimethylethyl)silyl]oxy]hepta-deca-12,16-diensäure (B)

Aus 1,589 g (1,834 mmol) der unter 13d beschriebenen Verbindung werden analog zu Beispiel 5r 664 mg der Titelverbindung A sowie 566 mg der Titelverbindung B erhalten. ¹H-NMR (CDCl₃) von A: δ= 0.00 (3H), 0.07-0.09 (9H), 0.12 (3H), 0.19 (3H), 0.86-1.03 (33H), 1.70 (3H), 2.70 (3H), 2.90 (1H), 3.73 (1H), 4.21 (1H), 4.48 (1H), 5.21 (1H), 6.38-6.52 (1 H), 7.38 (1 H) ppm.
¹H-NMR (CDCl₃) von B: δ= 0.00 (3H), 0.05 (3H), 0.07 (3H), 0.09 (3H), 0.15 (3H), 0.20 (3H), 0.84-0.99 (33H), 1.56 (3H), 2.69 (3H), 2.98 (1H), 3.87 (1H), 4.40 (1H), 5.12 (1H), 6.07-6.22 (1H), 7.38 (1H) ppm.

### Beispiel 13f

### (3S,6R,7S,8S,12Z,15S,16Z)-3,7-Bis[[dimethyl(1,1-dimethylethyl)silyl]oxy]-8,12-dimethyl 6-ethyl-16-fluor-15-hydroxy-17-(2-methyl-4-thiazolyl)-5-oxo-4,4-(1,3-trimethylen)heptadeca-12,16-diensäure

Aus 663 mg (0,752 mmol) der unter 13e beschriebenen Verbindung A werden analog zu Beispiel 5s 578 mg der Titelverbindung erhalten.
¹H-NMR (CDCl₃): δ = 0.03 (3H), 0.06 (3H), 0.09 (3H), 0.17 (3H), 0.85-1.00 (24H), 1.75 (3H), 2.71 (3H), 2.89 (1H), 3.78 (1H), 4.25 (1H), 4.49 (1H), 5.21 (1H), 6.43-6.57 (1H), 7.39 (1 H) ppm.

### Beispiel 13g

### 4S,7R,8S,9S,13(Z),16S(Z)-4,8-Bis[[dimethyl(1,1-dimethylethyl)silyl]oxy]-9,13-dimethyl-7-ethyl-16-(1-fluor-2-(2-methyl-4-thiazolyl)ethenyl)-1-oxa-5,5-(1,3-trimethylen)cyclohexadec-13-en-2,6-dion

Aus 578 mg (0,752 mmol) der unter 13f beschriebenen Verbindung werden analog zu Beispiel 5t 400 mg der Titelverbindung erhalten.
¹H-NMR (CDCl₃): δ = -0.09 (3H), 0.09 (3H), 0.15 (3H), 0.17 (3H), 0.80-0.97 (21 H), 1.00 (3H), 1.68 (3H), 2.70 (3H), 2.75-2.88 (1H), 2.98 (1H), 4.04 (1H), 4.42 (1H), 5.17 (3H), 6.07-6.20 (1H), 7.37 (1H) ppm.

### Beispiel 14

### (1S,3S(Z),7S,10R,11S,12S,16R)-7,11-Dihydroxy-12,16-dimethyl-10-ethyl-3-(1-fluor-2-(2-methyl-4-thiazolyl)ethenyl)-8,8-(1,3-trimethylen)-4,17-dioxabicyclo[14.1.0]hepta-decan-5,9-dion (A) und (1R,3S(Z),7S,10R,11S,12S,16S)-7,11-Dihydroxy-12,16-dimethyl-10-ethyl-3-(1-fluor-2-(2-methyl-4-thiazolyl)ethenyl)-8,8-(1,3-trimethylen)-4,17-dioxabicyclo[14.1.0]hepta-decan-5,9-dion (B)

Aus 40 mg (0,0767 mmol) der unter Beispiel 13 beschriebenen Verbindung werden analog zu Beispiel 7 26 mg der Titelverbindung A und 6 mg der Titelverbindung B erhalten.
¹H-NMR (CDCl₃) von A: δ= 0.95 (3H), 0.98 (3H), 1.29 (3H), 2.71 (3H), 2.78 (1H), 3.03 (1 H), 3.67 (1H), 4.40 (1H), 5.66 (1H), 6.16-6.79 (1H), 7.38 (1H) ppm.
¹H-NMR (CDCl₃) von B: δ= 0.95-1.00 (6H), 1.26 (3H), 2.70 (3H), 2.91 (1H), 2.95-3.05 (2H), 3.34 (1H), 3.73 (1H), 4.48 (1H), 5.73 (1H), 6.22-6.35 (1H), 7.40 (1H) ppm.

### Beispiel 15

### (4S,7R,8S,9S,13(E),16S(Z))-4,8-Dihydroxy-9,13-dimethyl-7-ethyl-16-(1-fluor-2-(2-methyl-4-thiazolyl)ethenyl)-1-oxa-5,5-(1,3-trimethylen)cyclohexadec-13-en-2,6-dion

Analog zu Beispiel 5 werden aus 433 mg (0,5778 mmol) der unter 15b beschriebenen Verbindung 214 mg der Titelverbindung erhalten.
¹H-NMR (CDCl₃): δ = 0.94 (3H), 1.02 (3H), 1.54 (3H), 2.61-2.74 (1H), 2.68 (3H), 3.08 (1H), 3.73 (1H), 3.98 (2H), 4.52 (1 H), 5.09 (1H), 5.54 (1H), 6.06-6.20 (1H), 7.37 (1 H) ppm.

### Beispiel 15a

### (3S,6R,7S,8S,12E,15S,16Z)-3,7-Bis[[dimethyl(1,1-dimethylethyl)silyl]oxy]-8,12-dimethyl-6-ethyl-16-fluor-15-hydroxy-17-(2-methyl-4-thiazolyl)-5-oxo-4,4-(1,3-trimethylen)heptadeca-12,16-diensäure

Aus 566 mg (0,642 mmol) der unter 13e beschriebenen Verbindung B werden analog zu Beispiel 5s 493 mg der Titelverbindung erhalten.
¹H-NMR (CDCl₃): δ = 0.01 (3H), 0.04 (3H), 0.09 (3H), 0.17 (3H), 0.82-0.95 (24H), 1.62 (3H), 2.68 (3H), 2.95 (3H), 3.82 (1H). 4.17-4.30 (1H). 4.40 (1H). 5.15 (1H). 6.15-6.28 (1H), 7.37 (1H) ppm.

### Beispiel 15b

### 4S,7R,8S,9S,13(E),16S(Z)-4,8-Bis[[dimethyl(1,1-dimethylethyl)silyl]oxy]-9,13-dimethyl-7-ethyl-16-(1-fluor-2-(2-methyl-4-thiazolyl)ethenyl)-1-oxa-5,5-(1,3-trimethylen)cyclohexadec-13-en-2,6-dion

Aus 493 mg (0,642 mmol) der unter 15a beschriebenen Verbindung werden analog zu Beispiel 5t 433 mg der Titelverbindung erhalten.
¹H-NMR (CDCl₃): δ = 0.07 (3H), 0.10 (3H), 0.12 (3H), 0.15 (3H), 0.85-1.04 (24H), 2.71 (3H), 2.92 (1H), 4.06 (1H), 5.15 (1 H), 5.36-5.47 (1H). 6.10-6.23 (1H), 7.37 (1H) ppm.

### Beispiel 16

### (1 S,3S(Z),7S,10R,11S,12S,16S)-7,11-Dihydroxy-12,16-dimethyl-10-ethyl-3-(1-fluor-2-(2-methyl-4-thiazolyl)ethenyl)-8,8-(1,3-trimethylen)-4,17-dioxabicyclo[14.1.0]heptadecan-5,9-dion (A) und (1R,3S(Z),7S,10R,11S,12S,16R)-7,11-Dihydroxy-12,16-dimethyl-10-ethyl-3-(1-fluor-2-(2-methyl-4-thiazolyl)ethenyl)-8,8-(1,3-trimethylen)-4,17-dioxabicyclo[14.1.0]heptadecan-5,9-dion (B)

Aus 100 mg (0,1917 mmol) der unter Beispiel 15 beschriebenen Verbindung werden analog zu Beispiel 7 40 mg der Titelverbindung A und 39 mg der Titelverbindung B erhalten.
¹H-NMR (CDCl₃) von A: δ= 0.94 (3H), 0.96 (3H), 1.27 (3H), 2.68 (3H), 2.90 (2H), 3.08 (1H), 3.59 (1H), 3.77 (1H), 5.67 (1H), 6.11-6.24 (1H), 7.37 (1H) ppm.
¹H-NMR (CDCl₃) von B: δ= 0.89-1.00 (6H), 1.24 (3H), 2.67 (3H), 2.89 (1H), 3.11 (1H), 3.47 (1 H), 3.68-3.81 (2H), 4.46 (1H), 5.68 (1 H), 6.19-6.32 (1H), 7.38 (1 H) ppm.

### Beispiel 17

### (4S,7R,8S,9S,13Z,16S(Z))-4,8-Dihydroxy-16-(1-fluor-2-(2-pyridyl)ethenyl)-7-ethyl-1-oxa-5,5,9,13-tetramethyl-cyclohexadec-13-en-2,6-dion

### Beispiel 17a

### 2-Pyridyl-carbaldehyd

Die Lösung von 50 ml (370 mmol) 2-Picolinsäureethylester in 11 wasserfreiem Dichlormethan kühlt man unter einer Atmosphäre aus trockenem Argon auf -78°C, versetzt mit 500ml einer 1,2 molaren Lösung von Düsobuthylaluminiumhydrid in Toluol und rührt noch 1 Stunde. Man versetzt mit 152 ml Isopropanol, 253 ml Wasser, läßt auf 23°C erwärmen und rührt noch so lange, bis sich ein feinkörniger Niederschlag gebildet hat. Nach Filtration und Lösungsmittelabzug isoliert man 32,6 g (304 mmol, 82%) der Titelverbindung als blass gelbes Öl.
¹H-NMR (CDCl₃): δ = 7,52 (1H), 7,89 (1H), 7,99 (1H), 8,80 (1H), 10,10 (1H) ppm.

### Beispiel 17b

### (2E/Z)-3-(2-Pyridyl)-2-fluor-2-propensäureethylester

Zu 20,7 g einer 55%igen Natriumhydrid-Dispersion in 230 ml wasserfreiem Ethylenglykoldimethylether tropft man unter einer Atmosphäre aus trockenem Argon bei 0°C die Lösung von 115 g 2-Fluor-2-phosphonoessigsäuretriethylester in 230 ml Ethylenglykoldimethylether und rührt 1 Stunde nach. Anschließend versetzt man mit der Lösung von 27,6 g (258 mmol) der nach Beispiel 17a dargestellten Verbindung in 230 ml Ethylenglykoldimethylether und läßt innerhalb 1 Stunde auf 23°C erwärmen. Man gießt auf eine gesättigte Ammoniumchloridlösung, extrahiert mehrfach mit Ethylacetat, wäscht die vereinigten organischen Extrakte mit gesättigter Natriumchloidlösung und trocknet über Natriumsulfat. Den nach Filtration und Lösungsmittelabzug erhaltenen Rückstand reinigt man durch Vakuumdestillation. Isoliert werden 33,7 g (173 mmol, 67%) der Titelverbindungen als farbloses Öl.
¹H-NMR (CDCl₃): δ = 1,22+1,39 (3H), 4,25+4,37 (2H), 6,90+7,13 (1H), 7,23+7,26 (1H), 7,56+7,90 (1H), 7,67+7,76 (1H), 8,59+8,67 (1H) ppm.

### Beispiel 17c

### (2Z)-3-(2-Pyridyl)-2-fluor-2-propensäureethylester

Die Lösung von 29,2 g (149 mmol) des nach Beispiel 17b dargestellten E/Z-Gemisches in 280 ml wasserfreiem Toluol versetzt man unter einer Atmosphäre aus trockenem Argon mit 2,0 g lod und erhitzt 7 Tage auf 100°C. Die erkaltete Lösung wäscht man mit gesättigter Natriumthiosulfatlösung und trocknet über Natriumsulfat. Den nach Filtration und Lösungsmittelabzug erhaltenen Rückstand reinigt man durch Chromatographie an ca. 11 feinem Kiselgel mit einem Gradientensystem aus n-Hexan und Ethylacetat. Isoliert werden 26,3 g (135 mmol, 90%) der Titelverbindung als farbloses Öl.
¹H-NMR (CDCl₃): δ = 1,39 (3H), 4,37 (2H), 7,13 (1H), 7,26 (1H), 7,76 (1H), 7,90 (1H), 8.67(1H)ppm.

### Beispiel 17d

### (2Z)-3-(2-Pyridyl)2-fluor-2-propen-1-ol

Die Lösung von 26,3 g (135 mmol) der nach Beispiel 17c dargestellten Verbindung in 800ml wasserfreiem Tetrahydrofuran kühlt man unter einer Atmosphäre aus trockenem Argon auf -78°C, versetzt mit 80 g Lithium-tri-*tert*.-butoxyaluminiumhydrid, läßt auf 23°C erwärmen und rührt 16 Stunden. Man versetzt mit Wasser, extrahiert mehrfach mit Ethylacetat, wäscht die vereinigten organischen Extrakte mit gesättigter Natriumchloidlösung und trocknet über Natriumsulfat. Den nach Filtration und Lösungsmittelabzug erhaltenen Rückstand reinigt man durch Chromatographie an ca. 1,51 feinem Kiselgel mit einem Gradientensystem aus n-Hexan und Ethylacetat. Isoliert werden 17,3 g (113 mmol, 84%) der Titelverbindung als farbloses Öl.
¹H-NMR (CDCl₃): δ = 1,93 (1H), 4,32 (2H), 6,19 (1H), 7,16 (1H), 7,69 (1H), 7,77 (1H), 8,52 (1H) ppm.

### Beispiel 17e

### (2Z)-3-(2-Pyridyl)-2-fluor-2-propenal

Die Lösung von 17,3 g (113 mmol) der nach Beispiel 17d dargestellten Verbindung in 2,5 I wasserfreiem Toluol versetzt man mit 100 g Braunstein und rührt 16 Stunden bei 23°C. Man filtriert über Celite und isoliert 13,8 g (91 mmol, 81%) der Titelverbindung als blass gelbes Öl.
¹H-NMR (CDCl₃): δ = 6,87 (1H), 7,32 (1H), 7,81 (1H). 7.99 (1H), 8,72 (1H), 9,43 (1 H) ppm.

### Beispiel 17f

### (3S,4Z)-5-(2-Pyridyl)-1-[(4S,5R)-4-methyl-5-phenyl-1,3-oxazolidin-2-on-3-yl]-3-hydroxy-4-fluor-4-penten-1-on (A) und (3R,4Z)-5-(2-Pyridyl)-1-[(4S,5R)-4-methyl-5-phenyl-1,3-oxazolidin-2-on-3-yl]-3-hydroxy-4-fluor-4-penten-1-on (B)

Zu der Lösung von 16,8 ml Diisopropylamin in 800 ml wasserfreiem Tetrahydrofuran tropft man bei -30°C unter einer Atmosphäre aus trockenem Argon 50 ml einer 2,4 molaren Lösung von n-Buthyllithium in n-Hexan, rührt 20 Minuten, kühlt auf -70°C und versetzt innerhalb von 4 Stunden mit der Lösung von 23,6 g (4S,5R)-3-Acetyl-4-methyl-5-phenyloxazolidin-2-on in 800 ml Tetrahydrofuran. Nach 1 Stunde tropft man innerhalb von 2 Stunden die Lösung von 10,3 g (68 mmol) der nach Beispiel 17e dargestellten Verbindung in 390 ml Tetrahydrofuran zu und rührt 16 Stunden bei -70°C. Man gießt auf eine gesättigte Ammoniumchloridlösung, extrahiert mehrfach mit Ethylacetat, wäscht die vereinigten organischen Extrakte mit gesättigter Natriumchloidlösung und trocknet über Natriumsulfat. Den nach Filtration und Lösungsmittelabzug erhaltenen Rückstand trennt man durch wiederholte Chromatographie an feinem Kieselgel mit einem Gradientensystem aus n-Hexan, Ethylacetat und Ethanol. Isoliert werden 8,60 g (23,2 mmol, 34%) der Titelverbindung A als kristalliner Feststoff sowie 5,04 g (13,6 mmol, 20%) der Titelverbindung B als farbloser Schaum.
¹H-NMR (CDCl₃) von A: δ = 0,94 (3H), 3,38 (1H), 3,56 (1H), 4,83 (1H), 4,89 (1 H), 5,70 (1H), 6,33 (1H), 7,14 (1H), 7,23-7,48 (5H), 7,68 (1H), 7,76 (1H), 8,58 (1H) ppm.
¹H-NMR (CDCl₃) von B: δ= 0,94 (3H), 3,47 (2H), 4,19 (1H), 4,81 (1H), 4,89 (1H), 5,72 (1H), 6,29 (1H), 7,16 (1H), 7,22-7,49 (5H), 7,69 (1H), 7,76 (1H), 8,59 (1H) ppm.

### Beispiel 17 g

### (4Z)-5-(2-Pyridyl)-1-[(4S,5R)-4-methyl-5-phenyl-1,3-oxazolidin-2-on-3-yl]-4-fluor-4-penten-1,3-dion

In Analogie zu Beispiel 17e setzt man 3,54 g (9,56 mmol) der nach Beispiel 17f dargestellten Verbindung B um und isoliert nach Aufarbeitung 3,01 g (8,17 mmol, 85%) der Titelverbindung als kristallinen Feststoff.
¹ H-NMR (CDCl₃) als Keton/Enol-Gemisch: δ = 0,97 (3H), 4,39+7,17+13,19 (2H), 4,88 (1H), 5,72+5,76 (1H), 6,99+7,07 (1H), 7,20-7,50 (6H), 7,75+7,78 (1H), 7,91 (1H), 8,65+8,70 (1H) ppm.

### Beispiel 17h

### (3S,4Z)-5-(2-Pyridyl)-1-[(4S,5R)-4-methyl-5-phenyl-1,3-oxazolidin-2-on-3-yl]-3-hydroxy-4-fluor-4-penten-1-on (A) und (3R,4Z)-5-(2-Pyridyl)-1-[(4S,5R)-4-methyl-5-phenyl-1,3-oxazolidin-2-on-3-yl]-3-hydroxy-4-fluor-4-penten-1-on (B)

Die Lösung von 12,2 g (33,1 mmol) der nach beispiel 17g dargestellten Verbindung in einem Gemisch aus 610 ml wasserfreiem Dichlormethan und 65 ml wasserfreiem Methanol versetzt man unter einer Atmosphäre aus trockenem Argon bei -40°C mit 732 mg Natriumborydrid und rührt 1 Stunde. Man gießt in eine gesättigte Natriumhydrogencarbonatlösung, extrahiert mehrfach mit Dichlormethan und trocknet die vereinigten organischen Extrakte über Natriumsulfat. Den nach Filtration und Lösungsmittelabzug erhaltenen Rückstand trennt man durch Chromatographie an feinem Kieselgel mit einem Gradientensystem aus Dichlormethan und Ethanol. Isoliert werden neben Ausgangsmaterial 3,46 g (9,3 mmol, 28%) der Titelverbindung A sowie 3,38 g (9,1 mmol, 28%) der Titelverbindung B die jeweils mit den unter Beispiel 17f beschriebenen Verbindungen identisch sind.

### Beispiel 17i

### (3S,4Z)-5-(2-Pyridyl)-1-[(4S,5R)-4-methyl-5-phenyl-1,3-oxazolidin-2-on-3-yl]-3-[[dimethyl(1,1-dimethylethyl)silyl]oxy]-4-fluor-4-penten-1-on

Die Lösung von 9,96g (26,89 mmol) der nach Beispiel 17f und/oder 1h dargestellten Verbindung A in 85ml wasserfreiem Dichlormethan kühlt man unter einer Atmosphäre aus trockenem Argon auf -70°C, versetzt mit 7 ml 2,6-Lutidin, 12,4 ml Trifluormethansulfonsäure-*tert*.-butyldimethylsilylester und rührt 2 Stunden. Man gießt auf eine gesättigte Natriumhydrogencarbonatlösung, extrahiert mehrfach mit Dichlormethan, wäscht die vereinigten organischen Extrakte mit gesättigter Natriumchloidlösung und trocknet über Natriumsulfat. Den nach Filtration und Lösungsmittelabzug erhaltenen Rückstand trennt man durch Chromatographie an feinem Kieselgel mit einem Gradientensystem aus n-Hexan, Ethylacetat und Ethanol. Isoliert werden 12,9g (26,6 mmol, 99%) der Titelverbindung als farbloses Öl.
¹H-NMR (CDCl₃): δ = 0,16 (6H), 0,90 (12H), 3,29 (1H), 3,59 (1H), 4,78 (1H), 4,92 (1H), 5,67 (1 H), 6,12 (1 H), 7,13 (1 H), 7,24-7,47 (5H), 7,68 (1 H), 7,76 (1 H), 8,58 (1 H) ppm.

### Beispiel 17j

### (3S,4Z)-5-(2-Pyridyl)-3-[[dimethyl(1,1-dimethylethyl)silyl]oxy]-4-fluor-4-pentensäureethylester

Die Lösung von 12,8g (26,5 mmol) der nach Beispiel 17i dargestellten Verbindung in 130 ml wasserfreiem Ethanol versetzt man bei 23°C unter einer Atmosphäre aus trockenem Argon mit 6,7 ml Titantetraethylat und erhitzt 2- Stunden auf 85°C. Man engt ein und reinigt den Rückstand durch Chromatographie an feinem Kieselgel mit einem Gradientensystem aus n-Hexan und Ethylacetat. Isoliert werden 9,3g (26,3 mmol, 99%) der Titelverbindung als farbloses Öl.
¹H-NMR (CDCl₃): δ = 0,12 (6H), 0,91 (9H), 1,28 (3H), 2,72 (2H), 4,17 (2H), 4,77 (1H), 6,09 (1H), 7,15 (1H), 7,68 (1H), 7,73 (1H), 8,59 (1H) ppm.

### Beispiel 17k

### (3S,4Z)-5-(2-Pyridyl)-3-[[dimethyl(1,1-dimethylethyl)silyl]oxy]-4-fluor-4-penten-1-ol

In Analogie zu Beispiel 17a setzt man 9,7g (27,4 mmol) der nach Beispiel 17j dargestellten Verbindung um und isoliert nach Aufarbeitung und Reinigung 6,8g (21,8 mmol, 80%) der Titelverbindung als farbloses Öl.
¹H-NMR (CDCl₃): δ = 0,12 (3H), 0,14 (3H), 0,93 (9H), 1,83 (1H), 2,00 (2H), 3,78 (1H), 3,85 (1H). 4,53 (1 H), 6,09 (1 H), 7,12 (1H), 7,65 (1 H), 7,72 (1H). 8,57 (1 H) ppm.

### Beispiel 17l

### (3S,4Z)-5-(2-Pyridyl)-3-[[dimethyl(1,1-dimethylethyl)silyl]oxy]-1-iod-4-fluor-4-penten

Die Lösung von 6,75g Triphenylphosphin in 120 ml wasserfreiem Dichlormethan versetzt man bei 23°C unter einer Atmosphäre aus trockenem Argon mit 1,78 g Imidazol, 6,47g lod und tropft unter Kühlung die Lösung von 6,8g (21,8 mmol) der nach Beispiel 17k dargestellten Verbindung in 40 ml Dichlormethan zu. Man rührt 1 Stunde und reinigt direkt durch Chromatographie an feinem Kieselgel mit einem Gradientensystem aus n-Hexan und Ethylacetat. Isoliert werden 6,7g (15,9 mmol, 73%) der Titelverbindung als farbloses Öl.
¹H-NMR (CDCl₃): δ = 0,13 (3H), 0,19 (3H), 0,93 (9H), 2,25 (2H), 3,28 (2H), 4,38 (1H), 6,09 (1H), 7,17 (1H), 7,69 (1H), 7,75 (1H), 8,58 (1H) ppm.

### Beispiel 17m

### (3S,4Z)-5-(2-Pyridyl)-3-[[dimethyl(1,1-dimethytethyl)sityl]oxy]-4-fluor-4-penten-1-triphenylphosphoniumiodid

6,7g (15,9 mmol) der nach Beispiel 17I dargestellten Verbindung versetzt man mit 8,4 ml. Ethyldiisopropylamin, 50,3g. Triphenylphosphin und erwärmt 4 Stunden auf 85°C. Den öligen Rückstand reinigt man durch Chromatographie an feinem Kieselgel mit einem Gradientensystem aus n-Hexan und Ethylacetat. Isoliert werden 8,9g (13,0 mmol, 82%) der Titelverbindung als kristalliner Feststoff.
¹H-NMR (CDCl₃): δ = 0,16 (3H), 0,22 (3H), 0,90 (9H), 2,01 (1H), 2,18 (1H), 3,50 (1H), 4,07 (1H), 4,90 (1H), 6,19 (1H), 7,12 (1H), 7,59-7,88 (17H), 8,54 (1H) ppm.

### Beispiel 17n

### (2S,6E2,9S,10Z)-9-[[Dimethyl(1,1-dimethylethyl)silyl]oxy]-10-fluor-11-(2-pyridyl)-1-(tetrahydropyran-2-yloxy)-2,6-dimethyl-undeca-6,10-dien

Die Suspension von 3,32 g (4,86 mmol) der nach Beispiel 17m dargestellten Verbindung in 22 ml wasserfreiem Tetrahydrofuran versetzt man bei 0°C unter einer Atmosphäre aus trockenem Argon mit 4,86 ml einer 1 M Lösung von Natrium-bis-(trimethylsilyl)-amid in Tetrahydrofuran. Zu der roten Lösung tropft man langsam die Lösung von 753 mg (3,30 mmol) (2S)-2-Methyl-6-oxo-heptan-1-(tetrahydropyran-2-yloxy), das man in Analogie zu den in DE 197 51 200.3 beschriebenen Verfahren hergestellt hat, in 22 ml Tetrahydrofuran, läßt 3 Stunden rühren, gießt auf gesättigte Ammmoniumchloridlösung und extrahiert mehrfach mit Ethylacetat. Die vereinigten organischen Extrakte trocknet man über Natriumsulfat und engt im Vakuum ein. Nach Säulenchromatographie an Kieselgel mit einem Gradientensystem aus n-Hexan und Ethylacetat werden neben 1,30 g (2,57 mmol, 78%) der Titelverbindung als farblosen schaum erhalten.
¹H-NMR (CDCl₃): δ = 0,10 (6H), 0,83-0,96 (12H), 1,10 (1H), 1,20-2,07 (12H), 1,60+1,68 (3H), 2,43 (2H), 3,04-3,27 (1H), 3,42-3,63 (2H), 3,85 (1H), 4,22 (1H), 4,57 (1H), 5,19 (1H), 6,04 (1H), 7,13 (1H), 7,68 (1H), 7,76 (1H), 8,57 (1H) ppm.

### Beispiel 17o (2S,6E/Z,9S,10Z)-9-[[Dimethyl(1,1-dimethylethyl)silyl]oxy]-10-fluor-11-(2-pyridyl)-1-hydroxy-2,6-dimethyl-undeca-6,10-dien

Zu einer Lösung von 1,30 g (2,57 mmol) der nach Beispiel 17n hergestellten Verbindung in 50 ml Ethanol gibt 700 mg Pyridinium-p-toluolsulfonat und rührt 3 Stunden bei 23°C. Anschließend wird im Vakuum eingeengt und der so erhaltene Rückstand durch Chromatographie an Kieselgel mit einem Gradientensystem aus n-Hexan und Ethylacetat gereinigt. Isoliert werden 832 mg (1,97 mmol, 77%) der Titelverbindung als farbloses Öl.
¹H-NMR (CDCl₃): δ = 0,11 (6H), 0,88+0,91 (3H), 0,95 (9H), 1,07 (1H), 1,24-1,71 (5H), 1,60+1,69 (3H), 1,92-2,11 (2H), 2,34-2,58 (2H), 3,34-3,54 (2H), 4,24 (1H), 5,19 (1H), 6,00+6,02 (1H), 7,12 (1H), 7,66 (1H), 7,75 (1H), 8,56 (1H) ppm.

### Beispiel 17p

### (2S,6E/Z,9S,10Z)-9-[[Dimethyl(1,1-dimethylethyl)silyl]oxy]-10-fluor-11-(2-pyridyl)-2,6-dimethyl-undeca-6,10-dienal

Zu 598 µl Oxalylchlorid gelöst in 25 ml Dichlormethan tropft man unter Stickstoff vorsichtig bei -70°C 971 µl Dimethylsulfoxid und rührt 10 Minuten bei dieser Temperatur. Anschließend tropft man eine Lösung von 1,45 g (3,44 mmol) des nach Beispiel 17o hergestellten Alkohols in 25 ml Dichlormethan zu und rührt 0,5 Stunden zwischen -60°C und -70°C. Dann gibt man 2,84 ml Triethylamin zu und nach 1 Stunde Rühren bei -60°C wird die Reaktionsmischung auf 30 ml Wasser gegeben. Nach Phasentrennung wird die wäßrige Phase mehrfach mit Dichlormethan extrahiert. Die vereinigten organischen Phasen werden mit gesättigter Natriumchlorid-Lösung gewaschen. Nach dem Trocknen über Natriumsulfat und Filtration wird im Vakuum eingeengt. Den Rückstand reinigt man durch Chromatographie an feinem Kieselgel mit einem Gradientensystem aus n-Hexan und Ethylacetat. Man erhält 1,31 g (3,12 mmol, 91 %) der Titelverbindung als farbloses Öl.

### Beispiel 17q

### (4S(4R,5S,6S,10E/Z,13S,14Z))-4-(13-[[(1,1-Dimethylethyl)dimethylsilyl]oxy]-4-ethyl-14-fluor-15-(2-pyridyl)-3-oxo-5-hydroxy-2,6,10-trimethyl-pentadeca-10,14-dien-2-yl)-2,2-dimethyl-[1,3]dioxan (A) und (4S(4S,5R,6S,10E/Z,13S,14Z))-4-(13-[[(1,1-Dimethylethyl)dimethylsilyl]oxy]-4-ethyl-14-fluor-15-(2-pyridyl)-3-oxo-5-hydroxy-2,6,10-trimethyl-pentadeca-10,14-dien-2-yl)-2,2-dimethyl-[1,3]dioxan (B)

Die Lösung von 1,57 ml Diisopropylamin in 40 ml wasserfreiem Tetrahydrofuran kühlt man unter einer Atmosphäre aus trockenem Argon auf -30°C, versetzt mit 4,72 ml einer 2,4 molaren Lösung von n-Butyllithium in n-Hexan und rührt noch 30 Minuten. Bei - 78°C tropft man die Lösung von 1,31 g (3,12 mmol) der nach Beispiel 17p dargestellten Verbindung in 40 ml Tetrahydrofuran zu und läßt 1 Stunde reagieren. Anschließend versetzt man mit der Lösung von 2.36 g (10,3 mmol) (4S)-4-(2-Methyl-3-oxo-hex-2-yl)-2,2-dimethyl-[1,3]dioxan, das man nach dem in DE 19751200.3 beschriebenen Verfahren hergestellt hat, in 40 ml Tetrahydrofuran und gießt nach 60 Minuten in gesättigte Ammoniumchloridlösung. Man verdünnt mit Wasser, extrahiert mehrfach mit Ethylacetat, wäscht die vereinigten organischen Extrakte mit gesättigter Natriumchloridlösung, trocknet über Natriumsulfat und engt im Vakuum ein. Nach Säulenchromatographie an Kieselgel mit einem Gradientensystem aus n-Hexan und Ethylacetat werden neben Ausgangsmaterial 1,56 g (2,41 mmol, 77%) der Titelverbindung A sowie 287 mg (0,44 mmol, 14%) der Titelverbindung B erhalten.
¹H-NMR (CDCl₃) von A: δ = 0,09 (6H), 0,81 (3H), 0,85 (3H), 0,92 (9H), 1,00 (3H), 1,08 (1H), 1,18-1,83 (8H), 1,26 (3H), 1,32 (3H), 1,39 (3H), 1,60+1,68 (3H), 1,88-2,08 (2H), 2,32-2,52 (2H), 2,87+2,91 (1H), 3,19 (1H), 3,44 (1H), 3,87 (1H), 3,98 (1H), 4,16 (1H), 4,22 (1H), 5,18 (1H), 6,00 (1H), 7,11 (1H), 7,65 (1H), 7,73 (1H), 8,56 (1H) ppm.

### Beispiel 17r

### (3S,6R,7S,8S,12E/Z,15S,16Z)-15-[[(1,1-Dimethylethyl)dimethylsilyl]oxy]-6-ethyl-16-fluor-1,3,7-trihydroxy-4,4,8,12-tetramethyl-17-(2-pyridyl)-heptadeca-12,16-dien-5-on

Die Lösung von 1,45 g (2,24 mmol) der nach Beispiel 17q dargestellten Verbindung in 36 ml wasserfreiem Ethanol versetzt man unter einer Atmosphäre aus trockenem Argon mit 1,06 g Pyridinium-p-Toluolsulfonat und rührt 4 Stunden bei 23°C. Nach Lösungsmittelabzug chromatographiert man den Rückstand an feinem Kieselgel mit einem Gemisch aus n-Hexan und Ethylacetat. Isoliert werden 1,36 g (2,24 mmol, 93%) der Titelverbindung als farbloses Öl.
¹H-NMR (CDCl₃): δ = 0,10 (6H), 0,78-0,90 (6H), 0,92 (9H), 0,99-2,12 (11H), 1,08 (3H), 1,26 (3H), 1,58+1,68 (3H), 2,32-2,53 (2H), 2,79-3,03 (2H), 3,19 (1H), 3,41 (1H), 3,73-3,93 (3H), 4,06-4,25 (2H), 5,13+5,21 (1H), 5,93 (1H), 7,13 (1H), 7,67 (1H), 7,77 (1H), 8,58 (1 H) ppm.

### Beispiel 17s

### (3S,6R,7S,8S,12E/Z,15S,16Z)-6-Ethyl-1,3,7,15-tetrakis-[[(1,1-dimethylethyl)dimethylsilyl]oxy]-16-fluor-4,4,8,12-tetramethyl-17-(2-pyridyl)-heptadeca-12,16-dien-5-on

Die Lösung von 1,36 g (2,24 mmol) der nach Beispiel 17r dargestellten Verbindung in ml wasserfreiem Dichlormethan kühlt man unter einer Atmosphäre aus trockenem Argon auf -78°C, versetzt mit 3,45 ml 2,6-Lutidin, 3,36 ml Triflüormethänsülfonsäure- - tert.butyldimethylsilylester, läßt innerhalb von 2 Stunden auf 0°C erwärmen und rührt noch 2 Stunden. Man gießt in gesättigte Natriumhydrogencarbonatlösung und extrahiert mehrfach mit Dichlormethan. Die vereinigten organischen Extrakte trocknet man über Natriumsulfat und engt im Vakuum ein. Nach Säulenchromatographie an Kieselgel mit einem Gradientensystem aus n-Hexan und Ethylacetat isoliert man 1,83 g (1,92 mmol, 86%) der Titelverbindung als farbloses Öl.
¹H-NMR (CDCl₃): δ = 0,00-0,12 (24H), 0,83 (3H), 0,85-0,98 (39H), 1,00-1,82 (9H), 1,03 (3H), 1,21 (3H), 1,61+1,68 (3H), 1,98 (2H), 2,42 (2H), 3,01. (1H), 3,47-3,73 (2H), 3,82 (1H), 3,91 (1H), 4,21 (1H), 5,19 (1H), 6,01 (1H), 7,12 (1H), 7,65 (1H), 7,73 (1H), 8,58 (1H) ppm.

### Beispiel 17t

### (3S,6R,7S,8S,12E/Z,15S,16Z)-6-Ethyl-3,7,15-tris-[[(1,1-dimethylethyl)dimethylsilyl]oxy]-1-hydroxy-16-fluor-4,4,8,12-tetramethyl-17-(2-pyridyl)-heptadeca-12,16-dien-5-on

Die Lösung von 1,83 g (1,92 mmol) der nach Beispiel 17s dargestellten Verbindung in einem Gemisch aus 20 ml Dichlormethan und 20 ml Methanol versetzt man bei 23°C unter einer Atmosphäre aus trockenem Argon mit 446 mg Campher-10-sulfonsäure und rührt 2 Stunden. Man gießt in eine gesättigte Natriumhydrogencarbonatlösung und extrahiert mehrfach mit Dichlormethan. Die vereinigten organischen Extrakte trocknet man über Natriumsulfat und engt im Vakuum ein. Nach Säulenchromatographie an feinem Kieselgel mit einem Gradientensystem aus n-Hexan und Ethylacetat isoliert man 1,40 g (1,67 mmol, 87%) der Titelverbindung als farbloses Öl.
¹H-NMR (CDCl₃): δ = 0,02-0,14 (18H), 0,85 (3H), 0,88-0,97 (30H), 1,03-1,80 (9H), 1,08 (3H), 1,20 (3H), 1,60+1,68 (3H), 1,90-2,06 (3H), 2,42 (2H), 3,01 (1H), 3,68 (2H), 3,83 (1H), 4,08 (1H). 4,21 (1H). 5,18 (1H). 6,01 (1H). 7,12 (1H), 7,63 (1H), 7,72 (1H). 8,56 (1H) ppm.

### Beispiel 17u

### (3S,6R,7S,8S,12E/Z,15S,16Z)-6-Ethyl-3,7,15-tris-[[(1,1-dimethylethyl)dimethylsilyl]oxy]-16-fluor-5-oxo-4,4,8,12-tetramethyl-17-(2-pyridyl)-heptadeca-12,16-dienal

Die Lösung von 400 µl Oxalylchlorid in 16 ml wasserfreiem Dichlormethan kühlt man unter einer Atmosphäre aus trockenem Argon auf -70°C, versetzt mit 650 µl Dimethylsulfoxid, der Lösung von 1,51 g (1,81 mmol) der nach Beispiel 17t dargestellten Verbindung in 16 ml wasserfreiem Dichlormethan und rührt 0,5 Stunden. Anschließend versetzt man mit 2 ml Triethylamin, läßt 1 Stunde bei -30°C reagieren und versetzt mit n-Hexan und gesättigter Natriumhydrogencarbonatlösung. Die organische Phase wird abgetrennt, die wässrige noch mehrfach mit n-Hexan extrahiert, die vereinigten organischen Extrakte mit Wasser gewaschen und über Magnesiumsulfat getrocknet. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 1,48 g (1,77 mmol, 98%) der Titelverbindung als blass gelbes Öl.
¹H-NMR (CDCl₃) einer gereinigten Probe: δ = 0,02-0,13 (18H), 0,82 (3H), 0,85-0,97 (30H), 1,01-1,80 (7H), 1,10 (3H), 1,22 (3H), 1,60+1,68 (3H), 1,89-2,07 (2H), 2,32-2,48 (3H), 2,57 (1H), 3,00 (1H), 3,81 (1H), 4,21 (1H), 4,48 (1H), 5,18 (1H), 6,01 (1H), 7,12 (1H), 7,66 (1H), 7,73 (1H), 8,57 (1H), 9,78 (1H) ppm.

### Beispiel 17v

### (3S,6R,7S,8S,12Z,15S,16Z)-6-Ethyl-3,7,15-tris-[[(1,1-dimethylethyl)dimethylsilyl]oxy]-16-fluor-5-oxo-4,4,8,12-tetramethyt-17-(2-pyridyl)-heptadeca-12,16-diensäure (A) und (3S,6R,7S, 8S,12E,15S,16Z)-6-Ethyl-3,7,15-tris-[[(1,1-dimethylethyl)dimethylsilyl]oxy]-16-fluor-5-oxo-4,4,8,12-tetramethyl-17-(2-pyridyl)-heptadeca-12,16-diensäure (B)

Die Lösung von 1,48 g (1,77 mmol) der nach Beispiel 17u dargestellten Verbindung in 54 ml tert.-Butanol versetzt man mit 50 ml einer 2 molaren Lösung von 2-Methyl-2-buten in Tetrahydrofuran, kühlt auf 2°C, versetzt mit 14 ml Wasser, 731 mg Natriumdihydrogenphosphat, 1,24 g Natriumchlorit, läßt auf 15°C erwärmen und rührt 2 Stunden. Man gießt in gesättigte Natriumthiosulfatlösung, verdünnt mit Wasser und extrahiert mehrfach mit Ethylacetat. Die vereinigten organischen Extrakte trocknet man über Natriumsulfat und reinigt den nach Filtration und Lösungsmittelabzug erhaltenen Rückstand durch Chromatographie an feinem Kieselgel mit einem Gradientensystem aus n-Hexan und Ethylacetat. Isoliert werden 487 mg (573 µmol, 32%) der Titelverbindung A sowie 506 mg (595 µmol, 34%) der Titelverbindung B jeweils als farbloses Öl.
¹H-NMR (CDCl₃) von A: δ = 0,00 (3H), 0,03-0,11 (12H), 0,13 (3H), 0,79-0,98 (33H), 1,03-1,80 (8H), 1,12 (3H), 1,20 (3H), 1,71 (3H), 1,89 (1H), 2,18 (1H), 2,30-2,48 (3H), 2,52 (1H), 3,03 (1H). 3,75 (1H), 4,22 (1H). 4,41 (1H). 5,20 (1H). 6,38 (1H). 7,20 (1H). 7,72 (1 H), 7,82 (1 H), 8,51 (1 H) ppm.
¹H-NMR (COCl₃) von B: δ = 0,00 (3H), 0,04 (3H), 0,07 (3H), 0,09 (3H), 0,11 (3H), 0,15 (3H), 0,74-0,95 (33H), 0,99-1,72 (8H), 1,10 (3H), 1,22 (3H), 1,53 (3H), 1,86 (1H), 1,98 (1H), 2,27-2,66 (4H), 3,08 (1H), 3,82 (1H), 4,16 (1H), 4,33 (1H), 5,13 (1H), 6,04 (1H), 7,18 (1H), 7,71 (1H), 7,82 (1H), 8,52 (1H) ppm.

### Beispiel 17w

### (3S,6R,7S,8S,12Z,15S,16Z)-6-Ethyl-3,7-bis-[[(1,1-dimethylethyl)dimethylsilyl]oxy]-16-fluor-15-hydroxy-5-oxo-4,4,8,12-tetramethyl-17-(2-pyridyl)-heptadeca-12,16-diensäure

Die Lösung von 487 mg (573 µmol) der nach Beispiel 17v dargestellten Verbindung A in 23 ml wasserfreiem Tetrahydrofuran versetzt man unter einer Atmosphäre aus trockenem Argon mit 8,55 ml einer 1 molaren Lösung von Tetrabutylammoniumfluorid in Tetrahydrofuran und rührt 1,5 Stunden bei 23°C. Man versetzt mit gesättigter Natriumhydrogencarbonatlösung, extrahiert mehrfach mit Ethylacetat, wäscht mit gesättigter Natriumchloridlösung und trocknet über Natriumsulfat. Den nach Filtration und Lösungsmittelabzug erhaltenen Rückstand setzt man ohne Reinigung weiter um.

### Beispiel 17x

### (4S,7R,8S,9S,13Z,16S(Z))-4,8-Bis-[[dimethyl(1,1-dimethylethyl)silyl]oxy]-16-(1-fluor-2-(2-pyridyl)ethenyl)-7-ethyl-1-oxa-5,5, 9,13-tetramethyl-cyclohexadec-13-en-2,6-dion

Die Lösung von 486 mg (max. 570 µmol) der nach Beispiel 17w dargestellten Verbindung in einem Gemisch aus 5 ml wasserfreiem Tetrahydrofuran und 50 ml Toluol versetzt man unter einer Atmosphäre aus trockenem Argon mit 474 µl Triethylamin, 454µl 2,4,6-Trichlorbenzoylchlorid und rührt 20 Minuten. Man tropft diese Lösung innerhalb von 4,5 Stunden zu der Lösung von 727mg 4-Dimethylaminopyridin in 215ml Toluol und rührt 0,5 Stunden bei 23°C nach. Man engt ein, nimmt in wenig Dichlormethan auf und reinigt durch Chromatographie an feinem Kieselgel mit einem Gradientensystem aus n-Hexan und Ethylacetat. Isoliert werden 310mg (432 µmol, 76%) der Titelverbindung als farbloses Öl.

### Beispiel 17

### (4S,7R,8S.9S,13Z,16S(Z))-4,8-Dihydroxy-16-(1-fluor-2-(2-pyridyl)ethenyl)-7-ethyl-1-oxa-5,5,9,13-tetramethyl-cyclohexadec-13-en-2,6-dion

Die Lösug von 308. mg (429 µmol) der nach Beispiel 17x dargestellten Verbindung in 27 ml wasserfreiem Tetrahydrofuran versetzt man unter einer Atmosphäre aus trockenem Argon portionsweise mit insgesamt 4,6 ml HF-Pyridin-Komplex und rührt bei 23°C 24 Stunden. Man gießt in gesättigte Natriumhydrogencarbonatlösung, extrahiert mehrfach mit Dichlormethan und trocknet die vereinigten organischen Extrakte über Natriumsulfat. Nach Filtration und Lösungsmittelabzug reinigt man den erhaltenen Rückstand durch Chromatographie an feinem Kieselgel mit einem Gemisch aus n-Hexan und Ethylacetat. Isoliert werden 135 mg (276 µmol, 64%) der Titelverbindung als farbloses Öl.
¹H-NMR (CDCl₃): δ = 0,88 (3H), 1,03 (3H), 1,10 (3H), 1,13-1,95 (8H), 1,32 (3H), 1,71 (3H), 2,28 (1H), 2,34-2,49 (3H), 2,56 (1H), 2,80 (1H), 3,21 (1H), 3,56 (1H), 3,70 (1H), 4,22 (1H), 5,13 (1H), 5,41 (1H), 6,12 (1H), 7,16 (1H), 7,63-7,75 (2H), 8,53 (1H) ppm.

### Beispiel 18

### (4S,7R,8S,9S,13E,16S(Z))-4,8-Dihydroxy-16-(1-fluor-2-(2-pyridyl)ethenyl)-7-ethyl-1-oxa-5,5,9,13-tetramethyl-cyclohexadec-13-en-2,6-dion

### Beispiel 18a

### (3S,6R,7S,8S,12E,15S,16Z)-6-Ethyl-3,7-bis-[[(1,1-dimethylethyl)dimethylsilyl]oxy]-16-fluor-15-hydroxy-5-oxo-4,4,8,12-tetramethyl-17 -(2-pyridyl)-heptadeca-12, 16-diensäure

In Analogie zu Beispiel 17w setzt man 506 mg (595 µmol) der nach Beispiel 17v dargestellten Verbindung B um und setzt das nach Aufarbeitung erhaltene Rohprodukt weiter umsetzt.

### Beispiel 18b

### (4S,7R,8S,9S,13E,16S(Z))-4,8-Bis-[[dimethyl(1,1-dimethylethyl)silyl]oxy]-16-(1-fluor-2-(2-pyridyl)ethenyl)-7-ethyl-1-oxa-5,5,9,13-tetramethyl-cyclohexadec-13-en-2,6-dion

In Analogie zu Beispiel 17x setzt man 577mg (max. 595 µmol) der nach Beispiel 18a dargestellten Verbindung um und isoliert nach Aufarbeitung und Reinigung 273 mg ( 380 µmol, 64%) der Titelverbindung als farbloses Öl.
¹H-NMR (CDCl₃): δ = 0,01-0,13 (12H), 0,78-0,96 (24H), 1,09 (3H), 1,20 (3H), 1,26-1,90 (8H), 1,59 (3H), 2,16 (1H), 2,39 (1H), 2,59 (1H), 2,68 (2H), 2,91 (1H), 3,91 (1H), 4,35 (1H), 5,22 (1H), 5,45 (1H), 6,08 (1H), 7,12 (1H), 7,65 (1H), 7,71 (1H), 8,56 (1H) ppm.

### Beispiel 18

### (4S,7R,8S,9S,13E,16S(Z))-4,8-Dihydroxy-16-(1-fluor-2-(2-pyridyl)ethenyl)-7-ethyl-1-oxa-5,5,9,13-tetramethyl-cyclohexadec-13-en-2,6-dion

In Analogie zu Beispiel 18 setzt man 273 mg (380 µmol) der nach Beispiel 18b dargestellten Verbindung um und isoliert nach Aufarbeitung und Reinigung 115 mg (235 µmol, 62%) der Titelverbindung als farbloses Öl.
¹H-NMR (CDCl₃): δ = 0,72 (1H), 0,84 (3H), 1,00 (6H), 1,22-2,02 (8H), 1,30 (3H), 1,60 (3H), 2,21 (1H), 2,33-2,57 (3H), 2,62 (1H), 3,40 (1H), 3,78 (1H), 4,51 (1H), 5,09 (1H), 5,22 (1H), 5,53 (1H), 6,11 (1H), 7,16 (1H), 7,70 (1H), 7,80 (1H), 8,43 (1H) ppm.

### Beispiel 19

### (1SR,3S(Z),7S,10R,11S,12S,16RS)-7,11-Dihydroxy-10-ethyl-3-(1-fluor-2-(N-oxido-2-pyridyl)ethenyl)-8,8,12,16-tetramethyl-4,17-dioxabicyclo[14.1.0]heptadecan-5,9-dion

Die Lösung von 50 mg (102 µmol) der nach Beispiel 17 dargestellten Verbindung in 3 ml Acetonitril versetzt man bei 0°C mit 958 µl einer 0,1 M wässrigen Lösung von Ethylendiamintetraacetat, 1,45 ml Trifluoraceton, 373 mg Natriumhydrogencarbonat, 448 mg Oxone und rührt 1,5 Stunden bei 23°C. Man versetzt mit Natriumthiosulfatlösung, extrahiert mehrfach mit Ethylacetat, wäscht die vereinigten organischen Extrakte mit gesättigter Natriumchloridlösung und trocknet über Natriumsulfat. Isoliert werden 61 mg (max. 102 µmmol) der Titelverbindungen, die man ohne Reinigung weiter umsetzt.

### Beispiel 20

### (1S,3S(Z),7S,10R,11S,12S,16R)-7,11-Dihydroxy-10-ethyl-3-(1-fluor-2-(2-pyridyl)ethenyl)-8,8,12,16-tetramethyl-4,17-dioxabicyclo[14.1.0]heptadecan-5,9-dion (A) und (1R,3S(Z),7S,10R,11S,12S,16S)-7,11-Dihydroxy-10-ethyl-3-(1-fluor-2-(2-pyridyl)ethenyl)-8,8,12,16-tetramethyl-4,17-dioxabicyclo[14.1.0]heptadecan-5,9-dion (B)

Die Lösung von 60 mg (max. 102 µmol) der nach Beispiel 19 dargestellten Verbindungen in 12 ml Trichlormethan versetzt man unter einer Atmosphäre aus trockenem Argon mit Molsieb, 2,2 ml Isopropanol, 39 mg Tetrapropylammoniumperruthenat und rührt 2 Tage bei 60°C. Man engt ein und reinigt den Rückstand durch Chromatographie an analytischen Dünnschichtplatten. Als Laufmittel dient ein Gemisch aus Dichlormethan und Isopropanol, als Elutionsmittel ein Gemisch aus Dichlormethan und Methanol. Isoliert werden 17 mg (34 µmol, 28%) der Titelverbindung A sowie 4,3 mg (9 µmol, 8%) der Titelverbindung B.
¹H-NMR (CDCl₃) von A: δ = 0,87 (3H), 1,00 (3H), 1,04 (3H), 1,25-1,98 (9H), 1,29 (3H), 1,37 (3H), 2,10-2,21 (2H), 2,42 (1H), 2,51 (1H), 2,62 (1H), 2,89 (1H), 3,33 (1H), 3,69 (1 H), 4,21 (1 H), 4,44 (1 H), 5,69 (1 H), 6,17 (1H), 7,18 (1 H), 7,70 (2H), 8,54 (1 H) ppm.
¹H-NMR (CDCl₃) von B: δ = 0,83 (3H), 0,94 (3H), 1,07 (3H), 1,16-2,03 (10H), 1,30 (3H), 1,38 (3H), 2,27 (1H), 2,49-2,52 (2H), 2,90-3,04 (2H), 3,21 (1H), 3,72 (1H), 3,89 (1 H), 4,32 (1 H), 5,78 (1 H), 6,19 (1 H), 7,18 (1H), 7,61-7,79 (2H), 8,52 (1 H) ppm.

### Beispiel 21

### (1SR,3S(Z),7S,10R,11 S,12S,16SR)-7,11-Dihydroxy-10-ethyl-3-(1-fluor-2-(N-oxido-2-pyridyl)ethenyl)-8,8,12,16-tetramethyl-4,17-dioxabicyclo[14.1.0]heptadecan-5,9-dion

In Analogie zu Beispiel 19 setzt man 112 mg (229 µmol) der nach Beispiel 18 dargestellten Verbindung um und isoliert nach Aufarbeitung 150 mg (max. 229 µmol) der Titelverbindungen als farbloses Öl.

### Beispiel 22

### (4S,7R,8S,9S,13(Z),16S(Z))-4,8-Dihydroxy-7-ethyl-16-(1-fluor-2-(2-methyl-4-thiazolyl)ethenyl)-1 -oxa-5,5,9,13-tetramethyl-cyclohexadec-1 3-en-2,6-dion

In Analogie zu Beispiel 1 und 5 unter Verwendung von (3S)-1,3-Bis[[dimethyl(1,1-dimethyl)silyl]oxy]-4,4-dimethyl-octan-5-on als Aldolkomponente (siehe Beispiel 5n) erhält man 86 mg der Titelverbindung als schwach gelbgefärbtes Öl.
¹H-NMR (CDCl₃): δ= 0.87 (3H), 1.04 (3H), 1.15-1.75 (8H), 1.10 (3H), 1.33 (3H), 1.72 (3H), 1.86 (2H), 2.20-2.40 (2H), 2.42 (1H), 2.56 (1H), 2.73 (3H), 2.82 (1H), 3.23 (1H), 3.71 (1H), 4.18 (1H), 5.12 (1H), 5.42 (1H), 6.23 (1H), 7.38 (1H) ppm.

### Beispiel 23

### (4S,7R,8S,9S,13(E),16S(Z))-4,8-Dihydroxy-7-ethyl-16-(1-fluor-2-(2-methyl-4-thiazolyl)ethenyl)-1-oxa-5,5,9,13-tetramethyl-cyclohexadec-13-en-2,6-dion

In Analogie zu Beispiel 2 und 6 unter Verwendung von (3S)-1,3-Bis[[dimethyl(1,1-dimethyl)silyl]oxy]-4,4-dimethyl-octan-5-on als Aldolkomponente (siehe Beispiel 5n) erhält man 96 mg der Titelverbindung als schwach gelbgefärbtes Öl.
¹H-NMR (CDCl₃): δ= 0.85 (3H), 0.7-1.6 (7H), 1.00 (3H), 1.02 (3H), 1.31 (3H), 1.62 (3H), 1.78 (1H), 1.82-2.01 (2H), 2.20 (1H), 2.40-2.67 (3H), 2.68 (3H), 3.37 (1H), 3.73 (1H), 4.40 (1H), 4.48 (1H), 5.10 (1H), 5.53 (1H), 6.15 (1H), 7.35 (1H) ppm.

### Beispiel 24

### (1R,3S(Z),7S,10R,11S,12S,16S)-7,11-Dihydroxy-10-ethyl-3-(1-fluor-2-(2-methyl-4-thiazolyl)ethenyl)-8,8,12,16-tetramethyl-4,17-dioxabicyclo[14.1.0]heptadecan-5,9-dion (A) und

### (1S,3S(Z),7S,10R,11S,12S,16R)-7,11-Dihydroxy-10-ethyl-3-(1-fluor-2-(2-methyl-4-thiazolyl)ethenyl)-8,8,12,16-tetramethyl-4,17-dioxabicyclo[14.1.0]heptadecan-5,9-dion (B)

In Analogie zu Beispiel 3 erhält man aus der in Beispiel 22 hergestellten Titelverbindung 8 mg der Titelverbindung A und B im Verhältnis 1:4 als schwach gelbgefärbtes Öl.
¹H-NMR (charakteristische Signale des Gemisches A und B, CDCl₃):δ= 0.86 (3H), 0.94 (3H, A), 1.00 (3H, B), 1.05 (3H), 1.26 (3H), 1.29 (3H), 1.36 (3H), 1.69 (1H. B), 1.75-1.95 (1H), 2.34 (1H, B), 2.22 (1H. A), 2.44 (1H), 2.60 (1H). 2.75 (3H), 2.86 (1H. B), 2.97 (1H. A), 3.22 (1H. A), 3.35 (1H. B), 3.70 (1H. B), 3.88 (1H, A), 4.21 (1H. B), 4.31 (1H. A), 5.68 (1H. B), 5.76 (1H. A), 6.29 (1H). 7.41 (1H) ppm.

Die reinen Titelverbindungen A und B werden durch HPLC an einer Chiralpak AD 10µ Säule mit Hexan/Ethanol 20-50% getrennt.

### Beispiel 25

### (1R,3S(Z),7S,10R,11S,12S,16R)-7,11-Dihydroxy-10-ethyl-3-(1-fluor-2-(2-methyl-4-thiazolyl)ethenyl)-8,8,12,16-tetramethyl-4,17-dioxabicyclo[14.1.0]heptadecan-5,9-dion (A) und

### (1S,3S(Z)7S,10R,11S,12S,16S)-7,11-Dihydroxy-10-ethyl-3-(1-fluor-2-(2-methyl-4-thiazolyl)ethenyl)-8,8,12,16-tetramethyl-4,17-dioxabicyclo[14.1.0]heptadecan-5,9-dion (B)

In Analogie zu Beispiel 3 erhält man aus der in Beispiel 23 hergestellten Titelverbindung 4,9 mg der Titelverbindung A als unpolare Komponente und 3,4 mg der Titelverbindung B als polare Komponente als farblose Öle.
¹H-NMR (CDCl₃) von A, δ= 0.86 (3H), 0.95 (3H), 1.0-1.7 (6H), 1.04 (3H), 1.30 (3H), 1.38 (3H), 1.76 (1H), 1.85 (2H), 1.90-2.30 (3H), 2.55 (2H), 2.70 (3H), 2.89 (1H), 3.32 (1H), 3.79 (1H), 4.13 (1H), 4.30(1H), 5.66 (1H), 6.25 (1H), 7.39 (1H) ppm.
¹H-NMR (CDCl₃) von B, δ= 0.86 (3H), 0.96 (3H), 1.10 (3H), 1.15-1.93 (7H), 1.23 (3H), 1.35 (3H), 1.95-2.38 (4H), 2.58 (2H), 2.70 (3H), 2.99 (1H), 3.01 (1H), 3.27 (1H), 3.65-3.75 (2H), 4.24 (1H), 5.62 (1H), 6.21 (1H), 7.35 (1H) ppm.

### Beispiel 26

### (4S,7R,8S,9S,13(Z),16S(Z))-4,8-Dihydroxy-7-ethyl-16-(1-chlor-2-(2-methyl-4-thiazolyl)ethenyl)-1-oxa-5,5,9,13-tetramethyl-cyclohexadec-13-en-2,6-dion

In Analogie zu Beispiel 5 unter Verwendung von (3S)-1,3-Bis[[dimethyl(1,1-dimethyl)silyl]oxy]-4,4-dimethyl-octan-5-on als Aldolkomponente (siehe Beispiel 5n) erhält man die Titelverbindung.

### Beispiel 27

### (4S,7R,8S,9S,13(E),16S(Z))-4,8-Dihydroxy-7-ethyl-16-(1-chlor-2-(2-methyl-4-thiazolyl)ethenyl)-1-oxa-5,5,9,13-tetramethyl-cyclohexadec-13-en-2,6-dion

In Analogie zu Beispiel 6 unter Verwendung von (3S)-1,3-Bis[[dimethyl(1,1-dimethyl)silyl]oxy]-4,4-dimethyl-octan-5-on als Aldolkomponente (siehe Beispiel 5n) erhält man die Titelverbindung.

### Beispiel 28

### (1S,3S(Z),7S,10R,11S,12S,16SR)-7,11-Dihydroxy-10-ethyl-3-(1-chlor-2-(2-methyl-4-thiazolyl)ethenyl)-8,8,12,16-tetramethyl-4,17-dioxabicyclo[14.1.0]heptadecan-5,9-dion (A) und

### (1R,3S(Z),7S,10R,11S,12S,16S)-7,11-Dihydroxy-10-ethyl-3-(1-chlor-2-(2-methyl-4-thiazolyl)ethenyl)-8,8,12,16-tetramethyl-4,17-dioxabicyclo[14.1.0]heptadecan-5,9-dion (B)

In Analogie zu Beispiel 3 erhält man aus der in Beispiel 26 hergestellten Titelverbindung die Titelverbindung.

### Beispiel 29

### (1S,3S(Z),7S,10R,11S,12S,16S)-7,11-Dihydroxy-10-ethyl-3-(1-chlor-2-(2-methyl-4-thiazolyl)ethenyl)-8,8,12,16-tetramethyl-4,17-dioxabicyclo[14.1.0]heptadecan-5,9-dion (A) und

(1 R,3S(Z),7S,10R,11 S,12S,16R)-7,11-Dihydroxy-10-ethyl-3-(1-chlor-2-(2-methyl-4-thiazolyl)ethenyl)-8,8,12,16-tetramethyl-4,17-dioxabicyclo[14.1.0]heptadecan-5,9-dion (B)

In Analogie zu Beispiel 3 erhält man aus der in Beispiel 27 hergestellten Titelverbindung die Titelverbindung.

### Beispiel 30

### (1S,3S(Z),7S,10R,11S,12S,16S)-7,11-Dihydroxy-10-ethyl-3-(1-fluor-2-(2-pyridyl)ethenyl)-8,8,12,16-tetramethyl-4,17-dioxabicyclo[14.1.0]heptadecan-5,9-dion (A) und (1R,3S(Z),7S,10R,11S,12S,16R)-7,11-Dihydroxy-10-ethyl-3-(1-fluor-2-(2-pyridyl)ethenyl)-8,8,12,16-tetr-amethyl-4,17-dioxabicyclo[14.1.0]heptadecan-5,9-dion (B)

In Analogie zu Beispiel 20 setzt man 150 mg (max. 229 µmol) der nach Beispiel 21 dargestellten Verbindungen um und isoliert nach Aufarbeitung und Reinigung 19 mg (38 µmol, 16%) der Titelverbindung A sowie 35 mg (69 µmol, 30% der Titelverbindung B jeweils als farbloses Öl.
¹H-NMR (CDCl₃) von A: δ = 0,83 (3H), 0,93 (3H), 1,08 (3H), 1,18-1,97 (9H), 1,21 (3H), 1,36 (3H), 2,09 (1H), 2,31 (1H), 2,59 (2H), 2,99 (1H). 3,30 (1H). 3,44 (1H). 3,70 (1H). 4,33 (1H), 4,40 (1H), 5,67 (1H), 6,19 (1H), 7,18 (1H), 7,70 (2H), 3,51 (1H) ppm.
¹H-NMR (CDCl₃) von B: δ = 0,85 (3H), 0,94 (3H), 1,00-1,97 (9H), 1,02 (3H), 1,29 (3H), 1,38 (3H), 2,06 (1H), 2,28 (1H). 2,54 (2H), 2,90 (1H). 3,35 (1H). 3,61 (1H), 3,79 (1 H), 4,39 (2H), 5,67 (1H). 6,22 (1H), 7,18 (1H). 7,69 (1H), 7,78 (1H), 8,51 (1H) ppm.

### Beispiel 31

### (4S,7R,8S,9S,13Z,16S(Z))-4,8-Dihydroxy-16-(1-chlor-2-(2-pyridyl)ethenyl)-7-ethyl-1-oxa-5,5,9,13-tetramethyl-cyclohexadec-13-en-2,6-dion

### Beispiel 31a

### 3-(2-Pyridyl)- 2-propin-1-ol

Das Gemisch aus 16,6 ml (173 mmol) 2-Brompyridin, 21,6 ml Propargylalkohol, 2,5 g Palladium-bis-triphenylphoshin-dichlorid und 173 mg Kupfer(I)iodid versetzt man mit 510 ml Diethylamin und erhitzt 1,5 Stunden auf 80°C. Nach Filtration und Lösungsmittelabzug reinigt man den Rückstand durch Chromatographie an feinem Kieselgel mit einem Gradientensystem aus n-Hexan und Ethylacetat. Isoliert werden 17,8 g (134 mmol, 77%) der Titelverbindung als farbloses Öl.
¹H-NMR (CDCl₃): δ = 3,70 (1H), 4,54 (2H), 7,24 (1H), 7,42 (1H), 7,67 (1H), 8,53 (1H) ppm.

### Beispiel 31b

### (2Z)-3-(2-Pyridyl)-2-chlor-2-propen-1-ol

12,3 g (92,6 mmol) der nach Beispiel 31a dargestellten Verbindung versetzt man mit 238 ml konz. Salzsäure und erhitzt 2,5 Stunden auf 80°C. Nach dem Erkalten gießt man vorsichtig in gesättigte Kaliumcarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 14,8 g (87,3 mmol, 94%) der Titelverbindung als kristalliner Feststoff.
¹H-NMR (CDCl₃): δ = 4,36 (2H), 5,47 (1H), 7,18 (1H), 7,21 (1H), 7,72 (1H), 7,99 (1H), 8,56 (1H) ppm.

### Beispiel 31c

### (2Z)-3-(2-Pyridyl)-2-chlor-2-propenal

In Analogie zu Beispiel 17e setzt man 14,8g (87,5 mmol) der nach Beispiel 31b dargestellten Verbindung um und isoliert nach Aufarbeitung 14,6g (87,1 mmol, 99%) der Titelverbindung als blass gelbes Öl.
¹H-NMR (CDCl₃): δ = 7,36 (1H), 7,74 (1H), 7,83 (1 H), 8,34 (1 H), 8,77 (1 H), 9,57 (1 H) ppm.

### Beispiel 31d

### (3S,4Z)-5-(2-Pyridyl)-1-[(4S,5R)-4-methyl-5-phenyl-1,3-oxazolidin-2-on-3-yl]-3-hydroxy-4-chlor-4-penten-1-on (A) und (3R,4Z)-5-(2-Pyridyl)-1-[(4S,5R)-4-methyl-5-phenyl-1,3-oxazolidin-2-on-3-yl]-3-hydroxy-4-chlor-4-penten-1-on (B)

In Analogie zu Beispiel 17f setzt man 14,6g (87,1 mmol) der nach Beispiel 31c dargestellten Verbindung um und isoliert nach Aufarbeitung und Trennung 12,3g (31,8 mmol, 37%) der kristallinen Titelverbindung A sowie 9,6g (24,8 mmol, 28%) der Titelverbindung B als farbloses Öl.
¹H-NMR (CDCl₃) von A: δ = 0,94 (3H), 3,42 (1H), 3,58 (1 H), 4,50 (1H), 4,81 (1H), 4,91 (1H), 5,70 (1H), 7,14-7,48 (7H), 7,72 (1H), 7,96 (1H), 8,62 (1H) ppm.
¹H-NMR (CDCl₃) von B: δ = 0,96 (3H), 3,50 (2H), 4,82 (1H), 4,96 (1H), 5,72 (1H), 7,13-7,50 (7H), 7,73 (1H), 7,97 (1H), 8,65 (1H) ppm.

### Beispiel 31e

### (4Z)-5-(2-Pyridyl)-1-[(4S,5R)-4-methyl-5-phenyl-1,3-oxazolidin-2-on-3-yl]-4-chlor-4-penten-1,3-dion

In Analogie zu Beispiel 17g setzt man 11,3g (29,2 mmol) der nach Beispiel 31d dargestellten Verbindung B um und isoliert nach Aufarbeitung 9,8g (25,5 mmol, 87%) der Titelverbindung als kristallinen Feststoff.
¹H-NMR (CDCl₃) als Keton/Enol-Gemisch:δ = 0,99 (3H), 4,49 (0,6H), 4,60 (0,6H), 4,87 (1H), 5,71+5,76 (1H), 7,21-7,52 (6,4H), 7,79 (1H), 7,92 (1H), 8,10+8,20 (1H), 8,72 (1H), 13,66 (0,4H) ppm.

### Beispiel 31f

### (3S,4Z)-5-(2-Pyridyl)-1-[(4S,5R)-4-methyl-5-phenyl-1,3-oxazolidin-2-on-3-yl]-3-[[dimethyl(1,1-dimethylethyl)silyl]oxy]-4-chlor-4-penten-1-on

In Analogie zu Beispiel 17i setzt man 12,3g (31,7 mmol) der nach Beispiel 31d und/oder Beispiel 31f dargestellten Verbindung A um und isoliert nach Aufarbeitung und Reinigung 12,2g (24,3 mmol, 77%) der Titelverbindung als farbloses Öl.
¹H-NMR (CDCl₃): δ = 0,13 (6H), 0,90 (12H), 3,30 (1H), 3,59 (1H), 4,78 (1H), 5,01 (1H), 5,66 (1H), 7,02 (1H), 7,19 (1H), 7,23-7,48 (5H), 7,71 (1H), 7,97 (1H), 8,62 (1H) ppm.

### Beispiel 31g

### (3S,4Z)-5-(2-Pyridyl)-3-[[dimethyl(1,1-dimethylethyl)silyl]oxy]-4-chlor-4-pentensäureethylester

In Analogie zu Beispiel 17j setzt man 12,1g (24,1 mmol) der nach Beispiel 31f dargestellten Verbindung um und isoliert nach Aufarbeitung und Reinigung 8,3g (22,4 mmol, 93%) der Titelverbindung als farbloses Öl.
¹H-NMR (CDCl₃): δ = 0,11 (6H), 0,90 (9H), 1,26 (3H), 2,75 (2H), 4,14 (2H), 4,83 (1H), 7,00 (1H), 7,18 (1H), 7,69 (1H), 7,91 (1H), 8,61 (1H) ppm.

### Beispiel 31h

### (3S,4Z)-5-(2-Pyridyl)-3-[[dimethyl(1,1-dimethylethyl)silyl]oxy]-4-chlor-4-penten-1-ol

In Analogie zu Beispiel 17k setzt man 8,1g (21,9 mmol) der nach Beispiel 31g dargestellten Verbindung um und isoliert nach Aufarbeitung und Reinigung 6,3g (19,2 mmol, 88%) der Titelverbindung als farbloses Öl.
¹H-NMR (CDCl₃): δ = 0,13 (3H), 0,18 (3H), 0,96 (9H), 1,98-2,10 (3H), 3,70-3,91 (2H), 4,60 (1H), 7,00 (1H), 7,19 (1H), 7,70 (1H), 7,93 (1H), 8,62 (1H) ppm.

### Beispiel 31i

### (3S,4Z)-5-(2-Pyridyl)-3-[[dimethyl(1,1-dimethylethyl)silyl]oxy]-1-iod-4-chlor-4-penten

In Analogie zu Beispiel 171 setzt man 6,3g (19,2 mmol) der nach Beispiel 31h dargestellten Verbindung um und isoliert nach Aufarbeitung und Reinigung 7,8g (17,8 mmol, 93%) der Titelverbindung als farbloses Öl.
¹H-NMR (CDCl₃): δ = 0,12 (3H), 0,19 (3H), 0,93 (9H), 2,25 (2H), 3,24 (2H), 4,48 (1H), 7,00 (1H), 7,20 (1H), 7,71 (1H), 7,97 (1H), 8,63 (1H) ppm.

### Beispiel 31k

### (3S,4Z)-5-(2-Pyridyl)-3-[[dimethyl(1,1-dimethylethyl)silyl]oxy]-4-chlor-4-penten-1-triphenylphosphoniumiodid

In Analogie zu Beispiel 17m setzt man 7,8g (17,8 mmol) der nach Beispiel 31i dargestellten Verbindung um und isoliert nach Aufarbeitung und Reinigung 11,4g (16,3 mmol, 91%) der Titelverbindung als kristallinen Feststoff.
¹H-NMR (CDCl₃): δ = 0,15 (3H), 0,21 (3H), 0,90 (9H), 1,96-2,20 (2H), 3,52-3,91 (2H), 5,02 (1H), 7,15 (1H), 7,25 (1H), 7,63-7,88 (17H), 8,61 (1H) ppm.

### Beispiel 31l

### (2S,6E/Z,9S,10Z)-9-[[Dimethyl(1,1-dimethylethyl)silyl]oxy]-10-chlor-11-(2-pyridyl)-1-(tetrahydropyran-2-yloxy)-2,6-dimethyl-undeca-6,10-dien

In Analogie zu Beispiel 17n setzt man 3,00 g der nach Beispiel 31 k dargestellten Verbindung mit 653 mg (2,86 mmol) (2S)-2-Methyl-6-oxo-heptan-1-(tetrahydropyran-2-yloxy), das man in Analogie zu den in DE 197 51 200.3 bzw. WO 99/07692 beschriebenen Verfahren hergestellt hat, um und isoliert nach Aufarbeitung und Reinigung neben ausgangsmaterial 202 mg (0,39 mmol, 14%) der Titelverbindung als farbloses Öl.
¹H-NMR (CDCl₃): δ = 0,08 (6H), 0,80-0,96 (12H), 1,08 (1H), 1,22-2,05 (12H), 1,61+1,67 (3H), 2,31-2,55 (2H), 3,03-3,25 (1H), 3,40-3,62 (2H), 3,84 (1H), 4,28 (1H), 4,53 (1H), 5,15 (1H), 6,91 (1H), 7,16 (1H), 7,68 (1H), 7,95 (1H), 8,60 (1H) ppm.

### Beispiel 31m

### (2S,6E/Z,9S,10Z)-9-[[Dimethyl(1,1-dimethylethyl)silyl]oxy]-1 0-chlor-11-(2-pyridyl)-1-hydroxy-2,6-dimethyl-undeca-6,10-dien

In Analogie zu Beispiel 17o setzt man 472 mg (904 µmol) der nach Beispiel 311 dargestellten Verbindung um und isoliert nach Aufarbeitung und Reinigung 278 mg (635 µmol, 70%) der Titelverbindung als farbloses Öl.
¹H-NMR (CDCl₃): δ = 0,09 (6H), 0,82-0,97 (12H), 0,98-2,12 (8H), 1,60+1,68 (3H), 2,32-2,58 (2H), 3,36-3,54 (2H), 4,30 (1H), 5,11+5,19 (1H), 6,89+6,92 (1H), 7,19 (1H), 7,70 (1H), 7,98+8,04 (1H), 8,59 (1H) ppm.

### Beispiel 31n

### (2S,6E/Z,9S,10Z)-9-[[Dimethyl(1,1-dimethylethyl)silyl]oxy]-10-chlor-11-(2-pyridyl)-2,6-dimethyl-undeca-6,10-dienal

In Analogie zu Beispiel 17p setzt man 278 mg (635 µmol) der nach Beispiel 31m dargestellten Verbindung um und isoliert nach Aufarbeitung 273 mg (626 µmol, 99%) der Titelverbindung als blass gelbesÖl.

### Beispiel 31o

### (4S(4R,5S,6S,10E/Z,13S,14Z))-4-(13-[[(1,1-Dimethylethyl)dimethylsilyl]oxy]-4-ethyl-14-chlor-15-(2-pyridyl)-3-oxo-5-hydroxy-2,6,10-trimethyl-pentadeca-10,14-dien-2-yl)-2,2-dimethyl-[1,3]dioxan (A) und (4S(4S,5R,6S,10E/Z,13S,14Z))-4-(13-[[(1,1-Dimethylethyl)dimethylsilyl]oxy]-4-ethyl-14-chlor-15-(2-pyridyl)-3-oxo-5-hydroxy-2,6,10-trimethyl-pentadeca-10,14-dien-2-yl)-2,2-dimethyl-[1,3]dioxan (B)

In Analogie zu Beispiel 17q setzt man 273 mg (626 µmol) der nach Beispiel 31n dargestellten Verbindung mit (4S)-4-(2-Methyl-3-oxo-hex-2-yl)-2,2-dimethyl-[1,3]dioxan, das man nach dem in DE 19751200.3 bzw. WO 99/07692 beschriebenen Verfahren hergestellt hat, um und isoliert nach Aufarbeitung und Reinigung 275 mg (414 µmol, 66%) der Titelverbindung A sowie Reinigung 58 mg (87 µmol, 14%) der Titelverbindung B jeweils als farbloses Öl.
¹H-NMR (CDCl₃) von A: δ = 0,07 (6H), 0,82 (6H), 0,91 (9H), 0,99 (3H), 1,08 (1H), 1,17-2,08 (9H), 1,23 (3H), 1,31 (3H), 1,39 (3H), 1,60+1,68 (3H), 2,31-2,56 (2H), 2,89 (1H), 3,19 (1H), 3,43 (1H), 3,88 (1H), 3,98 (1H), 4,18 (1H), 4,29 (1H), 5,16 (1H), 6,91 (1H), 7,18 (1H), 7,69 (1H), 7,97 (1H), 8,60 (1H) ppm.

### Beispiel 31p

### (3S.6R.7S.8S, 12E/Z,15S,16Z)-15-[[(1,1-Dimethylethyl)dimethylsilyl]oxy]-6-ethyl-16-chlor-1,3,7-trihydroxy-4,4,8,12-tetramethyl-17-(2-pyridyl)-heptadeca-12,16-dien-5-on

In Analogie zu Beispiel 17r setzt man 275 mg (414 mmol) der nach Beispiel 31o dargestellten Verbindung A um und isoliert nach Aufarbeitung und Reinigung 234 mg (375 µmol, 91%) der Titelverbindung als farbloses Öl.
¹H-NMR (CDCl₃): δ = 0,03-0,12 (6H), 0,78-0,95 (15H), 0,98-2,18 (10H), 1,09 (3H), 1,26 (3H), 1,60+1,68 (3H), 2,32-2,58 (2H), 2,72-2,98 (2H), 3,19 (1H), 3,41 (1H), 3,71-4,00 (3H), 4,12 (1H), 4,28 (1H), 5,11+5,21 (1H), 6,82+6,83 (1H), 7,20 (1H), 7,71 (1H), 8,03 (1H), 8,63 (1H) ppm.

### Beispiel 31q

### (3S,6R,7S,8S,12E/Z,15S,16Z)-6-Ethyl-1,3,7,15-tetrakis-[[(1,1-dimethylethyl)dimethylsilyl]oxy]-16-chlor-4,4,8,12-tetramethyl-17-(2-pyridyl)-heptadeca-12,16-dien-5-on

In Analogie zu Beispiel 17s setzt man 234 mg (375 µmol) der nach Beispiel 31p dargestellten Verbindung um und isoliert nach Aufarbeitung und Reinigung 325 mg (336 µmol, 90%) der Titelverbindung als farbloses Öl.
¹H-NMR (CDCl₃): δ = -0,04-0,10 (24H), 0,76-1,78 (51H), 1,01 (3H), 1,23 (3H), 1,59+1,63 (3H), 1,89-2,03 (2H), 2,29-2,54 (2H), 3,00 (1H), 3,50-3,71 (2H), 3,80 (1H), 3,(1H), 4,26 (1 H), 5,13 (1 H), 6,91 (1H), 7,15 (1 H), 7,68 (1H), 7,94 (1H), 8,60 (1 H) ppm.

### Beispiel 31r

### (3S,6R,7S,8S,12E/Z,15S,16Z)-6-Ethyl-3,7,15-tris-[[(1,1-dimethylethyl)dimethylsilyl]oxy]-1-hydroxy-16-chlor-4,4,8,12-tetramethyl-17-(2-pyridyl)-heptadeca-1 2,16-dien-5-on

In Analogie zu Beispiel 17t setzt man 325 mg (336 µmol) der nach Beispiel 31q dargestellten Verbindung um und isoliert nach Aufarbeitung und Reinigung 264 mg (310 µmol, 92%) der Titelverbindung als farbloses Öl.
¹H-NMR (CDCl₃): δ = 0,01-0,12 (18H), 0,79-0,97 (33H), 1,01-2,08 (12H), 1,08 (3H), 1,19 (3H), 1,60+1,68 (3H), 2,31-2,56 (2H), 3,01 (1H), 3,68 (2H), 3,84 (1H), 4,08 (1H), 4,29 (1H), 5,18 (1H), 6,93 (1H), 7,19 (1H), 7,69 (1H), 7,97 (1H), 8,61 (1H) ppm.

### Beispiel 31s

### (3S,6R,7S,8S,12E/Z, 15S, 16Z)-6-Ethyl-3,7,15-tris-[[(1,1-dimethylethyl)dimethylsilyl]oxy]-16-chlor-5-oxo-4,4,8,12-tetramethyl-17-(2-pyridyl)-heptadeca-12,16-dienal

In Analogie zu Beispiel 17u setzt man 264 mg (310 µmol) der nach Beispiel 31r dargestellten Verbindung um und isoliert nach Aufarbeitung 238 mg (280 µmol, 90%) der Titelverbindung als blass gelbes Öl, das man ohne Reinigung weiter umsetzt.

### Beispiel 31t

### (3S,6R,7S,8S,12Z,15S,16Z)-6-Ethyl-3,7,15-tris-[[(1,1-dimethylethyl)dimethylsilyl]oxy]-16-chlor-5-oxo-4,4,8,12-tetramethyl-17-(2-pyridyl)-heptadeca-12,16-diensäure (A) und (3S,6R,7S,8S,12E,15S,16Z)-6-Ethyl-3,7,15-tris-[[(1,1-dimethylethyl)dimethylsilyl]oxy]-16-chlor-5-oxo-4,4,8,12-tetramethyl-17-(2-pyridyl)-heptadeca-12,16-diensäure (B)

In Analogie zu Beispiel 17v setzt man 238 mg (280 µmol) der nach Beispiel 31s dargestellten Verbindung um und isoliert nach Aufarbeitung und Reinigung 111 mg (128 µmol, 46%) der Titelverbindung A sowie 102 mg (118 µmol, 42%) der Titelverbindung B jeweils als farbloses Öl.
¹H-NMR (CDCl₃) von A: δ = -0,01-0,15 (18H), 0,79-0,97 (33H), 1,02-2,43 (13H), 1,12 (3H), 1,21 (3H), 1,71 (3H), 2,56 (1H), 3,01 (1H), 3,77 (1H), 4,31 (1H), 4,39 (1H), 5,19 (1 H), 7,16 (1H), 7,24 (1H), 7,76 (1H), 8,09 (1H), 8,59 (1 H) ppm.
¹H-NMR (CDCl₃) von 3: δ = 0,00-0,19 (18H), 0,78-0,97 (33H), 1,00-1,73 (8H), 1.11 (3H), 1,21 (3H), 1,58 (3H), 1,87 (1H), 2,00 (1H), 2,29-2,43 (2H), 2,53 (1H), 2,63 (1H), 3,09 (1H), 3,87 (1H), 4,32 (2H), 5,13 (1H), 6,93 (1H), 7,26 (1H). 7,78 (1H), 8,12 (1H), 8,61 (1H) ppm.

### Beispiel 31u

### (3S,6R,7S,8S,12Z,15S,16Z)-6-Ethyl-3,7-bis-[[(1,1-dimethylethyl)dimethylsilyl]oxy]-16-chlor-15-hydroxy-5-oxo-4,4,8,12-tetramethyl-17-(2-pyridyl)heptadeca-12,16-diensäure

In Analogie zu Beispiel 17w setzt man 111 mg (128 µmol) der nach Beispiel 31t dargestellten Verbindung A um und isoliert nach Aufarbeitung 105 mg (max. 128 µmol) der Titelverbindung als als Rohprodukt, das man ohne Reinigung weiter umsetzt.

### Beispiel 31v

### (4S,7R,8S,9S,13Z,16S(Z))-4,8-Bis-[[dimethyl(1,1-dimethylethyl)silyl]oxy]-16-(1-chtor-2-(2-pyridyl)ethenyl)-7-ethyl-1-oxa-5,5,9,13-tetramethyl-cyclohexadec-13-en-2,6-dion

In Analogie zu Beispiel 17x setzt man 105 mg (max. 128 µmol) der nach Beispiel 31 u dargestellten Verbindung um und isoliert nach Aufarbeitung und Reinigung 61 mg (83 µmol, 65%) der Titelverbindung als farbloses Öl.
¹H-NMR (CDCl₃): δ = -0,11 (3H), 0,08 (3H), 0,11 (6H), 0,69-1,98 (19H), 0,73 (3H), 0,84 (9H), 0,94 (3H), 1,22 (3H), 1,68 (3H), 2,29 (1H), 2,45 (1H), 2,65 (1H), 2,84 (1H), 3,02 (1H), 3,99 (2H), 5,14 (2H), 6,98 (1H), 7,19 (1H), 7,69 (1H), 7,98 (1H), 8,61 (1H) ppm.

### Beispiel 31

### (4S,7R,8S,9S,13Z,16S(Z))-4,8-Dihydroxy-16-(1-chlor-2-(2-pyridyl)ethenyl)-7-ethyl-1-oxa-5,5,9,13-tetramethyl-cyclohexadec-13-en-2,6-dion

In Analogie zu Beispiel 17 setzt man 61 mg (83 µmol) der nach Beispiel 31v dargestellten Verbindung um und isoliert nach Aufarbeitung und Reinigung 24 mg (47 µmol, 57%) der Titelverbindung als farbloses Öl.
¹H-NMR (CDCl₃): δ = 0,88 (3H), 1,03 (3H), 1,09 (3H), 1,20-1,92 (7H), 1,36 (3H), 1,86 (3H), 2,24-2,62 (5H), 2,82 (1H), 3,22 (1H), 3,49 (1H), 3,70 (1H), 4,06 (1H), 4,32 (1H), 5,12 (1 H), 5,41 (1 H), 7,00 (1 H), 7,22 (1H), 7,72 (1 H), 7,91 (1 H), 8,57 (1 H) ppm.

### Beispiel 32

### (4S,7R,8S,9S,13E,16S(Z))-4,8-Dihydroxy-16-(1-chlor-2-(2-pyridyl)ethenyl)-7-ethyl-1-oxa-5,5,9,13-tetramethyl-cyctohexadec-13-en-2,6-dion

### Beispiel 32a

### (3S,6R,7S,8S,12E,15S,16Z)-6-Ethyl-3,7-bis-[[(1,1-dimethylethyl)dimethylsilyl]oxy]-16-chlor-15-hydroxy-5-oxo-4,4,8,12-tetramethyl-17-(2-pyridyl)-heptadeca-12,16-diensäure

In Analogie zu Beispiel 17w setzt man 102 mg (118 µmol) der nach Beispiel 31t dargestellten Verbindung B um und isoliert nach Aufarbeitung 92 mg (max. 118 µmol) der Titelverbindung als als Rohprodukt, das man ohne Reinigung weiter umsetzt.

### Beispiel 32b

### (4S,7R,8S,9S,13E,16S(Z))-4,8-Bis-[[dimethyl(1,1-dimethylethyl)silyl]oxy]-16-(1-chlor-2-(2-pyridyl)ethenyl)-7-ethyl-1-oxa-5,5,9,13-tetramethyl-cyclohexadec-13-en-2,6-dion

In Analogie zu Beispiel 17x setzt man 92 mg (max. 118 µmol) der nach Beispiel 32a dargestellten Verbindung um und isoliert nach Aufarbeitung und Reinigung 62 mg (84 µmol, 72%) der Titelverbindung als farbloses Öl.
¹H-NMR (CDCl₃): δ = 0,04-0,19 (12H), 0,78-2,00 (14H), 0,58 (9H), 0,90 (9H), 1,11 (3H), 1,22 (3H), 1,62 (3H), 2,16 (1H), 2,41 (1H), 2,52-2,81 (3H), 2,91 (1H), 3,91 (1H), 4,36 (1H), 5,23 (1H), 5,47 (1H), 6,98 (1H), 7,18 (1H), 7,69 (1H), 7,89 (1H), 8,61 (1H) ppm.

### Beispiel 32

### (4S,7R,8S,9S,13E,16S(Z))-4,8-Dihydroxy-16-(1-chlor-2-(2-pyridyl)ethenyl)-7-ethyl-1-oxa-5,5,9,13-tetramethyl-cyclohexadec-13-en-2,6-dion

In Analogie zu Beispiel 17 setzt man 62 mg (84 µmol) der nach Beispiel 32b dargestellten Verbindung um und isoliert nach Aufarbeitung und Reinigung 17 mg (34 µmol, 40%) der Titelverbindung als farbloses Öl.
¹H-NMR (COCl₃): δ = 0,76 (1H), 0,83 (3H), 0,99 (3H), 1,02 (3H), 1,28 (3H), 1,37-2,00 (8H), 1,61 (3H), 2,21 (1H), 2,42 (1H), 2,51 (1H), 2,61 (2H), 3,40 (1H), 3,76 (1H), 4,55 (1H), 5,04 (1H), 5,10 (1H), 5,51 (1H), 6,96 (1H), 7,21 (1H), 7,73 (1H), 8,17 (1H), 8,49 (1H) ppm.

### Beispiel 33

### (1RS,3S(Z),7S,10R,11S,12S,16RS)-7,11-Dihydroxy-10-ethyl-3-(1-fluor-2-(2-pyridyl)ethenyl)-8,8,12,16-tetramethyl-4,17-dioxabicyclo[14.1.0]heptadecan-5,9-dion

In Analogie zu Beispiel 19 setzt man 17 mg (34 µmol) der nach Beispiel 32 dargestellten Verbindung um und isoliert nach Aufarbeitung 23 mg (max. 34 µmol) der Titelverbindungen als farbloses Öl.

### Beispiel 34

### (1S,3S(Z),7S,10R,11S,12S,16S)-7,11-Dihydroxy-10-ethyl-3-(1-fluor-2-(2-pyridyl)ethenyl)-8,8,12,16-tetramethyl-4,17-dioxabicyclo[14.1.0]heptadecan-5,9-dion (A) und (1R,3S(Z),7S,10R,11S,12S,16R)-7,11-Dihydroxy-10-ethyl-3-(1-fluor-2-(2-pyridyl)ethenyl)-8,8,12,16-tetramethyl-4,17-dioxabicyclo[14.1.0]heptadecan-5,9-dion (B)

In Analogie zu Beispiel 17 setzt man 23 mg (max. 34 µmol) der nach Beispiel 32 dargestellten Verbindungen um und isoliert nach Aufarbeitung und Reinigung 3,6 mg (6,9 µmol, 20,3 %) der Titelverbindung A sowie 4,9 mg (9,4 µmol, 27,7 %) der Titelverbindung B jeweils als farbloses Öl.
¹H-NMR (CDCl₃) von A: δ = 0,85 (3H), 0,95 (3H), 1,08 (3H), 1,24 (3H), 1,37 (3H), 1,72-1,95 (3H), 2,24 (2H), 2,50-2,64 (2H), 2,98 (1H), 3,23 (1H), 3,30 (1H), 3,69 (1H), 4,07 (1H), 4,34 (1H), 5,64 (1H), 7,07 (1H), 7,23 (1H), 7,73 (1H), 7,97 (1H), 8,58 (1H) ppm.
¹H-NMR (CDCl₃) von B: δ = 0,87 (3H), 0,97 (3H), 1,04 (3H), 1,29 (3H), 1,37 (3H), 1,75-2,09 (6H), 2,40 (1H), 2,54 (2H), 2,87 (1H), 3,38 (1H), 3,80 (1H), 4,20 (1H), 4,47 (1H), 5,61 (1H), 7,11 (1H), 7,23 (1H), 7,76 (1H), 8,10 (1H), 8,57 (1H) ppm.

## Patentansprüche

1. Epothilon-Derivate der allgemeinen Formel I 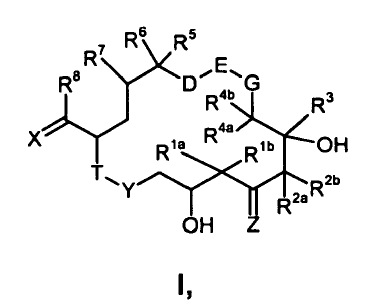 worin
R^{1a}, R^{1b} gleich oder verschieden sind und Wasserstoff, C₁-C₁₀-Alkyl, Aryl, C₇-C₂₀-Aralkyl, oder gemeinsam eine -(CH₂)ₘ-Gruppe mit m = 2, 3, 4 oder 5,
R^{2a}, R^{2b} gleich oder verschieden sind und Wasserstoff, C₁-C₁₀-Alkyl, Aryl, C₇-C₂₀-Aralkyl oder gemeinsam eine -(CH₂)ₙ-Gruppe mit n = 2, 3, 4 oder 5
R³ Wasserstoff, C₁-C₁₀-Alkyl, Aryl, C₇-C₂₀-Aralkyl,
G ein Sauerstoffatom oder eine Gruppe -CH₂,
R^{4a}, R^{4b} gleich oder verschieden sind und Wasserstoff, C₁-C₁₀-Alkyl, Aryl,C₇-C₂₀-Aralkyl oder gemeinsam eine -(CH₂)ₚ-Gruppe mit p = 2, 3, 4 oder 5,
D-E eine Gruppe H₂C-CH₂ HC=CH, C≡C 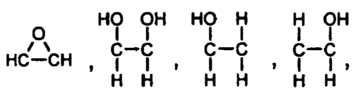 _{-H2C}-₀-, -O-C^{H}₂-
R⁵ Wasserstoff, C₁-C₁₀-Alkyl, Aryl, C₇-C₂₀-Aralkyl, CO₂H, CO₂-Alkyl, CH₂OH, CH₂O-Alkyl, CH₂O-Acyl, CN, CH₂NH₂, CH₂N(Alkyl, Acyl)_{1,2,} CH₂Hal
R⁶, R⁷ je ein Wasserstoffatom, gemeinsam eine zusätzliche Bindung oder ein Sauerstoffatom,
R⁸ ein Halogenatom oder eine Cyanogruppe,
X eine Gruppierung CR¹⁰R¹¹,
wobei einer der Reste
R¹⁰ für Wasserstoff, und der andere Rest
R¹¹ für einen gegebenenfalls substituierten Heteroarylrest stehen,
T-Y eine Gruppe O-C(=O), O-CH₂, CH₂C(=O), NR²⁴-C(=O), NR²4-SO₂,
R²⁴ Wasserstoff, C₁-C₁₀-Alkyl,
Z ein Sauerstoffatom oder H/OR¹²,
wobei
R¹² Wasserstoff oder eine Schutzgruppe PG^{z}
ist,
bedeuten.

2. Verbindungen nach Anspruch 1, worin R⁸ ein Fluoratom oder ein Chloratom ist.

3. Verbindungen nach Anspruch 1 oder 2, worin R^{2a} eine Methyl-, Ethyl- oder Propylgruppe bedeuten.

4. Verbindungen nach Anspruch 1 oder 2, worin R^{1a} und R^{1b} gemeinsam eine Trimethylengruppe bedeuten.

5. Verbindungen nach Anspruch 1 oder 2, worin R^{1a} und R^{1b} je eine Methylgruppe bedeuten.

6. Verbindungen nach Anspruch 1 oder 2, worin R¹⁰/R¹¹ für 2-Pyridylrest/Wasserstoff stehen.

7. Verbindungen nach Anspruch 1 oder 2, worin R¹⁰/R¹¹ für 2-Methyl-4-thiazolylrestlWasserstoff stehen.

8. Verbindungen nach Anspruch 1 oder 2, worin R¹⁰/R¹¹ für 2-Hydroxymethyl-4-thiazolylrest/Wasserstoff oder 2-Methyl-4-oxazolylrest/Wasserstoff oder 2-Hydroxymethyl-4-oxazolylrest/Wasserstoff.stehen.

9. Verbindungen nach Anspruch 1 oder 2, worin T-Y eine Gruppe O-C(=O) ist.

10. Verbindungen nach Anspruch 1 oder 2, worin T-Y eine Gruppe NR²⁴-C(=O) mit R²⁴ in der bereits angegebenen Bedeutung ist.

11. Verbindungen nach Anspruch 1 oder 2, worin G eine Methylengruppe ist.

12. Verbindungen nach Anspruch 1 oder 2, worin Z ein Sauerstoffatom ist.

13. Verbindungen nach Anspruch 1 oder 2, worin -D-E- für eine Ethylengruppe steht.

14. Verbindungen nach Anspruch 1 oder 2, worin R³ für ein Wasserstoffatom steht.

15. Verbindungen nach Anspruch 1 oder 2, worin R^{4a}/R^{4b} für H/CH₃ stehen.

16. Verbindungen nach Anspruch 2, worin R^{2a}/R^{2b} für Methyl oder Ethyl/Wasserstoff stehen.

17. Verbindungen nach Anspruch 4 oder 5, worin R¹⁰/R¹¹ für für 2-Pyridylrest/Wasserstoff oder 2-Methyl-4-thiazolylrest/Wasserstoff oder 2-Hydroxymethyl-4-thiazolylrest/Wasserstoff oder 2-Methyl-4-oxazolylrest/Wasserstoff oder 2-Hydroxymethyl-4-oxazolylrest/Wasserstoff stehen.

18. Verbindungen nach Anspruch 17, worin R^{2a}/R^{2b} für Methyl oder Ethyl/Wasserstoff stehen.

19. Verbindungen der allgemeinen Formel I, nämlich
(4S,7R,8S,9S,13(Z oder E),16S(Z))-4,8-Dihydroxy-16-(1-fluor-2-(2-methyl-4-thiazolyl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-fluor-2-(2-methyl-4-thiazolyl)ethenyl)-8,8,10,12,16-pentamethyl-4,17-dioxabicyclo[14.1.0]heptadecan-5,9-dion
(4S,7R,8S,9S,13(Z oder E), 16S(Z))-4,8-Dihydroxy-7-ethyl-16-(1-fluor-2-(2-methyl-4-thiazolyl)ethenyl)-1-oxa-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-methyl-4-thiazolyl)ethenyl)-8,8,12,16-tetramethyl-4,17-dioxabicyclo[14.1.0]heptadecan-5,9-dion
(4S,7R,8S,9S,13(Z oder E),16S(Z))-4,8-Dihydroxy-16-(1-fluor-2-(2-methyloxazol-4-yl)ethenyl)-1-oxa-5,5,7,9,13-pentamethyl-cyclohexadec-13-en-2,6-dion
(1 RS,3S(Z),7S,10R,11S,12S,16RS)-7,11-Dihydroxy-3-(1-fluor-2-(2-methyloxazol-4-yl)ethenyl)-8,8,10,12,16-pentamethyl-4,17-dioxabicyclo[14.1.0]heptadecan-5,9-dion
(4S,7R,8S,9S, 13(Z oder E), 16S(Z))-4,8-Dihydroxy-7-ethyl-16-(1-fluor-2-(2-methyloxazol-4-yl)ethenyl)-1-oxa-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-methyloxazol-4-yl)ethenyl)-8,8,12,16-tetramethyl-4,17-dioxabicyclo[14.1.0]heptadecan-5,9-dion
(4S,7R,8S,9S, 13(Z oder E), 16S(Z))-4,8-Dihydroxy-16-(1-fluor-2-(2-pyridyl)ethenyl)-1 - oxa-5,5,7,9,13-pentamethyl-cyclohexadec-13-en-2,6-dion
(1RS,3S(Z),7S, 10R,11S, 12S, 16RS)-7,11-Dihydroxy-3-(1-fluor-2-(2-pyridyl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7-ethyl-16-(1-fluor-2-(2-pyridyl)ethenyl)-1-oxa-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-pyridyl)ethenyl)-8,8,12,16-tetramethyl-4,17-dioxabicycio[14.1.0]heptadecan-5,9-dion
(4S,7R,8S,9S,13(Z oder E),16S(Z))-4,8-Dihydroxy-16-(1-fluor-2-(2-methyl-4-thiazolyl)ethenyl)-1-aza-5,5,7,9,13-pentamethyl-cyclohexadec-13-en-2,6-dion
(1RS,3S(Z),7S,10R,11S,12S,16RS)-7,11-Dihydroxy-3-(1-fluor-2-(2-methyl-4-thiazolyl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-fluor-2-(2-methyl-4-thiazolyl)ethenyl)-1-aza-5,5,9,13-tetramethyl-cyclohexadec-13-en-2,6-dion
(1RS,3S(Z),7S,10R,11S,12S,16RS)-7,11-Dihydroxy-10-ethyl-3-(1-fluor-2-(2-methy)-4-thiazolyl)ethenyl)-8,8,12,16-tetramethyl-4-aza-17-oxabicyclo[14.1.0]heptadecan-5,9-dion
(4S,7R,8S,9S,13(Z oder E), 16S(Z))-4,8-Dihydroxy-16-(1-fluor-2-(2-methyloxazol-4-yl)ethenyl)-1-aza-5,5,7,9,13-pentamethyl-cyclohexadec-13-en-2,6-dion
(1RS,3S(Z),7S,10R,11S,12S,16RS)-7,11-Dihydroxy-3-(1-fluor-2-(2-methyloxazol-4-yl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-fluor-2-(2-methyloxazol-4-yl)ethenyl)-1-aza-5,5,9,13-tetramethyl-cyclohexadec-13-en-2,6-dion
(1 RS,3S(Z),7S,10R,11S,12S,16RS)-7,11-Dihydroxy-10-ethyl-3-(1-fluor-2-(2-methyloxazol-4-yl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-fluor-2-(2-pyridyl)ethenyl)-1-aza-5,5,7,9,13-pentamethyl-cyclohexadec-13-en-2,6-dion
(1 RS,3S(Z),7S,10R,11S,12S,16RS)-7,11-Dihydroxy-3-(1-fluor-2-(2-pyridyl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-fluor-2-(2-pyridyl)ethenyl)-1-aza-5,5,9,13-tetramethyl-cyclohexadec-13-en-2,6-dion
(1 RS,3S(Z),7S, 10R,11 S,12S,16RS)-7,11-Dihydroxy-10-ethyl-3-(1-fluor-2-(2-pyridyl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-chlor-2-(2-methyl-4-thiazolyl)ethenyl)-1-oxa-5,5,7,9,13-pentamethyl-cyclohexadec-13-en-2,6-dion
(1 RS,3S(Z),7S, 10R,11 S,12S,16RS)-7,11-Dihydroxy-3-(1-chlor-2-(2-methyl-4-thiazolyl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7-ethyl-16-(1-chlor-2-(2-methyl-4-thiazolyl)ethenyl)-1-oxa-5,5,9,13-tetramethyl-cyclohexadec-13-en-2,6-dion
(1 RS,3S(Z),7S,1 10R, 11S, 12S, 16RS)-7,11-Dihydroxy-10-ethyl-3-(1-chlor-2-(2-methyl-4-thiazolyl)ethenyl)-8,8,12,16-tetramethyl-4,17-dioxabicyclo[14.1.0]heptadecan-5,9-dion
(4S,7R,8S,9S,13(Z oder E),16S(Z))-4,8-Dihydroxy-16-(1-chlor-2-(2-methyloxazol-4-yl)ethenyl)-1-oxa-5,5,7,9,13-pentamethyl-cyclohexadec-13-en-2,6-dion
(1 RS,3S(Z),7S,10R,11S,12S,16RS)-7,11-Dihydroxy-3-(1-chlor-2-(2-methyloxazol-4-yl)ethenyl)-8,8,10,12,16-pentamethyl-4,17-dioxabicyclo[14.1.0]heptadecan-5,9-dion
(4S,7R,8S,9S,13(Z oder E),16S(Z))-4,8-Dihydroxy-7-ethyl-16-(1-chlor-2-(2-methyloxazol-4-yl)ethenyl)-1-oxa-5, 5, 9,13-tetramethyl-cyclohexadec-13-en-2,6-dion
(1 RS,3S(Z),7S, 10R, 11S, 12S, 16RS)-7,11-Dihydroxy-10-ethyl-3-(1-chlor-2-(2-methyloxazol-4-yl)ethenyl)-8,8,12,16-tetramethyl-4,17-dioxabicyclo[14.1.0]heptadecan-5,9-dion
(4S,7R,8S,9S,13(Z oder E),16S(Z))-4,8-Dihydroxy-16-(1-chlor-2-(2-pyridyl)ethenyl)-1-oxa-5,5,7,9,13-pentamethyl-cyclohexadec-13-en-2,6-dion
(1RS,3S(Z),7S,10R,11S,12S,16RS)-7,11-Dihydroxy-3-(1-chlor-2-(2-pyridyl)ethenyl)-8,8,10,12,16-pentamethyl-4,17-dioxabicyclo[14.1.0]heptadecan-5,9-dion
(4S,7R,8S,9S, 13(Z oder E), 16S(Z))-4,8-Dihydroxy-7-ethyl-16-(1-chlor-2-(2-pyridyl)ethenyl)-1-oxa-5,5,9,13-tetramethyl-cyclohexadec-13-en-2, 6-dion
(1RS,3S(Z),7S,10R,11S,12S,16RS)-7,11-Dihydroxy-10-ethyl-3-(1-chlor-2-(2-pyridyl)ethenyl)-8,8,12,16-tetramethyl-4,17-dioxabicyclo[14.1.0]heptadecan-5,9-dion
(4S,7R,8S,9S,13(Z oder E),16S(Z))-4,8-Dihydroxy-16-(1-chlor-2-(2-methyl-4-thiazolyl)ethenyl)-1-aza-5,5,7,9,13-pentamethyl-cyclohexadec-13-en-2,6-dion
(1RS, 3S(Z),7S,10R,11 S,12S,16RS)-7,11-Dihydroxy-3-(1-chlor-2-(2-methyl-4-thiazolyl)ethenyl)-8,8, 1 0, 12, 16-pentamethyl-4-aza-17 -oxabicyclo[14. 1.0]heptadecan-5,9-dion
(4S,7R,8S,9S,13(Z oder E),16S(Z))-4,8-Dihydroxy-7-ethyl-16-(1-chlor-2-(2-methyl-4-thiazolyl)ethenyl)-1-aza-5,5,9,13-tetramethyl-cyclohexadec-13-en-2,6-dion
(1 RS,3S(Z),7S,10R,11 S,12S,16RS)-7,11-Dihydroxy-10-ethyl-3-(1-chlor-2-(2-methyl-4-thiazolyl)ethenyl)-8,8,12,16-tetramethyl-4-aza-17-oxabicyclo[14.1.0]heptadecan-5,9-dion
(4S,7R,8S,9S, 13(Z oder E), 16S(Z))-4,8-Dihydroxy-16-(1-chlor-2-(2-methyloxazol-4-yl)ethenyl)-1-aza-5,5,7,9,13-pentamethyl-cyclohexadec-13-en-2,6-dion
(1 RS,3S(Z),7S,10R,11S,12S,16RS)-7,11-Dihydroxy-3-(1-chlor-2-(2-methyloxazol-4-yl)ethenyl)-8,8,10,12,16-pentamethyl-4-aza-17-oxabicyclo[14.1.0]heptadecan-5,9-dion
(4S,7R,8S,9S,13(Z oder E),16S(Z))-4,8-Dihydroxy-7-ethyl-16-(1-chlor-2-(2-methyloxazol-4-yl)ethenyl)-1-aza-5,5,9,13-tetramethyl-cyclohexadec-13-en-2,6-dion
(1RS,3S(Z),7S,10R,11S,12S,16RS)-7,11-Dihydroxy-10-ethyl-3-(1-chlor-2-(2-methyloxazol-4-yl)ethenyl)-8, 8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-chlor-2-(2-pyridyl)ethenyl)-1-aza-5,5,7,9,13-pentamethyl-cyclohexadec-13-en-2,6-dion
(1 RS,3S(Z),7S,10R,11S,12S,16RS)-7,11-Dihydroxy-3-(1-chlor-2-(2-pyridyl)ethenyl)-8,8,10,12,16-pentamethyl-4-aza-17-oxabicyclo[14.1.O]heptadecan-5,9-dion
(4S,7R,8S,9S,13(Z oder E),16S(Z))-4,8-Dihydroxy-7-ethyl-16-(1-chlor-2-(2-pyridyl)ethenyl)-1 -aza-5,5,9,13-tetramethyl-cyclohexadec-1 3-en-2,6-dion
(1RS,3S(Z),7S,10R,11S,12S,16RS)-7,11-Dihydroxy-10-ethyl-3-(1-chlor-2-(2-pyridyl)ethenyl)-8,8,12,16-tetramethyl-4-aza-17-oxabicyclo[14.1.0]heptadecan-5,9-dion
(4S,7R,8S,9S,13(Z oder E),16S(Z))-4,8-Dihydroxy-16-(1-fluor-2-(2-methyl-4-thiazolyl)ethenyl)-1-oxa-7,9,13-trimethyl-5,5-(1,3-trimethylen)cyclohexadec-13-en-2,6-dion
(1 RS,3S(Z),7S,1 OR,11 S, 12S, 16RS)-7,1 1 -Dihydroxy-3-(1 -fluor-2-(2-methyl-4-thiazolyl)ethenyl)-10,12,16-trimethyl-8,8-(1,3-trimethylen)-4,17-dioxabicyclo[14.1.0]hepta-deca-5,9-dion
(4S,7R,8S,9S,13(Z oder E),16S(Z))-4,8-Dihydroxy-9,13-dimethyl-7-ethyl-16-(1-fluor-2-(2-methyl-4-thiazolyl)ethenyl)-1-oxa-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-fluor-2-(2-methyl-4-thiazolyl)ethenyl)-8,8-(1,3-trimethylen)-4,17-dioxabicyclo[14.1.0]heptadecan-5,9-dion
4S,7R,8S,9S,13(Z oder E),16S(Z))-4,8-Dihydroxy-16-(1-fluor-2-(2-methyl-4-thiazolyl)ethenyl)-1-aza-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-fluor-2-(2-methyl-4-thiazolyl)ethenyl)-10,12,16-trimethyl-8,8-(1,3-trimethylen)-4-aza-17-oxabicyclo[14.1.0]hepta-deca-5,9-dion
(4S,7R,8S,9S, 13(Z oder E),16S(Z))-4,8-Dihydroxy-9, 13-dimethyl-7 -ethyl-16-(1-fluor-2-(2-methyl-4-thiazolyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-fluor-2-(2-methyl-4-thiazolyl)ethenyl)-8,8-(1,3-trimethylen)-4-aza-17-oxabicyclo[14.1.0]heptadecan-5,9-dion
(4S,7R,8S,9S,13(Z oder E),16S(Z))-4,8-Dihydroxy-16-(1-chlor-2-(2-methyl-4-thiazolyl)ethenyl)-1-oxa-7,9,13-trimethyl-5,5-(1,3-trimethylen)cyclohexadec-13-en-2,6-dion
(1 RS,3S(Z),7S,10R,11S,12S,16RS)-7,11-Dihydroxy-3-(1-chlor-2-(2-methyl-4-thiazolyl)ethenyl)-10,12,16-trimethyl-8,8-(1,3-trimethylen)-4,17-dioxabicyclo[14.1.0]hepta-deca-5,9-dion
(4S,7R,8S,9S, 13(Z oder E), 16S(Z))-4,8-Dihydroxy-9,13-dimethyl-7-ethyl-16-(1-chlor-2-(2-methyl-4-thiazolyl)ethenyl)-1-oxa-5,5-(1,3-trimethylen)cyclohexadec-13-en-2,6-dion
(1RS,3S(Z),7S,10R,11S, 12S,16RS)-7,11-Dihydroxy-12,16-dimethyl-10-ethyl-3-(1-chlor-2-(2-methyl-4-thiazolyl)ethenyl)-8,8-(1,3-trimethylen)-4,17-dioxabicyclo[14.1.0]heptadecan-5,9-dion
4S,7R,8S,9S,13(Z oder E),16S(Z))-4,8-Dihydroxy-16-(1-chlor-2-(2-methyl-4-thiazolyl)ethenyl)-1-aza-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-chlor-2-(2-methyl-4-thiazolyl)ethenyl)-10,12,16-trimethyl-8,8-(1,3-trimethylen)-4-aza-17-oxabicyclo[14.1.0]hepta-deca-5,9-dion
(4S,7R,8S,9S,13(Zoder E),16S(Z))-4,8-Dihydroxy-9,13-dimethyl-7-ethyl-16-(1-chlor-2-(2-methyl-4-thiazolyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-chlor-2-(2-methyl-4-thiazolyl)ethenyl)-8,8-(1,3-trimethylen)-4-aza-17-oxabicyclo[14.1.0]heptadecan-5,9-dion
(4S,7R,8S,9S,13(Zoder E),16S(Z))-4,8-Dihydroxy-16-(1-fluor-2-(2-pyridyl)ethenyl)-1-oxa-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-fluor-2-(2-pyridyl)ethenyl)-10,12,16-trimethyl-8,8-(1,3-trimethylen)-4,17-dioxabicyclo[14.1.0]hepta-deca-5,9-dion
(4S,7R,8S,9S,13(Z oder E),16S(Z))-4,8-Dihydroxy-9,13-dimethyl-7-ethyl-16-(1-fluor-2-(2-pyridyl)ethenyl)-1-oxa-5,5-(1,3-trimethylen)cyclohexadec-13-en-2,6-dion
(1 RS,3S(Z),7S,10R,11S,12S,16RS)-7,11-Dihydroxy-12,16-dimethyl-10-ethyl-3-(1-fluor-2-(2-pyridyl)ethenyl)-8,8-(1,3-trimethylen)-4,17-dioxabicyclo[14.1.0]hepta-decan-5,9-dion
4S,7R,8S,9S,13(Z oder E),16S(Z))-4,8-Dihydroxy-16-(1-fluor-2-(2-pyridyl)ethenyl)-1-aza-7,9,13-trimethyl-5,5-(1,3-trimethylen)cyclohexadec-13-en-2,6-dion
(1RS,3S(Z),7S,10R,11S,12S,16RS)-7,11 -Dihydroxy-3-(1-fluor-2-(2-pyridyl)ethenyl)-10,12,16-trimethyl-8,8-(1,3-trimethylen)-4-aza-17-oxabicyclo[14.1.0]hepta-deca-5,9-dion
(4S.7R.8S.9S. 13(Z oder E),16S(Z))-4,8-Dihydroxy-9,13-dimethyl-7-ethyl-16-(1-fluor-2-(2-pyridyl)ethenyl)-1-aza-5,5-(1,3-trimethylen)cyclohexadec-13-en-2,6-dion
(1RS,3S(Z),7S,10R,11S,12S,16RS)-7,11-Dihydroxy-12,16-dimethyl-10-ethyl-3-(1-fluor-2-(2-pyridyl)ethenyl)-8,8-(1,3-trimethylen)-4-aza-17-oxabicyclo[14.1.0]hepta-decan-5,9-dion
(4S,7R,8S,9S,13(Z oder E),16S(Z))-4,8-Dihydroxy-16-(1-chlor-2-(2-pyridyl)ethenyl)-1-oxa-7,9,13-trimethyl-5,5-(1,3-trimethylen)cyclohexadeo-13-en-2,6-dion
(1RS,3S(Z),7S,10R,11S,12S,16RS)-7,11-Dihydroxy-3-(1-chlor-2-(2-pyridyl)ethenyl)-10,12,16-trimethyl-8,8-(1,3-trimethylen)-4,17-dioxabicyclo[14.1.0]hepta-deca-5,9-dion
(4S,7R,8S,9S,13(Z oder E),16S(Z))-4,8-Dihydroxy-9,13-dimethyl-7-ethyl-16-(1-chlor-2-(2-pyridyl)ethenyl)-1 -oxa-5,5-(1,3-trimethylen)cyclohexadec-1 3-en-2,6-dion
(1 RS,3S(Z),7S,10R,11S,12S,16RS)-7,11-Dihydroxy-12,16-dimethyl-10-ethyl-3-(1-chlor-2-(2-pyridyl)ethenyl)-8,8-(1,3-trimethylen)-4,17-dioxabicyclo[14.1.0]hepta-decan-5,9-dion
4S,7R,8S,9S,13(Z oder E),16S(Z))-4,8-Dihydroxy-16-(1-chlor-2-(2-pyridyl)ethenyl)-1-aza-7,9,13-trimethyl-5,5-(1,3-trimethylen)cyclohexadec-13-en-2,6-dion
(1RS,3S(Z),7S,10R,11S,12S,16RS)-7,11-Dihydroxy-3-(1-chlor-2-(2-pyridyl)ethenyl)-10,12,16-trimethyl-8,8-(1,3-trimethylen)-4-aza-17-oxabicyclo[14.1.0]hepta-deca-5,9-dion
(4S,7R,8S,9S,13(Z oder E),16S(Z))-4,8-Dihydroxy-9,13-dimethyl-7-ethyl-16-(1-chlor-2-(2-pyridyl)ethenyl)-1-aza-5,5-(1,3-trimethylen)cyclohexadec-13-en-2,6-dion
(1 RS,3S(Z),7S, 10R, 11 S,12S,16RS)-7,11-Dihydroxy-12,16-dimethyl-10-ethyl-3-(1-chlor-2-(2-pyridyl)ethenyl)-8,8-(1,3-trimethylen)-4-aza-17-oxabicyclo[14.1.O]hepta-decan-5,9-dion

20. Pharmazeutische Präparate, enthaltend mindestens eine Verbindung der allgemeinen Formel I gemäß einem der vorstehenden Ansprüche 1 bis 19 sowie einen pharmazeutisch verträglichen Träger.

21. Verwendung der Verbindungen der allgemeinen Formel I gemäß den vorstehenden Ansprüchen 1 bis 19 zur Herstellung von Arzneimitteln.

22. Zwischenprodukte der allgemeinen Formel C 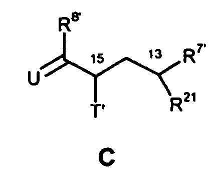 worin
R^{8'} die in der allgemeinen Formel für R⁸ genannte Bedeutung hat und
R^{7'} ein Wasserstoffatom,
T' eine Gruppe OR²⁰, wobei R²⁰ ein Wasserstoffatom oder eine Schutzgruppe PG² ist, ein Halogenatom, vorzugsweise ein Brom- oder Iod-Atom, eine Azido- oder eine geschützte Aminogruppe,
R²¹ eine Hydroxygruppe, Halogen, eine geschützte Hydroxygruppe OPG³, ein Phosphoniumhalogenidrest PPh₃⁺Hal⁻ (Ph = Phenyl; Hal = F, CI, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
U eine Gruppierung CR¹⁰R¹¹,
wobei
R¹⁰ R¹¹ gleich oder verschieden sind und für Wasserstoff, einen C₁-C₂₀-Alkyl-, Aryl-, C₇-C₂₀-Aralkylrest oder
R¹⁰ und R¹¹ zusammen mit dem Methylenkohlenstoffatom gemeinsam für einen 5- bis 7-gliedrigen carbocyclischen Ring
stehen,
ausgenommen die Verbindungen (E)-3-Fluor-1-phenyl-hex-2-en-4,6-diol und
2-Brom-3-(tert.-butyldimethylsilyloxy)-1-penten-5-ol.
bedeuten.

23. Zwischenprodukte der allgemeinen Formel BC 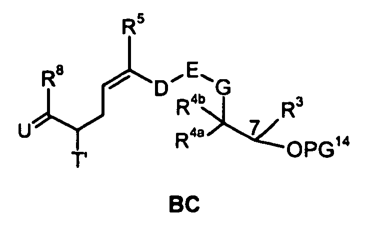 worin R³, R^{4a}, R^{4b}, R⁵, R⁸, D, E, G, T' und U die bereits genannten Bedeutungen haben und PG¹⁴ ein Wasserstoffatom oder eine Schutzgruppe PG darstellt.

24. Zwischenprodukte der allgemeinen Formel ABC 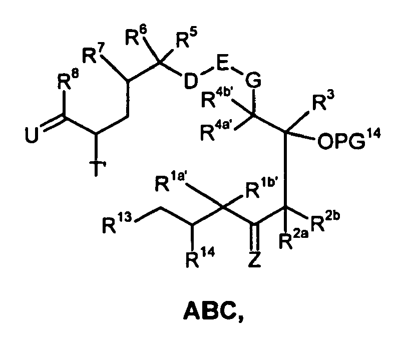 worin R^{1a'}, R^{1b}', R^{2a}', R^{2b}', R³, R^{4a}', R^{4b}', R⁵, R⁶, R⁷, R⁸, R¹³, R¹⁴, D, E, G, T', U und Z die bereits genannten Bedeutungen haben.

## Claims

1. Epothilone derivatives of general formula I, 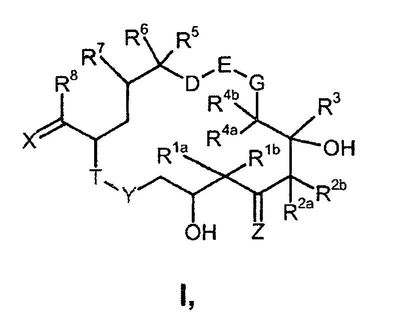 in which
R^{1a}, R^{1b} are the same or different and mean hydrogen, C₁-C₁₀ alkyl, aryl, or C₇-C₂₀ aralkyl, or together are a -(CH₂)ₘ group with m=2, 3, 4 or 5,
R^{2a}, R^{2b} are the same or different and mean hydrogen, C₁-C₁₀ alkyl, aryl, or C₇-C₂₀ aralkyl or together are a -(CH₂)ₙ group with n=2, 3, 4 or 5,
R³ is hydrogen, C₁-C₁₀ alkyl, aryl, or C₇-C₂₀ aralkyl,
G is an oxygen atom or a group -CH₂,
R^{4a}, R^{4b} are the same or different and mean hydrogen, C₁-C₁₀ alkyl, aryl, or C₇-C₂₀ aralkyl, or together are a-(CH₂)ₚ group with p=2, 3, 4 or 5,
D-E is a group of
H₂C-CH₂ HC=CH C≡C 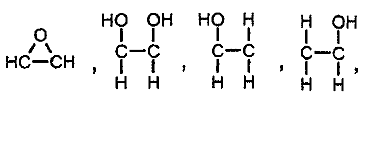 -H₂C-O-, -O-CH₂-
R⁵ is hydrogen, C₁-C₁₀ alkyl, aryl, C₇-C₂₀ aralkyl, CO₂H, CO₂-alkyl, CH₂ OH, CH₂O-alkyl, CH₂O-acyl, CN, CH₂ NH₂, CH₂ N(alkyl, acyl)_{1,2}, or CH₂ Hal
R⁶, R⁷ each are a hydrogen atom, or together an additional bond or an oxygen atom,
R⁸ is a halogen atom, or a cyano group,
X is a grouping CR¹⁰ R¹¹,
whereby one of the radicals
R¹⁰ stands for hydrogen, and the other radical
R¹¹ stands for an optionally substituted heteroaryl radical,
T-Y is a group O-C(=O), O-CH₂, CH₂C (=O), NR²⁴-C (=O), or NR²⁴-SO₂,
R²⁴ is hydrogen, or C₁-C₁₀ alkyl,
Z is an oxygen atom or H/OR¹², where
R¹² is hydrogen or a protective group PG^{z}.

2. A compound according to Claim 1, in which R⁸ is a fluorine atom or a chlorine atom.

3. A compound according to Claim 1 or 2, in which R^{2a} is a methyl, ethyl or propyl group.

4. A compound according to Claim 1 or 2, in which R^{1a} and R^{1b} together form a trimethylene group.

5. A compound according to Claim 1 or 2, in which R^{1a} and R^{1b} each are a methyl group.

6. A compound according to Claim 1 or 2, in which R¹⁰/R¹¹ is a 2-pyridyl radical/hydrogen.

7. A compound according to Claim 1 or 2, in which R¹⁰/R¹¹ is a 2-methyl-4-thiazolyl radical/hydrogen.

8. A compound according to Claim 1 or 2, in which R¹⁰/R¹¹ is a 2-hydroxymethyl-4-thiazolyl radical/hydrogen or a 2-methyl-4-oxazolyl radical/hydrogen or a 2-hydroxymethyl-4-oxazolyl radical/hydrogen.

9. A compound according to Claim 1 or 2, in which T-Y is a group O-C(=O).

10. A compound according to Claim 1 or 2, in which T-Y is a group NR²⁴-C(=O) with R²⁴ having the meaning given above.

11. A compound according to Claim 1 or 2, in which G is a methylene group.

12. A compound according to Claim 1 or 2, in which Z is an oxygen atom.

13. A compound according to Claim 1 or 2, in which -D-E is an ethylene group.

14. A compound according to Claim 1 or 2, in which R³ is a hydrogen atom.

15. A compound according to Claim 1 or 2, in which R^{4a}/R^{4b} is H/CH₃.

16. A compound according to Claim 2, in which R^{2a} /R^{2b} is methyl or ethyl/hydrogen.

17. A compound according to Claim 4 or 5, in which R¹⁰/R¹¹ is a 2-pyridyl radical/hydrogen or 2-methyl-4-thiazolyl radical/hydrogen or a 2-hydroxymethyl-4-thiazolyl radical/hydrogen or a 2-methyl-4-oxazolyl radical/hydrogen or a 2-hydroxymethyl-4-oxazolyl radical/hydrogen.

18. A compound according to Claim 17, in which R^{2a}/R^{2b} is methyl or ethyl/hydrogen.

19. A compound of general formula I, which is
(4S,7R,8S,9S,13(Z or E),165(Z))-4,8-Dihydroxy-16-(1-fluoro-2-(2-methyl-4-thiazolyl)ethenyl)-1-oxa-5,5,7,9,13-pentamethyl-cyclohexadec-13-ene-2,6-dione,
(1RS,3S(Z),7S,10R,11S,12S,16RS)-7,11-dihydroxy-3-(1-fluoro-2-(2-methyl-4-thiazolyl)ethenyl)-8,8,10,12,16-pentamethyl-4,17-dioxabicyclo[14.1.0]heptadecane-5,9-dione,
(4S,7R,8S,9S,13(Z or E),16S(Z))-4,8-dihydroxy-7-ethyl-16-(1-fluoro-2-(2-methyl-4-thiazolyl)ethenyl)-1-oxa-5,5,9,13-tetramethyl-cyclohexadec-13-ene-2,6-dione,
(1RS,3S(Z),7S,10R,11S,12S,16RS)-7,11-dihydroxy-10-ethyl-3-(1-fluoro-2-(2-methyl-4-thiazolyl)ethenyl)-8,8,12,16-tetramethyl-4,17-dioxabicyclo[14.1.0]heptadecane-5,9-dione,
(4S,7R,8S,9S,13(Z or E),165(z))-4,8-dihydroxy-16-(1-fluoro-2-(2-methyloxazol-4-yl)ethenyl)-1-oxa-5,5,7,9,13-pentamethyl-cyclohexadec-13-ene-2,6-dione,
(1RS,3S(Z),7S,10R,11S,12S,16RS)-7,11-dihydroxy-3-(1-fluoro-2-(2-methyloxazol-4-yl)ethenyl)-8,8,10,12,16-pentamethyl-4,17-dioxabicyclo[14.1.0]heptadecane-5,9-dione,
(4S,7R,8S,9S,13(Z or E),16S(Z))-4,8-dihydroxy-7-ethyl-16-(1-fluoro-2-(2-methyloxazol-4-yl)ethenyl)-1-oxa-5,5,9,13-tetramethyl-cyclohexadec-13-ene-2,6-dione,
(1RS,3S(Z),7S,10R,11S,12S,16RS)-7,11-dihydroxy-10-ethyl-3-(1-fluoro-2-(2-methyloxazol-4-yl)ethenyl)-8,8,12,16-tetramethyl-4,17-dioxabicyclo[14.1.0]heptadecane-5,9-dione,
(4S,7R,8S,9S,13(Z or E),16S(Z))-4,8-dihydroxy-16-(1-fluoro-2-(2-pyridyl)ethenyl)-1-oxa-5,5,7,9,13-pentamethyl-cyclohexadec-13-ene-2,6-dione,
(1RS,3S(Z),7S,10R,11S,12S,16RS)-7,11-dihydroxy-3-(1-fluoro-2-(2-pyridyl)ethenyl)-8,8,10,12,16-pentamethyl-4,17-dioxabicyclo[14.1. 0]heptadecane-5,9-dione,
(4S,7R,8S,9S,13(Z or E),16S(Z))-4,8-dihydroxy-7-ethyl-16-(1-fluoro-2-(2-pyridyl)ethenyl)-1-oxa-5,5,9,13-tetramethyl-cyclohexadec-13-ene-2,6-dione,
(1RS,3S(Z),7S,10R,11S,12S,16RS)-7,11-dihydroxy-10-ethyl-3-(1-fluoro-2-(2-pyridyl)ethenyl)-8,8,12,16-tetramethyl-4,17-dioxabicyclo[14.1.0]heptadecane-5,9-dione,
(4S,7R,8S,9S,13(Z or E),16S(Z))-4,8-dihydroxy-16-(1-fluoro-2-(2-methyl-4-thiazolyl)ethenyl)-1-aza-5,5,7,9,13-pentamethyl-cyclohexadec-13-ene-2,6-dione,
(1RS,3S(Z),7S,10R,11S,12S,16RS)-7,11-dihydroxy-3-(1-fluoro-2-(2-methyl-4-thiazolyl)ethenyl)-8,8,10,12,16-pentamethyl-4-aza-17-oxabicyclo[14.1. 0]heptadecane-5,9-dione,
(4S,7R,8S,9S,13(Z or E),16S(Z))-4,8-dihydroxy-7-ethyl-16-(1-fluoro-2-(2-methyl-4-thiazolyl)ethenyl)-1-aza-5,5,9,13-tetramethyl-cyclohexadec-13-ene-2,6-dione,
(1RS,3S(Z),7S,10R,11S,12S,16RS)-7,11-dihydroxy-10-ethyl-3-(1-fluoro-2-(2-methyl-4-thiazolyl)ethenyl)-8,8,12,16-tetramethyl-4-aza-17-oxabicyclo[14.1.0]-heptadecane-5,9-dione,
(4S,7R,8S,9S,13(z or E),16S(Z))-4,8-dihydroxy-16-(1-fluoro-2-(2-methyloxazol-4-yl)ethenyl)-1-aza-5,5,7,9,13-pentamethyl-cyclohexadec-13-ene-2,6-dione,
(1RS,3S(Z),7S,10R,11S,12S,16RS)-7,11-dihydroxy-3-(1-fluoro-2-(2-methyloxazol-4-yl)ethenyl)-8,8,10,12,16-pentamethyl-4-aza-17-oxabicyclo[14.1.0]heptadecane-5,9-dione,
(4S,7R,8S,9S,13(Z or E), 165(Z))-4,8-dihydroxy-7-ethyl-16-(1-fluoro-2-(2-methyloxazol-4-yl)ethenyl)-1-aza-5,5,9,13-tetramethyl-cyclohexadec-13-ene-2,6-dione,
(1RS,3S(Z),7S,10R,11S,12S,16RS)-7,11-dihydroxy-10-ethyl-3-(1-fluoro-2-(2-methyloxazol-4-yl)ethenyl)-8,8,12,16-tetramethyl-4-aza-17-oxabicyclo[14.1.0]-heptadecane-5,9-dione
(4S,7R,8S,9S,13(Z or E),16S(Z))-4,8-dihydroxy-16-(1-fluoro-2-(2-pyridyl)ethenyl)-1-aza-5,5,7,9,13-pentamethyl-cyclohexadec-13-ene-2,6-dione,
(1RS,3S(Z),7S,10R,11S,12S,16RS)-7,11-dihydroxy-3-(1-fluoro-2-(2-pyridyl)ethenyl)-8,8,10,12,16-pentamethyl-4-aza-17-oxabicyclo[14.1.0]heptadecane-5,9-dione,
(4S,7R,8S,9S,13(Z or E),16S(Z))-4,8-dihydroxy-7-ethyl-16-(1-fluoro-2-(2-pyridyl)ethenyl)-1-aza-5,5,9,13-tetramethyl-cyclohexadec-13-ene-2,6-dione,
(1RS,3S(Z),7S,10R,11S,12S,16RS)-7,11-dihydroxy-10-ethyl-3-(1-fluoro-2-(2-pyridyl)ethenyl)-8,8,12,16-tetramethyl-4-aza-17-oxabicyclo[14.1.0]heptadecane-5,9-dione,
(4S,7R,8S,9S,13(Z or E),16S(Z))-4,8-dihydroxy-16-(1-chloro-2-(2-methyl-4-thiazolyl)ethenyl)-1-oxa-5,5,7,9,13-pentamethyl-cyclohexadec-13-ene-2,6-dione,
(1RS,3S(Z),7S,10R,11S,12S,16RS)-7,11-dihydroxy-3-(1-chloro-2-(2-methyl-4-thiazolyl)ethenyl)-8,8,10,12,16-pentamethyl-4,17-dioxabicyclo[14.1.0]heptadecane-5,9-dione,
(4S,7R,8S,9S,13(Z or E),16S(Z))-4,8-dihydroxy-7-ethyl-16-(1-chloro-2-(2-methyl-4-thiazolyl)ethenyl)-1-oxa-5,5,9,13-tetramethyl-cyclohexadec-13-ene-2,6-dione,
(1RS,3S(Z),7S,10R,11S,12S,16RS)-7,11-dihydroxy-10-ethyl-3-(1-chloro-2-(2-methyl-4-thiazolyl)ethenyl)-8,8,12,16-tetramethyl-4,17-dioxabicyclo[14.1.0]-heptadecane-5,9-dione,
(4S,7R,8S,9S,13(Z or E),16S(Z))-4,8-dihydroxy-16-(1-chloro-2-(2-methyloxazol-4-yl)ethenyl)-1-oxa-5,5,7,9,13-pentamethyl-cyclohexadec-13-ene-2,6-dione,
(1RS,3S(Z),7S,10R,11S,12S,16RS)-7,11-dihydroxy-3-(1-chloro-2-(2-methyloxazol-4-yl)ethenyl)-8,8,10,12,16-pentamethyl-4,17-dioxabicyclo[14.1.0]heptadecane-5,9-dione,
(4S,7R,8S,9S,13(Z or E),16S(Z))-4,8-dihydroxy-7-ethyl-16-(1-chloro-2-(2-methyloxazol-4-yl)ethenyl)-1-oxa-5,5,9,13-tetramethyl-cyclohexadec-13-ene-2,6-dione,
(1RS,3S(Z),7S,10R,11S,12S,16RS)-7,11-dihydroxy-10-ethyl-3-(1-chloro-2-(2-methyloxazol-4-yl)ethenyl)-8,8,12,16-tetramethyl-4,17-dioxabicyclo[14.1.0]-heptadecane-5,9-dione,
(4S,7R,8S,9S,13(Z or E),16S(Z))-4,8-dihydroxy-16-(l-chloro-2-(2-pyridyl)ethenyl)-1-oxa-5,5,7,9,13-pentamethyl-cyclohexadec-13-ene-2,6-dione,
(1RS,3S(Z),7S,10R,11S,12S,16RS)-7,11-dihydroxy-3-(1-chloro-2-(2-pyridyl)ethenyl)-8,8,10,12,16-pentamethyl-4,17-dioxabicyclo[14.1.0]heptadecane-5,9-dione,
(4S,7R,8S,9S,13(Z or E),16S(Z))-4,8-dihydroxy-7-ethyl-16-(1-chloro-2-(2-pyridyl)ethenyl)-1-oxa-5,5,9,13-tetramethyl-cyclohexadec-13-ene-2,6-dione,
(1RS,3S(Z),7S,10R,11S,12S,16RS)-7,11-dihydroxy-10-ethyl-3-(1-chloro-2-(2-pyridyl)ethenyl)-8,8,12,16-tetramethyl-4,17-dioxabicyclo[14.1.0]heptadecane-5,9-dione,
(4S,7R,8S,9S,13(Z or E),16S(Z))-4,8-dihydroxy-16-(1-chloro-2-(2-methyl-4-thiazolyl)ethenyl)-l-aza-5,5,7,9,13-pentamethyl-cyclohexadec-13-ene-2,6-dione,
(1RS,3S(Z),7S,10R,11S,12S,16RS)-7,11-dihydroxy-3-(1-chloro-2-(2-methyl-4-thiazolyl)ethenyl)-8,8,10,12,16-pentamethyl-4-aza-17-oxabicyclo[14.1.0]heptadecane-5,9-dione,
(4S,7R,8S,9S,13(Z or E),16S(Z))-4,8-dihydroxy-7-ethyl-16-(1-chloro-2-(2-methyl-4-thiazolyl)ethenyl)-1-aza-5,5,9,13-tetramethyl-cyclohexadec-13-ene-2,6-dione,
(1RS,3S(Z),7S,10R,11S,12S,16RS)-7,11-dihydroxy-10-ethyl-3-(1-chloro-2-(2-methyl-4-thiazolyl)ethenyl)-8,8,12,16-tetramethyl-4-aza-17-oxabicyclo[14.1.0]-heptadecane-5,9-dione,
(4S,7R,8S,9S,13(Z or E), 16S(Z))-4,8-dihydroxy-16-(1-chloro-2-(2-methyloxazol-4-yl)ethenyl)-1-aza-5,5,7,9,13-pentamethyl-cyclohexadec-13-ene-2,6-dione,
(1RS,35(Z),75,10R,11S,12S,16RS)-7,11-dihydroxy-3-(1-chloro-2-(2-methyloxazol-4-yl)ethenyl)-8,8,10,12,16-pentamethyl-4-aza-17-oxabicyclo[14.1.0]heptadecane-5,9-dione,
(4S,7R,8S,9S,13(Z or E),16S(Z))-4,8-dihydroxy-7-ethyl-16-(1-chloro-2-(2-methyloxazol-4-yl)ethenyl)-1-aza-5,5,9,13-tetramethyl-cyclohexadec-13-ene-2,6-dione,
(1RS,3S(Z),7S,10R,11S,12S,16RS)-7,11-dihydroxy-10-ethyl-3-(1-chloro-2-(2-methyloxazol-4-yl)ethenyl)-8,8,12,16-tetramethyl-4-aza-17-oxabicyclo[14.1.0]heptadecane-5,9-dione,
(4S, 7R, 8S, 9S, 13(Z or E), 16S(Z))-4,8-dihydroxy-16-(1-chloro-2-(2-pyridyl)ethenyl)-1-aza-5,5,7,9,13-pentamethyl-cyclohexadec-13-ene-2,6-dione,
(1RS,3S(Z), 7S, 10R, 11S, 12S, 16RS)-7,11-dihydroxy-3-(1-chloro-2-(2-pyridyl)ethenyl)-8, 8, 10, 12, 16-pentamethyl-4-aza-17-oxabicyclo[14.1.0]heptadecane-5,9-dione,
(4S, 7R, 8S, 9S, 13 (Z or E),16S(Z))-4,8-dihydroxy-7-ethyl-16-(1-chloro-2-(2-pyridyl)ethenyl)-1-aza-5,5,9,13-tetramethyl-cyclohexadec-13-ene-2,6-dione,
(1RS,3S(Z),7S,10R,11S,12S,16RS)-7,11-dihydroxy-10-ethyl-3-(1-chloro-2-(2-pyridyl)ethenyl)-8,8,12,16-tetramethyl-4-aza-17-oxabicyclo[14.1.0]heptadecane-5,9-dione,
(4S,7R,8S,9S,13(Z or E),16S(Z))-4,8-dihydroxy-16-(1-fluoro-2-(2-methyl-4-thiazolyl)ethenyl)-1-oxa-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-fluoro-2-(2-methyl-4-thiazolyl)ethenyl)-10,12,16-trimethyl-8,8-(1,3-trimethylene)-4,17-dioxabicyclo-[14.1.0]hepta-decane-5,9-dione,
(4S,7R,8S,9S,13(Z or E),16S(Z))-4,8-dihydroxy-9,13-dimethyl-7-ethyl-16-(1-fluoro-2-(2-methyl-4-thiazolyl)-ethenyl)-1-oxa-5,5-(1,3-trimethylene)cyclohexadec-13-ene-2,6-dione,
(1RS,3S(Z),7S,10R,11S,12S,16RS)-7,11-dihydroxy-12,16-dimethyl-10-ethyl-3-(1-fluoro-2-(2-methyl-4-thiazolyl)ethenyl)-8,8-(1,3-trimethylene)-4,17-dioxabicyclo[14.1.0]hepta-decane-5,9-dione,
(4S,7R,8S,9S,13(Z or E),16S(Z))-4,8-dihydroxy-16-(1-fluoro-2-(2-methyl-4-thiazolyl)ethenyl)-l-aza-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-fluoro-2-(2-methyl-4-thiazolyl)ethenyl)-10,12,16-trimethyl-8,8-(1,3-trimethylene)-4-aza-17-oxabicy clo[14.1.0]hepta-decane-5,9-dione,
(4S,7R,8S,9S,13(Z or E),16S(Z))-4,8-dihydroxy-9,13-dimethyl-7-ethyl-16-(1-fluoro-2-(2-methyl-4-thiazolyl)-ethenyl)-1-aza-5,5-(1,3-trimethylene)cyclohexadec-13-ene-2,6-dione,
(1RS,3S(Z),7S,10R,11S,12S,16RS)-7,11-dihydroxy-12,16-dimethyl-10-ethyl-3-(1-fluoro-2-(2-methyl-4-thiazolyl)-ethenyl)-8,8-(1,3-trimethylene)-4-aza-17-oxabicyclo-[14.1.0]hepta-decane-5,9-dione,
(4S,7R,8S,9S,13(Z or E),16S(Z))-4,8-dihydroxy-16-(1-chloro-2-(2-methyl-4-thiazolyl)ethenyl)-1-oxa-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-chloro-2-(2-methyl-4-thiazolyl)ethenyl)-10,12,16-trimethyl-8,8-(1,3-trimethylene)-4,17-dioxabicyclo-[14.1.0]hepta-decane-5,9-dione,
(4S,7R,8S,9S,13(Z or E),16S(Z))-4,8-dihydroxy-9,13-dimethyl-7-ethyl-16-(1-chloro-2-(2-methyl-4-thiazolyl)-ethenyl)-1-oxa-5,5-(1,3-trimethylene)cyclohexadec-13-ene-2,6-dione,
(1RS,3S(Z),7S,10R,11S,12S,16RS)-7,11-dihydroxy-12,16-dimethyl-10-ethyl-3-(1-chloro-2-(2-methyl-4-thiazolyl)-ethenyl)-8,8-(1,3-trimethylene)-4,17-dioxabicyclo-[14.1.0]heptadecane-5,9-dione,
4S,7R,8S,9S,13(Z or E), 165(Z))-4,8-dihydroxy-16-(1-chloro-2-(2-methyl-4-thiazolyl)ethenyl)-1-aza-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-chloro-2-(2-methyl-4-thiazolyl)ethenyl)-10,12,16-trimethyl-8,8-(1,3-trimethylene)-4-aza-17-oxabicy clo[14.1.0]hepta-decane-5,9-dione,
(4S,7R,8S,9S,13(z or E),16S(Z))-4,8-dihydroxy-9,13-dimethyl-7-ethyl-16-(1-chloro-2-(2-methyl-4-thiazolyl)-ethenyl)-1-aza-5,5-(1,3-trimethylene)cyclohexadec-13-ene-2,6-dione,
(1RS,3S(Z),7S,10R,11S,12S,16RS)-7,11-dihydroxy-12,16-dimethyl-10-ethyl-3-(1-chloro-2-(2-methyl-4-thiazolyl)-ethenyl)-8,8-(1,3-trimethylene)-4-aza-17-ox abicyclo[14.1.0]heptadecane-5,9-dione,
(4S,7R,8S,9S,13(Z or E),16S(Z))-4,8-dihydroxy-16-(1-fluoro-2-(2-pyridyl)ethenyl)-1-oxa-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-fluoro-2-(2-pyridyl)ethenyl)-10,12,16-trimethyl-8,8-(1,3-trimethylene)-4,17-dioxabicyclo[14.1.0]heptadecane-5,9-dione,
(4S,7R,8S,9S,13(Z or E),165(Z))-4,8-dihydroxy-9,13-dimethyl-7-ethyl-16-(1-fluoro-2-(2-pyridyl)ethenyl)-1-oxa-5,5-(1,3-trimethylene)cyclohexadec-13-ene-2,6-dione,
(1RS,3S(Z),7S,10R,11S,12S,16RS)-7,11-dihydroxy-12,16-dimethyl-10-ethyl-3-(1 -fluoro-2-(2-pyridyl)ethenyl)-8,8-(1,3-trimethylene)-4,17-dioxabicyclo[14. 1.0]heptadecane-5,9-dione,
4S,7R,8S,9S,13(Z or E),16S(Z))-4,8-dihydroxy-16-(1-fluoro-2-(2-pyridyl)ethenyl)-1-aza-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-fluoro-2-(2-pyridyl)ethenyl)-10,12,16,-trimethyl-8,8-(1,3-trimethylene)-4-aza-17-oxabicyclo[14.1.0]heptadeca-5,9-dione,
(4S,7R,8S,9S,13(Z or E),16S(Z))-4,8-dihydroxy-9,13-dimethyl-7-ethyl-16-(1-fluoro-2-(2-pyridyl)ethenyl)-1-aza-5,5-(1,3-trimethylene)cyclohexadec-13-ene-2,6-dione,
(1RS,3S(Z),7S,10R,11S,12S,16RS)-7,11-dihydroxy-12,16-dimethyl-10-ethyl-3-(1-fluoro-2-(2-pyridyl)ethenyl)-8,8-(1,3-trimethylene)-4-aza-17-oxabicyclo[14 .1.0]Hepta-decane-5,9-dione,
(4S,7R,8S,9S,13 (Z or E),16S(Z))-4,8-dihydroxy-16-(1-chloro-2-(2-pyridyl)ethenyl)-1-oxa-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-chloro-2-(2-pyridyl)ethenyl)-10,12,16-trimethyl-8,8-(l,3-trimethylene)-4,17-dioxabicyclo[14.1. 0]heptadecane-5,9-dione,
(4S,7R,8S,9S,13(Z or E),165(Z))-4,8-dihydroxy-9,13-dimethyl-7-ethyl-16-(1-chloro-2-(2-pyridyl)ethenyl)-1-oxa-5,5-(1,3-trimethylene)cyclohexadec-13-ene-2,6-dione,
(1RS,3S(Z),7S,lOR,11S,12S,16RS)-7,11-dihydroxy-12,16-dimethyl-10-ethyl-3-(1-chloro-2-(2-pyridyl)ethenyl)-8,8-(1,3-trimethylene)-4,17-dioxabicyclo[14. 1.0]heptadecane-5,9-dione,
4S,7R,8S,9S,13(Z or E),165(Z))-4,8-dihydroxy-16-(1-chloro-2-(2-pyridyl)ethenyl)-1-aza-7,9,13-trimethyl-5,5-(1,3-trimethylene)cyclohexadec-13-ene-2,6-dione,
(1RS,3S(Z),7S,10R,11S,12S,16RS)-7,11-dihydroxy-3-(1-chloro-2-(2-pyridyl)ethenyl)-10,12,16-trimethyl-8,8-(1,3-trimethylene)-4-aza-17-oxabicyclo[14.1. 0]heptadecane-5,9-dione,
(4S,7R,8S,9S,13(Z or E),16S(Z))-4,8-dihydroxy-9,13-dimethyl-7-ethyl-16-(l-chloro-2-(2-pyridyl)ethenyl)-l-aza-5,5-(1,3-trimethylene)cyclohexadec-13-ene-2,6-dione, or
(1RS,3S(Z),7S,10R,11S,12S,16RS)-7,11-dihydroxy-12,16-dimethyl-10-ethyl-3-(1-chloro-2-(2-pyridyl)ethenyl)-8,8-(1,3-trimethylene)-4-aza-17-oxabicyclo[14 .1.0]hepta-decane-5,9-dione.

20. A pharmaceutical composition that contains at least one compound of general formula I according to one of the preceding Claims 1 to 19 and a pharmaceutically compatible vehicle.

21. Use of the compounds of the general formula I according to the preceding Claims 1 to 19 in the manufacture of medicaments.

22. Intermediates of the general formula C 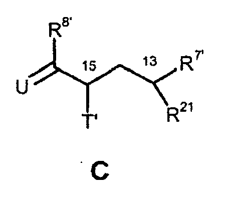 where
R⁸' has the meaning mentioned for R⁸ in the general formula I and
R⁷, means a hydrogen atom,
T' means a group OR²⁰, whereby R²⁰ is a hydrogen atom or a protective group PG², a halogen atom, preferably a bromine or iodine atom, an azido group or a protected amino group,
R²¹ means a hydroxy group, halogen, a protected hydroxy group OPG³, a phosphonium halide radical PPh₃⁺Hal⁻ (ph= phenyl; Hal=F, Cl, Br, I), a phosphonate radical P(O) (OQ)₂ (Q=C₁-C₁₀ alkyl or phenyl) or a phosphine oxide radical P(O)Ph₂ (Ph=phenyl),
U means a grouping CR¹⁰ R¹¹, whereby
R¹⁰, R¹¹ are the same or different and stand for hydrogen, a C₁-C₂₀ alkyl, aryl, C₇-C₂₀ aralkyl radical or R¹⁰ and R¹¹ together with the methylene carbon atom commonly stand for a 5- to 7-membered carbocyclic ring,
excluding the compounds (E)-3-fluoro-1-phenylhex-2-ene-4,6-diol and 2-bromo-3-(tert-butyldimethylsilyloxy)-1-penten-5-ol.

23. Intermediates of the general formula BC 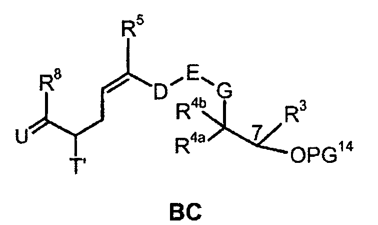 where R³, R^{4a}, R^{4b}, R⁵, R⁸, D, E, G, T' and U are each as defined above and PG¹⁴ is a hydrogen atom or a protective group PG.

24. Intermediates of the general formula ABC 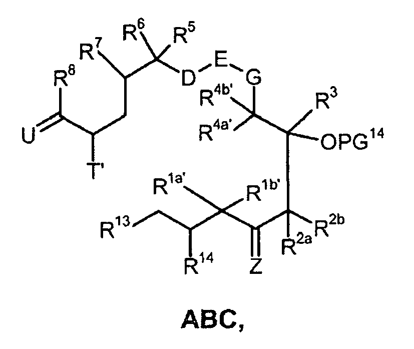 where R^{1a'}, R^{1b'}, R^{2a'}, R^{2b'}, R³, R^{4a'}, R^{4b'}, R⁵, R⁶, R⁷, R⁸, R¹³, R¹⁴, D, E, G, T', U and Z are each as defined above.

## Revendications

1. Dérivés d'épothilone de formule générale I 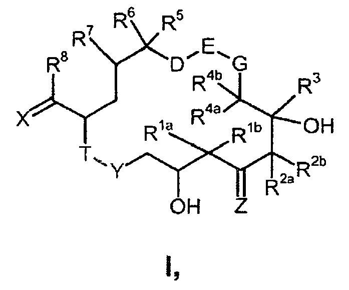 où
R^{1a}, R^{1b} sont identiques ou différents et signifient hydrogène, C₁-C₁₀-alkyle, aryle, C₇-C₂₀-aralkyle ou ensemble un groupe -(CH₂)ₘ- avec m = 2, 3, 4 ou 5,
R^{2a} R^{2b} sont identiques ou différents et signifient hydrogène, C₁-C₁₀-alkyle, aryle, C₇-C₂₀-aralkyle ou ensemble un groupe -(CH₂)ₙ- avec n = 2, 3, 4 ou 5,
_{R}3 signifie hydrogène, C₁-C₁₀-alkyle, aryle, C₇-C₂₀-aralkyle,
G signifie un atome d'oxygène ou un groupe -CH₂,
R^{4a}, R^{4b} sont identiques ou différents et signifient hydrogène, C₁-C₁₀-alkyle, aryle, C₇-C₂₀-aralkyle ou ensemble un groupe -(CH₂)ₚ- avec p = 2, 3, 4 ou 5,
D-E signifie un groupe
H₂C-CH₂, HC=CH, C≡C , 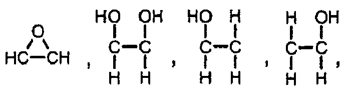 -H₂C-O-, -O-CH₂-
R⁵ signifie hydrogène, C₁-C₁₀-alkyle, aryle, C₇-C₂₀-aralkyle, CO₂H, CO₂-alkyle, CH₂OH, CH₂O-alkyle, CH₂O-acyle, CN, CH₂NH₂, CH₂N(alkyle, acyle)_{1,2}, CH₂Hal
R⁶, R⁷ signifient chacun un atome d'hydrogène, ensemble une liaison supplémentaire ou un atome d'oxygène,
R⁸ signifie un atome d'halogène ou un groupe cyano,
X signifie un groupe CR¹⁰R¹¹, un des radicaux
R¹⁰ représentant hydrogène et l'autre radical
R¹¹ représentant un radical hétéroaryle le cas échéant substitué,
T-Y signifie un groupe O-C(=O), O-CH₂, CH₂C(=O), NR²⁴-C (=O), NR²⁴-SO₂,
R²⁴ signifie hydrogène, C₁-C₁₀-alkyle,
Z signifie un atome d'oxygène ou H/OR¹²,
R¹² représentant hydrogène ou un groupe de protection PG^{z}.

2. Composés selon la revendication 1, où R⁸ représente un atome de fluor ou de chlore.

3. Composés selon la revendication 1 ou 2, où _{R}^{2a} signifie un groupe méthyle, éthyle ou propyle.

4. Composés selon la revendication 1 ou 2, où R^{1a} et R^{1b} signifient ensemble un groupe triméthylène.

5. Composés selon la revendication 1 ou 2, où R^{1a} et R^{1b} signifient chacun un groupe méthyle.

6. Composés selon la revendication 1 ou 2, où R¹⁰/R¹¹ représentent un radical 2-pyridyle/hydrogène.

7. Composés selon la revendication 1 ou 2, où R¹⁰/R¹¹ représentent un radical 2-méthyl-4-thiazolyle/hydrogène.

8. Composés selon la revendication 1 ou 2, où R¹⁰/R¹¹ représentent un radical 2-hydroxyméthyl-4-thiazolyle/hydrogène ou un radical 2-méthyl-4-oxazolyle/hydrogène ou un radical 2-hydroxyméthyl-4-oxazolyle/hydrogène.

9. Composés selon la revendication 1 ou 2, où T-Y représente un groupe O-C(=O).

10. Composés selon la revendication 1 ou 2, où T-Y représente un groupe NR²⁴-C (=O) avec R²⁴ ayant la signification déjà indiquée.

11. Composés selon la revendication 1 ou 2, où G représente un groupe méthylène.

12. Composés selon la revendication 1 ou 2, où Z représente un atome d'oxygène.

13. Composés selon la revendication 1 ou 2, où -DE- représente un groupe éthylène.

14. Composés selon la revendication 1 ou 2, où R³ représente un atome d'hydrogène.

15. Composés selon la revendication 1 ou 2, où R^{4a}/R^{4b} représentent H/CH₃.

16. Composés selon la revendication 2, où R^{2a}/R^{2b} représentent méthyle ou éthyle/hydrogène.

17. Composés selon la revendication 4 ou 5, où R¹⁰/R¹¹ représentent un radical 2-pyridyle/hydrogène ou un radical 2-méthyl-4-thiazolyle/hydrogène ou un radical 2-hydroxyméthyl-4-thiazolyle/hydrogène ou un radical 2-méthyl-4-oxazolyle/hydrogène ou un radical 2-hydroxyméthyl-4-oxazolyle/hydrogène.

18. Composés selon la revendication 17, où R^{2a}/R^{2b} représentent méthyle ou éthyle/hydrogène.

19. Composés de formule générale I, notamment
- (4S,7R,8S,9S,13(Z ou E),165(Z))-4,8-dihydroxy-16-(1-fluoro-2-(2-méthyl-4-thiazolyl)éthényl)-1-oxa-5,5,7,9,13-pentaméthylcyclohexadéc-13-ène-2,6-dione
- (1RS,3S(Z),7S,10R,11S,12S,16RS)-7,11-dihydroxy-3-(1-fluoro-2-(2-méthyl-4-thiazolyl)éthényl)-8,8,10,12,16-pentaméthyl-4,17-dioxabicyclo[14.1.0]heptadécane-5,9-dione
- (4S,7R,8S,9S,13(Z ou E),165(Z))-4,8-dihydroxy-7-éthyl-16-(1-fluoro-2-(2-méthyl-4-thiazolyl)éthényl)-1-oxa-5,5,9,13-tétraméthylcyclohexadéc-13-ène-2,6-dione
- (1RS,3S(Z),7S,10R,11S,12S,16RS)-7,11-dihydroxy-10-éthyl-3-(1-fluoro-2-(2-méthyl-4-thiazolyl)éthényl)-8,8,12,16-tétraméthyl-4,17-dioxabicyclo[14.1.0]heptadécane-5,9-dione
- (4S,7R,8S,9S,13(Z ou E),165(Z))-4,8-dihydroxy-16-(1-fluoro-2-(2-méthyloxazol-4-yl)éthényl)-1-oxa-5,5,7,9,13-pentaméthylcyclohexadéc-13-ène-2,6-dione
- (1RS,3S(Z),7S,10R,11S,12S,16RS)-7,11-dihydroxy-3-(1-fluoro-2-(2-méthyloxazol-4-yl)éthényl)-8,8,10,12,16-pentaméthyl-4,17-dioxabicyclo[14.1.0]heptadécane-5,9-dione
- (4S,7R,8S,9S,13(z ou E),16S(Z))-4,8-dihydroxy-7-éthyl-16-(1-fluoro-2-(2-méthyloxazol-4-yl-éthényl)-1-oxa-5,5,9,13-tétraméthylcyclohexadéc-13-ène-2,6-dione
- (1RS,3S(Z),7S,10R,11S,12S,16RS)-7,11-dihydroxy-10-éthyl-3-(1-fluoro-2-(2-méthyloxazol-4-yl)éthényl)-8,8,12,16-tétraméthyl-4,17-dioxabicyclo[14.1.0]heptadécane-5,9-dione
- (4S,7R,8S,9S,13(Z ou E),16S(Z))-4,8-dihydroxy-16-(l-fluoro-2-(2-pyridyl)éthényl)-1-oxa-5,5,7,9,13-pentaméthylcyclohexadéc-13-ène-2,6-dione
- (1RS,3S(Z),7S,10R,11S,12S,16RS)-7,11-dihydroxy-3-(1-fluoro-2-(2-pyridyl)éthényl)-8,8,10,12,16-pentaméthyl-4,17-dioxabicyclo[14.1.0]heptadécane-5,9-dione
- (4S,7R,8S,9S,13(Z ou E),16S(Z))-4,8-dihydroxy-7-éthyl-16-(1-fluoro-2-(2-pyridyl)éthényl)-1-oxa-5,5,9,13-tétraméthylcyclohexadéc-13-ène-2,6-dione
- (1RS,3S(Z),7S,10R,11S,12S,16RS)-7,11-dihydroxy-10-éthyl-3-(1-fluoro-2-(2-pyridyl)éthényl)-8,8,12,16-tétraméthyl-4,17-dioxabicyclo[14.1.0]heptadécane-5,9-dione
- (4S,7R,8S,9S,13(Z ou E),165(Z))-4,8-dihydroxy-16-(1-fluoro-2-(2-méthyl-4-thiazolyl)éthényl)-1-aza-5,5,7,9,13-pentaméthylcyclohexadéc-13-ène-2,6-dione
- (1RS,3S(Z),7S,10R,11S,12S,16RS)-7,11-dihydroxy-3-(1-fluoro-2-(2-méthyl-4-thiazolyl)éthényl)-8,8,10,12,16-pentaméthyl-4-aza-17-oxabicyclo[14.1.0]heptadécane-5,9-dione
- (4S,7R,8S,9S,13(Z ou E),165(Z))-4,8-dihydroxy-7-éthyl-16-(1-fluoro-2-(2-méthyl-4-thiazolyl)éthényl)-1-aza-5,5,9,13-tétraméthylcyclohexadéc-13-ène-2,6-dione
- (1RS,3S(Z),7S,10R,11S,12S,16RS)-7,11-dihydroxy-10-éthyl-3-(1-fluoro-2-(2-méthyl-4-thiazolyl)éthényl)-8,8,12,16-tétraméthyl-4-aza-17-oxabicyclo[14.1.0]heptadécane-5,9-dione
- (4S,7R,8S,9S,13(Z ou E), 16S(Z))-4,8-dihydroxy-16-(1-fluoro-2-(2-méthyloxazol-4-yl)éthényl)-1-aza-5,5,7,9,13-pentaméthylcyclohexadéc-13-ène-2,6-dione
- (1RS,3S(Z),7S,10R,11S,12S,16RS)-7,11-dihydroxy-3-(1-fluoro-2-(2-méthyloxazol-4-yl)éthényl)-8,8,10,12,16-pentaméthyl-4-aza-17-oxabicyclo[14.1.0]heptadécane-5,9-dione
- (4S,7R,8S,9S,13(Z ou E),16S(Z))-4,8-dihydroxy-7-éthyl-16-(1-fluoro-2-(2-méthyloxazol-4-yl)éthényl)-1-aza-5,5,9,13-tétraméthylcyclohexadéc-13-ène-2,6-dione
- (1RS,3S(Z),7S,10R,11S,12S,16RS)-7,11-dihydroxy-10-éthyl-3-(1-fluoro-2-(2-méthyloxazol-4-yl)éthényl)-8,8,12,16-tétraméthyl-4-aza-17-oxabicyclo[14.1.0]heptadécane-5,9-dione
- (4S,7R,8S,9S,13(Z ou E),165(Z))-4,8-dihydroxy-16-(1-fluoro-2-(2-pyridyl)éthényl)-1-aza-5,5,7,9,13-pentaméthylcyclohexadéc-13-ène-2,6-dione
- (1RS,3S(Z),7S,10R,11S,12S,16RS)-7,11-dihydroxy-3-(1-fluoro-2-(2-pyridyl)éthényl)-8,8,10,12,16-pentaméthyl-4-aza-17-oxabicyclo[14.1.0]heptadécane-5,9-dione
- (4S,7R,8S,9S,13(Z ou E),16S(Z))-4,8-dihydroxy-7-éthyl-16-(1-fluoro-2-(2-pyridyl)éthényl)-1-aza-5,5,9,13-tétraméthylcyclohexadéc-13-ène-2,6-dione
- (1RS,3S(Z),7S,10R,11S,12S,16RS)-7,11-dihydroxy-10-éthyl-3-(1-fluoro-2-(2-pyridyl)éthényl)-8,8,12,16-tétraméthyl-4-aza-17-oxabicyclo[14.1.0]heptadécane-5,9-dione
- (4S, 7R, 8S, 9S, 13(Z ou E), 16S(Z))-4,8-dihydroxy-16-(1-chloro-2-(2-méthyl-4-thiazolyl)éthényl)-1-oxa-5, 5, 7, 9, 13-pentaméthylcyclohexadéc-13-ène-2,6-dione
- (1RS, 3S(Z), 7S, 10R, 11S, 12S, 16RS)-7,11-dihydroxy-3-(1-chloro-2-(2-méthyl-4-thiazolyl)éthényl)-8,8,10,12,16-pentaméthyl-4,17-dioxabicyclo[14.1.0]heptadécane-5,9-dione
- (4S,7R,8S,9S,13(Z ou E),16S(Z))-4,8-dihydroxy-7-éthyl-16-(1-chloro-2-(2-méthyl-4-thiazolyl)éthényl)-1-oxa-5,5,9,13-tétraméthylcyclohexadéc-13-ène-2,6-dione
- (1RS,3S(z),7S,10R,11S,12S,16RS)-7,11-dihydroxy-10-éthyl-3-(1-chloro-2-(2-méthyl-4-thiazolyl)éthényl)-8,8,12,16-tétraméthyl-4,17-dioxabicyclo[14.1.0]heptadécane-5,9-dione
- (4S,7R,8S,9S,13(Z ou E),165(Z))-4,8-dihydroxy-16-(1-chloro-2-(2-méthyloxazol-4-yl)éthényl)-1-oxa-5,5,7,9,13-pentaméthylcyclohexadéc-13-ène-2,6-dione
- (1RS,3S(Z),7S,10R,11S,12S,16RS)-7,11-dihydroxy-3-(1-chloro-2-(2-méthyloxazol-4-yl)éthényl)-8,8,10,12,16-pentaméthyl-4,17-dioxabicyclo[14.1.0]heptadécane-5,9-dione
- (4S,7R,8S,9S,13(Z ou E),16S(Z))-4,8-dihydroxy-7-éthyl-16-(1-chloro-2-(2-méthyloxazol-4-yl)éthényl)-1-oxa-5,5,9,13-tétraméthylcyclohexadéc-13-ène-2,6-dione
- (1RS,3S(Z),7S,10R,11S,12S,16RS)-7,11-dihydroxy-10-éthyl-3-(1-chloro-2-(2-méthyloxazol-4-yl)éthényl)-8,8,12,16-tétraméthyl-4,17-dioxabicyclo[14.1.0]heptadécane-5,9-dione
- (4S,7R,8S,9S,13(Z ou E),16S(Z))-4,8-dihydroxy-16-(1-chloro-2-(2-pyridyl)éthényl)-1-oxa-5,5,7,9,13-pentaméthylcyclohexadéc-13-ène-2,6-dione
- (1RS,3S(Z),7S,10R,11S,12S,16RS)-7,11-dihydroxy-3-(1-chloro-2-(2-pyridyl)éthényl)-8,8,10,12,16-pentaméthyl-4,17-dioxabicyclo[14.1.0]heptadécane-5,9-dione
- (4S,7R,8S,9S,13(Z ou E),16S(Z))-4,8-dihydroxy-7-éthyl-16-(1-chloro-2-(2-pyridyl)éthényl)-1-oxa-5,5,9,13-tétraméthylcyclohexadéc-13-ène-2,6-dione
- (1RS,3S(Z),7S,10R,11S,12S,16RS)-7,11-dihydroxy-10-éthyl-3-(1-chloro-2-(2-pyridyl)éthényl)-8,8,12,16-tétraméthyl-4,17-dioxabicyclo[14.1.0]heptadécane-5,9-dione
- (4S,7R,8S,9S,13(Z ou E),16S(Z))-4,8-dihydroxy-16-(1-chloro-2-(2-méthyl-4-thiazolyl)éthényl)-1-aza-5,5,7,9,13-pentaméthylcyclohexadéc-13-ène-2,6-dione
- (1RS,3S(Z),7S,10R,11S,12S,16RS)-7,11-dihydroxy-3-(1-chloro-2-(2-méthyl-4-thiazolyl)éthényl)-8,8,10,12,16-pentaméthyl-4-aza-17-oxabicyclo[14.1.0]heptadécane-5,9-dione
- (4S,7R,8S,9S,13(Z ou E),16S(Z))-4,8-dihydroxy-7-éthyl-16-(1-chloro-2-(2-méthyl-4-thiazolyl)éthényl)-1-aza-5,5,9,13-tétraméthylcyclohexadéc-13-ène-2,6-dione
- (1RS,3S(Z),7S,10R,11S,12S,16RS)-7,11-dihydroxy-10-éthyl-3-(1-chloro-2-(2-méthyl-4-thiazolyl)éthényl)-8,8,12,16-tétraméthyl-4-aza-17-oxabicyclo[14.1.0]heptadécane-5,9-dione
- (4S,7R,8S,9S,13(Z ou E),16S(Z))-4,8-dihydroxy-16-(1-chloro-2-(2-méthyloxazol-4-yl)éthényl)-1-aza-5,5,7,9,13-pentaméthylcyclohexadéc-13-ène-2,6-dione
- (1RS,3S(Z),7S,10R,11S,12S,16RS)-7,11-dihydroxy-3-(1-chloro-2-(2-méthyloxazol-4-yl)éthényl)-8,8,10,12,16-pentaméthyl-4-aza-17-oxabicyclo[14.1.0]heptadécane-5,9-dione
- (4S,7R,8S,9S,13(Z ou E),16S(Z))-4,8-dihydroxy-7-éthyl-16-(1-chloro-2-(2-méthyloxazol-4-yl)éthényl)-1-aza-5,5,9,13-tétraméthylcyclohexadéc-13-ène-2,6-dione
- (1RS,3S(Z),7S,10R,11S,12S,16RS)-7,11-dihydroxy-10-éthyl-3-(1-chloro-2-(2-méthyloxazol-4-yl)éthényl)-8,8,12,16-tétraméthyl-4-aza-17-oxabicyclo[14.1.0]heptadécane-5,9-dione
- (4S,7R,8S,9S,13(Z ou E),16S(Z))-4,8-dihydroxy-16-(1-chloro-2-(2-pyridyl)éthényl)-1-aza-5,5,7,9,13-pentaméthylcyclohexadéc-13-ène-2,6-dione
- (1RS,3S(Z),7S,10R,11S,12S,16RS)-7,11-dihydroxy-3-(1-chloro-2-(2-pyridyl)éthényl)-8,8,10,12,16-pentaméthyl-4-aza-17-oxabicyclo[14.1.0]heptadécane-5,9-dione
- (4S,7R,8S,9S,13(Z ou E),16S(Z))-4,8-dihydroxy-7-éthyl-16-(1-chloro-2-(2-pyridyl)éthényl)-1-aza-5,5,9,13-tétraméthylcyclohexadéc-13-ène-2,6-dione
- (1RS,3S(Z),7S,10R,11S,12S,16RS)-7,11-dihydroxy-10-éthyl-3-(1-chloro-2-(2-pyridyl)éthényl)-8,8,12,16-tétraméthyl-4-aza-17-oxabicyclo[14.1.0]heptadécane-5,9-dione
- (4S,7R,8S,9S,13(Z ou E),165(Z))-4,8-dihydroxy-16-(1-fluoro-2-(2-méthyl-4-thiazolyl)éthényl)-1-oxa-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-fluoro-2-(2-méthyl-4-thiazolyl)éthényl)-10,12,16-triméthyl-8,8-(1,3-triméthylène)-4,17-dioxabicyclo[14.1.0]heptadécane-5,9-dione
- (4S,7R,8S,9S,13(Z ou E),165(Z))-4,8-dihydroxy-9,13-diméthyl-7-éthyl-16-(1-fluoro-2-(2-méthyl-4-thiazolyl)éthényl)-1-oxa-5,5-(1,3-triméthylène)cyclohexadéc-13-ène-2,6-dione
- (1RS,3S(Z),7S,10R,11S,12S,16RS)-7,11-dihydroxy-12,16-diméthyl-10-éthyl-3-(1-fluoro-2-(2-méthyl-4-thiazolyl)éthényl)-8,8-(1,3-triméthylène)-4,17-dioxabicyclo[14.1.0]heptadécane-5,9-dione
- 4S,7R,8S,9S,13(Z ou E),165(Z))-4,8-dihydroxy-16-(1-fluoro-2-(2-méthyl-4-thiazolyl)éthényl)-1-aza-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-fluoro-2-(2-méthyl-4-thiazolyl)éthényl)-10,12,16-triméthyl-8,8-(1,3-triméthylène)-4-aza-17-oxabicyclo[14.1.0]heptadécane-5,9-dione
- (4S,7R,8S,9S,13(Z ou E),16S(Z))-4,8-dihydroxy-9,13-diméthyl-7-éthyl-16-(1-fluoro-2-(2-méthyl-4-thiazolyl)éthényl)-1-aza-5,5-(1,3-triméthylène)cyclohexadéc-13-ène-2,6-dione
- (1RS,3S(Z),7S,10R,11S,12S,16RS)-7,11-dihydroxy-12,16-diméthyl-10-éthyl-3-(1-fluoro-2-(2-méthyl-4-thiazolyl)éthényl)-8,8-(1,3-triméthylène)-4-aza-17-oxabicyclo[14.1.0]heptadécane-5,9-dione
- (4S,7R,8S,9S,13(Z ou E),16S(Z))-4,8-dihydroxy-16-(1-chloro-2-(2-méthyl-4-thiazolyl)éthényl)-1-oxa-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-chloro-2-(2-méthyl-4-thiazolyl)éthényl)-10,12,16-triméthyl-8,8-(1,3-triméthylène)-4,17-dioxabicyclo[14.1.0]heptadécane-5,9-dione
- (4S,7R,8S,9S,13(Z ou E),16S(Z))-4,8-dihydroxy-9,13-diméthyl-7-éthyl-16-(1-chloro-2-(2-méthyl-4-thiazolyl)éthényl)-1-oxa-5,5-(1,3-triméthylène)cyclohexadéc-13-ène-2,6-dione
- (1RS,3S(Z),7S,10R,11S,12S,16RS)-7,11-dihydroxy-12,16-diméthyl-10-éthyl-3-(1-chloro-2-(2-méthyl-4-thiazolyl)éthényl)-8,8-(1,3-triméthylène)-4,17-dioxabicyclo[14.1.0]heptadécane-5,9-dione
- 4S,7R,8S,9S,13(Z ou E),165(Z))-4,8-dihydroxy-16-(1-chloro-2-(2-méthyl-4-thiazolyl)éthényl)-1-aza-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-chloro-2-(2-méthyl-4-thiazolyl)éthényl)-10,12,16-triméthyl-8,8-(1,3-triméthylène)-4-aza-17-oxabicyclo[14.1.0]heptadécane-5,9-dione
- (4S,7R,8S,9S,13(Z ou E),165(Z))-4,8-dihydroxy-9,13-diméthyl-7-éthyl-16-(1-chloro-2-(2-méthyl-4-thiazolyl)éthényl)-1-aza-5,5-(1,3-triméthylène)cyclohexadéc-13-ène-2,6-dione
- (1RS,3S(Z),7S,10R,11S,12S,16RS)-7,11-dihydroxy-12,16-diméthyl-10-éthyl-3-(1-chloro-2-(2-méthyl-4-thiazolyl)éthényl)-8,8-(1,3-triméthylène)-4-aza-17-oxabicyclo[14.1.0]heptadécane-5,9-dione
- (4S,7R,8S,9S,13(Z ou E),165(Z))-4,8-dihydroxy-16-(1-fluoro-2-(2-pyridyl)éthényl)-1-oxa-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-fluoro-2-(2-pyridyl)éthényl)-10,12,16-triméthyl-8,8-(1,3-triméthylène)-4,17-dioxabicyclo[14.1.0]heptadécane-5,9-dione
- (4S,7R,8S,9S,13(Z ou E),16S(Z))-4,8-dihydroxy-9,13-diméthyl-7-éthyl-16-(1-fluoro-2-(2-pyridyl)éthényl)-1-oxa-5,5-(1,3-triméthylène)cyclohexadéc-13-ène-2,6-dione
- (lRS,3S(Z),7S,10R,11S,12S,16RS)-7,11-dihydroxy-12,16-diméthyl-10-éthyl-3-(1-fluoro-2-(2-pyridyl)éthényl)-8,8-(1,3-triméthylène)-4,17-dioxabicyclo[14.1.0]heptadécane-5,9-dione
- 4S,7R,8S,9S,13(Z ou E),165(Z))-4,8-dihydroxy-16-(1-fluoro-2-(2-pyridyl)éthényl)-1-aza-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-fluoro-2-(2-pyridyl)éthényl)-10,12,16-triméthyl-8,8-(1,3-triméthylène)-4-aza-17-oxabicyclo[14.1.0]heptadécane-5,9-dione
- (4S,7R,8S,9S,13(Z ou E),165(Z))-4,8-dihydroxy-9,13-diméthyl-7-éthyl-16-(1-fluoro-2-(2-pyridyl)éthényl)-1-aza-5,5-(1,3-triméthylène)cyclohexadéc-13-ène-2,6-dione
- (1RS,3S(Z),7S,10R,11S,12S,16RS)-7,11-dihydroxy-12,16-diméthyl-10-éthyl-3-(1-fluoro-2-(2-pyridyl)éthényl)-8,8-(1,3-triméthylène)-4-aza-17-oxabicyclo[14.1.0]heptadécane-5,9-dione
- (4S,7R,8S,9S,13(Z ou E),165(Z))-4,8-dihydroxy-16-(1-chloro-2-(2-pyridyl)éthényl)-1-oxa-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-chloro-2-(2-pyridyl)éthényl)-10,12,16-triméthyl-8,8-(1,3-triméthylène)-4,17-dioxabicyclo[14.1.0]heptadécane-5,9-dione
- (4S,7R,8S,9S,13(Z ou E),16S(Z))-4,8-dihydroxy-9,13-diméthyl-7-éthyl-16-(1-chloro-2-(2-pyridyl)éthényl)-1-oxa-5,5-(1,3-triméthylène)cyclohexadéc-13-ène-2,6-dione
- (1RS,3S(Z),7S,10R,11S,12S,16RS)-7,11-dihydroxy-12,16-diméthyl-10-éthyl-3-(1-chloro-2-(2-pyridyl)éthényl)-8,8-(1,3-triméthylène)-4,17-dioxabicyclo[14.1.0]heptadécane-5,9-dione
- 4S,7R,8S,9S,13(Z ou E), 16S(Z))-4,8-dihydroxy-16-(1-chloro-2-(2-pyridyl)éthényl)-1-aza-7,9,13-triméthyl-5,5-(1,3-triméthylène)cyclohexadéc-13-ène-2,6-dione
- (1RS,3S(Z),7S,10R,11S,12S,16RS)-7,11-dihydroxy-3-(1-chloro-2-(2-pyridyl)éthényl)-10,12,16-triméthyl-8,8-(1,3-triméthylène)-4-aza-17-oxabicyclo[14.1.0]heptadécane-5,9-dione
- (4S,7R,8S,9S,13(Z ou E),16S(Z))-4,8-dihydroxy-9,13-diméthyl-7-éthyl-16-(1-chloro-2-(2-pyridyl)éthényl)-1-aza-5,5-(1,3-triméthylène)cyclohexadéc-13-ène-2,6-dione
- (1RS,3S(z),7S,10R,11S,12S,16RS)-7,11-dihydroxy-12,16-diméthyl-10-éthyl-3-(1-chloro-2-(2-pyridyl)éthényl)-8,8-(1,3-triméthylène)-4-aza-17-oxabicyclo[14.1.0]heptadécane-5,9-dione

20. Préparation pharmaceutique, contenant au moins un composé de formule générale I selon l'une quelconque des revendications précédentes 1 à 19 ainsi qu'un support pharmaceutiquement acceptable.

21. Utilisation des composés de formule générale I selon les revendications précédentes 1 à 19 pour la préparation de médicaments.

22. Produits intermédiaires de formule générale C 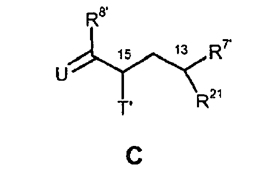 où
R^{8'} a la signification mentionnée pour R⁸ dans la formule générale I et
R^{7'} signifie un atome d'hydrogène,
T' représente un groupe OR²⁰, R²⁰ représentant un atome d'hydrogène ou un groupe de protection PG² , un atome d'halogène, de préférence un atome de brome ou d'iode, un groupe azido ou un groupe amino protégé,
R²¹ signifie un groupe hydroxy, halogène, un groupe hydroxy protégé OPG³, un radical halogénure de phosphonium PPh₃⁺Hal⁻ (Ph = phényle ; Hal = F, Cl, Br, I), un radical phosphonate P(O) (OQ)₂ (Q = C₁-C₁₀-alkyle ou phényle) ou un radical phosphinoxyde P(O)Ph₂ (Ph = phényle),
U signifie un groupe CR¹⁰R¹¹,
R¹⁰, R¹¹ étant identiques ou différents et représentant hydrogène, un radical C₁-C₂₀-alkyle, aryle, C₇-C₂₀-aralkyle ou
R¹⁰ et R¹¹ représentant, ensemble avec l'atome de carbone du groupe méthylène, un cycle carbocyclique de 5 à 7 chaînons
à l'exception des composés (E)-3-fluoro-1-phénylhex-2-ène-4,6-diol et 2-bromo-3-(tert-butyldiméthylsilyloxy)-1-pentén-5-ol

23. Produits intermédiaires de formule générale BC 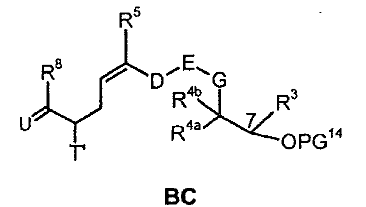 dans laquelle R³, R^{4a}, R^{4b}, R⁵, R⁸, D, E, G, T' et U ont les significations déjà mentionnées et PG¹⁴ représente un atome d'hydrogène ou un groupe de protection PG.

24. Produits intermédiaires de formule générale ABC 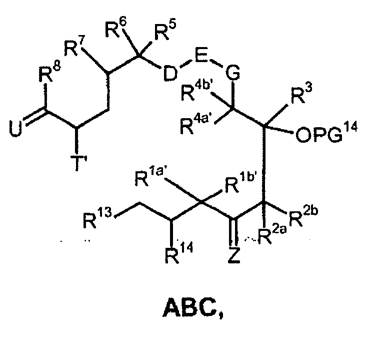 dans laquelle R^{1a'}, R^{1b'}, R^{2a'}, R^{2b'}, R³, R^{4a'}, R^{4b'}, R⁵, R⁶, R⁷, R⁸, R¹³, R¹⁴, D, E, G, T' , U et Z ont les significations déjà mentionnées.
